(19) Europäisches Patentamt
European Patent Office
Office européen des brevets

(11) EP 3 318 121 A1

(12) EUROPEAN PATENT APPLICATION

(43) Date of publication:
**09.05.2018 Bulletin 2018/19**

(21) Application number: **17191951.7**

(22) Date of filing: **07.10.2005**

(51) Int Cl.:
***A01H 5/10*** *(2018.01)* ***C07C 57/00*** *(2006.01)*
***A23L 19/18*** *(2016.01)*

(84) Designated Contracting States:
**AT BE BG CH CY CZ DE DK EE ES FI FR GB GR HU IE IS IT LI LT LU LV MC NL PL PT RO SE SI SK TR**

(30) Priority: **08.10.2004 US 617532 P**

(62) Document number(s) of the earlier application(s) in accordance with Art. 76 EPC:
**11000857.0 / 2 338 328**
**05802517.2 / 1 799 027**

(71) Applicant: **Dow AgroSciences LLC**
**Indianapolis IN 46268-1054 (US)**

(72) Inventors:
- **THOMPSON, Mark**
  **Zionsville, IN 46077 (US)**
- **REDDY, Sam**
  **Carmel, IN 46032 (US)**

(74) Representative: **Zwicker, Jörk**
**ZSP Patentanwälte PartG mbB**
**Hansastraße 32**
**80686 München (DE)**

Remarks:
•This application was filed on 20-09-2017 as a divisional application to the application mentioned under INID code 62.
•Claims filed after the date of receipt of the divisional application (Rule 68(4) EPC).

# (54) CERTAIN PLANTS WITH "NO SATURATE" OR REDUCED SATURATE LEVELS OF FATTY ACIDS IN SEEDS, AND OIL DERIVED FROM THE SEEDS

(57) The subject invention provides "no sat" canola oil. The subject invention also provides seeds that can be used to produce such oils. Plants that produce these seeds are also included within the subject invention. All of this was surprisingly achieved by using a delta-9 desaturase gene in canola. This technology can be applied to other plants as disclosed herein. Oils of the subject invention have particularly advantageous characteristics and fatty acid profiles, which were not heretofore attained. The subject invention still further provides a plant-optimized delta-9 desaturase gene. The subject invention still further provides a plant-optimized delta-9 desaturase gene. In some preferred embodiments, a preferred plant comprises at least two copies of a delta-9 desaturase gene of the subject invention. Seeds produced by such plants surprisingly do not exhibit effects of gene silencing but rather have further surprising reductions in levels of total saturates.

EP 3 318 121 A1

SEQUENCE LISTING

<110> Dow AgroSciences LLC

<120> Certain Plants with "No Saturate" or Reduced Saturate Levels of Fatty Acids in Seeds, and Oil Derived from the Seeds

<130> 502-20 EPT2

<140> EP 11000857.0
<141> 2005-10-07

<150> US 60/617,532
<151> 2004-10-08

<160> 5

<170> PatentIn version 3.3

<210> 1
<211> 1365
<212> DNA
<213> Artificial Sequence

<220>
<223> Nucleic acid sequence of the open reading frame for the plant-optimized, delta-9 desaturase gene

<400> 1

```
atgtctgctc caaccgctga catcagggct agggctccag aggctaagaa ggttcacatc      60

gctgataccg ctatcaacag gcacaattgg tacaagcacg tgaactggct caacgtcttc     120

ctcatcatcg gaatcccact ctacggatgc atccaagctt tctgggttcc acttcaactc     180

aagaccgcta tctgggctgt gatctactac ttcttcaccg gacttggaat caccgctgga     240

taccacaggc tttgggctca ctgctcttac tctgctactc ttccacttag gatctggctt     300

gctgctgttg gaggaggagc tgttgaggga tctatcagat ggtgggctag ggatcacagg     360

gctcatcata ggtacaccga taccgacaag gacccatact ctgttaggaa gggacttctc     420

tactctcacc ttggatggat ggtgatgaag cagaacccaa agaggatcgg aaggaccgac     480

atctctgatc tcaacgagga cccagttgtt gtttggcaac acaggaacta cctcaaggtt     540

gtgttcacca tgggacttgc tgttccaatg cttgttgctg gacttggatg gggagattgg     600

cttggaggat tcgtgtacgc tggaatcctt aggatcttct tcgttcaaca agctaccttc     660

tgcgtgaact ctcttgctca ctggcttgga gatcaaccat tcgatgatag gaactctcct     720

agggatcacg tgatcaccgc tcttgttacc cttggagagg gataccacaa cttccaccac     780

gagttcccat ctgactacag gaacgctatc gagtggcacc agtacgatcc taccaagtgg     840

tctatctggg cttggaagca acttggattg gcttacgatc tcaagaagtt cagggctaac     900

gagatcgaga agggaagggt tcaacaactt cagaagaagc ttgataggaa gagggctact     960

cttgattggg gaaccccact tgatcaactt ccagtgatgg aatgggatga ctacgttgag    1020
```

caagctaaga acggaagggg acttgttgct atcgctggag ttgttcacga tgttaccgac 1080

ttcatcaagg atcacccagg aggaaaggct atgatctctt ctggaatcgg aaaggatgct 1140

accgctatgt tcaacggagg agtgtactac cactctaacg cagctcacaa ccttcttagc 1200

accatgaggg tgggagtgat caggggagga tgcgaggttg agatctggaa gagggctcag 1260

aaggagaacg ttgagtacgt tagggatgga tctggacaaa gggtgatcag ggctggagag 1320

caaccaacca agatcccaga gccaatccca accgctgatg ctgct 1365

<210> 2
<211> 1381
<212> DNA
<213> Artificial Sequence

<220>
<223> ORF of SEQ ID NO:1 preceded by a Kozak sequence and a BamHI cloning site, plus a translational terminator at the end of the ORF

<220>
<221> misc_feature
<222> (1)..(10)
<223> Kozak sequence and BamHI cloning site

<220>
<221> misc_feature
<222> (1379)..(1381)
<223> Translational terminator

<400> 2
ggatccaaca atgtctgctc caaccgctga catcagggct agggctccag aggctaagaa 60

ggttcacatc gctgataccg ctatcaacag gcacaattgg tacaagcacg tgaactggct 120

caacgtcttc ctcatcatcg gaatcccact ctacggatgc atccaagctt tctgggttcc 180

acttcaactc aagaccgcta tctgggctgt gatctactac ttcttcaccg gacttggaat 240

caccgctgga taccacaggc tttgggctca ctgctcttac tctgctactc ttccacttag 300

gatctggctt gctgctgttg gaggaggagc tgttgaggga tctatcagat ggtgggctag 360

ggatcacagg gctcatcata ggtacaccga taccgacaag gacccatact ctgttaggaa 420

gggacttctc tactctcacc ttggatggat ggtgatgaag cagaacccaa agaggatcgg 480

aaggaccgac atctctgatc tcaacgagga cccagttgtt gtttggcaac acaggaacta 540

cctcaaggtt gtgttcacca tgggacttgc tgttccaatg cttgttgctg gacttggatg 600

gggagattgg cttggaggat tcgtgtacgc tggaatcctt aggatcttct tcgttcaaca 660

agctaccttc tgcgtgaact ctcttgctca ctggcttgga gatcaaccat tcgatgatag 720

gaactctcct agggatcacg tgatcaccgc tcttgttacc cttggagagg gataccacaa 780

cttccaccac gagttcccat ctgactacag gaacgctatc gagtggcacc agtacgatcc 840

```
taccaagtgg tctatctggg cttggaagca acttggattg gcttacgatc tcaagaagtt    900

cagggctaac gagatcgaga agggaagggt tcaacaactt cagaagaagc ttgataggaa    960

gagggctact cttgattggg gaaccccact tgatcaactt ccagtgatgg aatgggatga   1020

ctacgttgag caagctaaga acggaagggg acttgttgct atcgctggag ttgttcacga   1080

tgttaccgac ttcatcaagg atcacccagg aggaaaggct atgatctctt ctggaatcgg   1140

aaaggatgct accgctatgt tcaacggagg agtgtactac cactctaacg cagctcacaa   1200

ccttcttagc accatgaggg tgggagtgat caggggagga tgcgaggttg agatctggaa   1260

gagggctcag aaggagaacg ttgagtacgt tagggatgga tctggacaaa gggtgatcag   1320

ggctggagag caaccaacca agatcccaga gccaatccca accgctgatg ctgcttgatg   1380

a                                                                   1381


<210>  3
<211>  21
<212>  DNA
<213>  Artificial Sequence

<220>
<223>  Delta-9 forward B primer

<400>  3
tgagttcatc tcgagttcat g                                               21


<210>  4
<211>  20
<212>  DNA
<213>  Artificial Sequence

<220>
<223>  Delta-9 reverse B primer

<400>  4
gatccaacaa tgtctgctcc                                                 20


<210>  5
<211>  455
<212>  PRT
<213>  Artificial Sequence

<220>
<223>  Protein encoded by the nucleic acid sequence of the open reading
       frame for the plant-optimized, delta-9 desaturase gene

<400>  5

Met Ser Ala Pro Thr Ala Asp Ile Arg Ala Arg Ala Pro Glu Ala Lys
1               5                   10                  15


Lys Val His Ile Ala Asp Thr Ala Ile Asn Arg His Asn Trp Tyr Lys
            20                  25                  30
```

```
His Val Asn Trp Leu Asn Val Phe Leu Ile Ile Gly Ile Pro Leu Tyr
        35                  40                  45

Gly Cys Ile Gln Ala Phe Trp Val Pro Leu Gln Leu Lys Thr Ala Ile
    50                  55                  60

Trp Ala Val Ile Tyr Tyr Phe Phe Thr Gly Leu Gly Ile Thr Ala Gly
65                  70                  75                  80

Tyr His Arg Leu Trp Ala His Cys Ser Tyr Ser Ala Thr Leu Pro Leu
                85                  90                  95

Arg Ile Trp Leu Ala Ala Val Gly Gly Gly Ala Val Glu Gly Ser Ile
            100                 105                 110

Arg Trp Trp Ala Arg Asp His Arg Ala His His Arg Tyr Thr Asp Thr
        115                 120                 125

Asp Lys Asp Pro Tyr Ser Val Arg Lys Gly Leu Leu Tyr Ser His Leu
    130                 135                 140

Gly Trp Met Val Met Lys Gln Asn Pro Lys Arg Ile Gly Arg Thr Asp
145                 150                 155                 160

Ile Ser Asp Leu Asn Glu Asp Pro Val Val Val Trp Gln His Arg Asn
                165                 170                 175

Tyr Leu Lys Val Val Phe Thr Met Gly Leu Ala Val Pro Met Leu Val
            180                 185                 190

Ala Gly Leu Gly Trp Gly Asp Trp Leu Gly Gly Phe Val Tyr Ala Gly
        195                 200                 205

Ile Leu Arg Ile Phe Phe Val Gln Gln Ala Thr Phe Cys Val Asn Ser
    210                 215                 220

Leu Ala His Trp Leu Gly Asp Gln Pro Phe Asp Asp Arg Asn Ser Pro
225                 230                 235                 240

Arg Asp His Val Ile Thr Ala Leu Val Thr Leu Gly Glu Gly Tyr His
                245                 250                 255

Asn Phe His His Glu Phe Pro Ser Asp Tyr Arg Asn Ala Ile Glu Trp
            260                 265                 270

His Gln Tyr Asp Pro Thr Lys Trp Ser Ile Trp Ala Trp Lys Gln Leu
```

```
            275                 280                 285

Gly Leu Ala Tyr Asp Leu Lys Lys Phe Arg Ala Asn Glu Ile Glu Lys
    290                 295                 300

Gly Arg Val Gln Gln Leu Gln Lys Lys Leu Asp Arg Lys Arg Ala Thr
305                 310                 315                 320

Leu Asp Trp Gly Thr Pro Leu Asp Gln Leu Pro Val Met Glu Trp Asp
                325                 330                 335

Asp Tyr Val Glu Gln Ala Lys Asn Gly Arg Gly Leu Val Ala Ile Ala
            340                 345                 350

Gly Val Val His Asp Val Thr Asp Phe Ile Lys Asp His Pro Gly Gly
        355                 360                 365

Lys Ala Met Ile Ser Ser Gly Ile Gly Lys Asp Ala Thr Ala Met Phe
    370                 375                 380

Asn Gly Gly Val Tyr Tyr His Ser Asn Ala Ala His Asn Leu Leu Ser
385                 390                 395                 400

Thr Met Arg Val Gly Val Ile Arg Gly Gly Cys Glu Val Glu Ile Trp
                405                 410                 415

Lys Arg Ala Gln Lys Glu Asn Val Glu Tyr Val Arg Asp Gly Ser Gly
            420                 425                 430

Gln Arg Val Ile Arg Ala Gly Glu Gln Pro Thr Lys Ile Pro Glu Pro
        435                 440                 445

Ile Pro Thr Ala Asp Ala Ala
    450                 455

        1
```

## Description

### Cross-Reference to Related Application

The subject application claims priority to U.S. provisional application serial number 60/617,532 filed on October 8, 2004.

### Background of the Invention

Vegetable-derived oils have gradually replaced animal-derived oils and fats as the major source of dietary fat intake. However, saturated fat intake in most industrialized nations has remained at about 15% to 20% of total caloric consumption. In efforts to promote healthier lifestyles, the United States Department of Agriculture (USDA) has recently recommended that saturated fats make up less than 10% of daily caloric intake. To facilitate consumer awareness, current labeling guidelines issued by the USDA now require total saturated fatty acid levels be less than 1.0 g per 14 g serving to receive the "low-sat" label and less than 0.5 g per 14 g serving to receive the "no-sat" label. This means that the saturated fatty acid content of plant oils needs to be less than 7% and 3.5% to receive the "low sat" and "no sat" label, respectively. Since issuance of these guidelines, there has been a surge in consumer demand for "low-sat" oils. To date, this has been met principally with canola oil, and to a much lesser degree with sunflower and safflower oils.

The characteristics of oils, whether of plant or animal origin, are determined predominately by the number of carbon and hydrogen atoms, as well as the number and position of double bonds comprising the fatty acid chain. Most oils derived from plants are composed of varying amounts of palmitic (16:0), stearic (18:0), oleic (18:1), linoleic (18:2) and linolenic (18:3) fatty acids. Conventionally, palmitic and stearic acids are designated as "saturated" because their carbon chains are saturated with hydrogen atoms and hence have no double bonds; they contain the maximal number of hydrogen atoms possible. However, oleic, linoleic, and linolenic are 18-carbon fatty acid chains having one, two, and three double bonds, respectively, herein. Oleic acid is typically considered a mono-unsaturated fatty acid, whereas linoleic and linolenic are considered to be poly-unsaturated fatty acids. The U.S. Department of Agriculture defines "no saturates" or "no sat" products as a product having less than 3.5% by weight combined saturated fatty acids (as compared to the total amount of fatty acids).

While unsaturated fats (monounsaturated and polyunsaturated) are beneficial (especially when consumed in moderation), saturated and trans fats are not. Saturated fat and trans fat raise LDL cholesterol levels in the blood. Dietary cholesterol also raises LDL cholesterol and may contribute to heart disease even without raising LDL. Therefore, it is advisable to choose foods low in saturated fat, trans fat, and cholesterol as part of a healthful diet.

The health value of high levels of monounsaturates, particularly oleic acid, as the major dietary fat constituent has b een established by recent studies. Such diets are thought to reduce the incidence of arteriosclerosis that results from diets high in saturated fatty acids. There is accordingly a need for an edible vegetable oil having a high content of monounsaturates. Seed mutagenesis has been used to produce a rapeseed oil with no more than 4% saturated fatty acid content (PCT International Patent Application Publication Number WO 91/15578).

Over 13% of the world's supply of edible oil in 1985 was produced from the oilseed crop species Brassica, commonly known as rapeseed or mustard. Brassica is the third most important source of edible oil, ranking behind only soybean and palm. Because Brassica is able to germinate and grow at relatively low temperatures, it is also one of the few commercially important edible oilseed crops that can be cultivated in cooler agricultural regions, as well as serving as a winter crop in more temperate zones. Moreover, vegetable oils in general, and rapeseed oil in particular, are gaining increasing consideration for use in industrial applications because they have the potential to provide performance comparable to that of synthetic or mineral/naphthenic-based oils with the very desirable advantage of also being biodegradable.

Canola oil has the lowest level of saturated fatty acids of all vegetable oils. "Canola" refers to rapeseed (Brassica) which has an erucic acid (C22:1) content of at most 2 percent by weight based on the total fatty acid content of a seed (preferably at most 0.5 percent by weight and most preferably essentially 0 percent by weight) and which produces, after crushing, an air-dried meal containing less than 30 micromoles per gram of defatted (oil-free) meal. These types of rapeseed are distinguished by their edibility in comparison to more traditional varieties of the species.

Modification of vegetable oils maybe effected chemically. This approach has been used to obtain a salad/cooking oil which contains saturated fatty acids of less than about 3% (U.S. Pat. No. 4,948,811); the oil may be formed by chemical reaction, or by physical separation of the saturated lipids. A general reference is made to using "genetic engineering" to achieve an oil of the desired characteristics (*see* column 3, line 58 *et seq.*). However, there is no detailed disclosure of how any particular oilseed plant could be so modified to provide a vegetable oil of the characteristics desired.

Typically, the fatty acid composition of vegetable oils has instead been modified through traditional breeding techniques. These techniques utilize existing germplasm as a source of naturally occurring mutations that affect fatty acid composition. Such mutations are uncovered and selected for by the use of appropriate screening, in conjunction with subsequent breeding. For example, such an approach has been used to decrease the amount of the long chain fatty acid erucate in rapeseed oil (Stefansson, B. R. (1983) in High and Low Erucic Acid Rapeseed Oils, Kramer J. K. G. et al., eds; Academic Press, New York; pp. 144-161), and to increase the amount of the monounsaturated fatty acid oleate in corn oil (U.S. patent application, Ser. No. 07/554,526).

Recently, attempts have been made to increase the pool of available mutations from which to select desired characteristics through the use of mutagens. However, mutagens generally act by inactivation or modification of genes already present, resulting in the loss or decrease of a particular function. The introduction of anew characteristic through mutagenesis thus often depends on the loss of some trait already present. In addition, the achievement of desired goals with mutagens is generally uncertain. Only a few types of modified fatty acid compositions in vegetable oils have been achieved using this approach. One example of such a "created" mutation which affects fatty acid composition is the decrease of polyunsaturated fatty acids, in particular oflinoleate and linolenate, in rapeseed oil, with a concomitant increase in the monounsaturated fatty acid oleate (Auld, M., et al., (1992) Crop Sci. in press). Another is the decrease of saturated fatty acids in rapeseed oil (PCT International Patent Application Publication Number WO 91/15578). However, the biochemistry of seed oil synthesis is complex, and not well understood; there may be several mechanisms which contribute to the changes in the fatty acid compositions observed in rapeseed oil (PCT International Patent Application Publication Number WO 91/15578). The use of mutagenesis to affect such changes is essentially random, and non-specific.

The possibility of modifying fatty acid composition through the use of genetic engineering would, in theory, allow the precise, controlled introduction of specific desirable genes, as well as the inactivation of specific undesirable genes or gene products. Thus, novel traits completely independent of genes already present could be introduced into plants, or preselected genes could be inactivated or modified. However, one predicate to making effective use of genetic engineering to modify fatty acid compositions is a reasonably accurate model of the mechanisms at work in the plant cell regulating fatty acid synthesis and processing.

U.S. Patent No. 6,495,738 (*see also* WO 99/50430) shows that the levels of saturated fatty acids in corn oil and tobacco seeds can be altered by expressing a fungal palmitate-CoA delta-9 desaturase within a plant cell. These proteins most likely enzymatically desaturate palmitate-CoA molecules, preferentially, by removing two hydrogen atoms and adding a double bond between the 9th and 10th carbon atoms from the CoA portion of the molecule, thus producing palmitoleic-CoA (16:1 delta-9). The palmitoleic-CoA is ultimately incorporated into seed oil thus lowering the total saturate levels of said oil. The total saturated fatty acid level of corn oil, averaging about 13.9%, does not meet the current labeling guidelines discussed above. Furthermore, corn is typically not considered to be an oil crop as compared to soybean, canola, sunflower, and the like. In fact, the oil produced and extracted from corn is considered to be a byproduct of the wet milling process used in starch extraction. Because of this, there has been little interest in modifying the saturate levels of corn oil.

It is postulated that, in oilseeds, fatty acid synthesis occurs primarily in the plastid, and that the newly synthesized fatty acids are exported from the plastid to the cytoplasm. In the cytoplasm they are utilized in the assembly of triglycerides, which occurs in the endoreticular membranes.

The major product of fatty acid synthesis is palmitate (16:0), which appears to be efficiently elongated to stearate (18:0). While still in the plastid, the saturated fatty acids may then be desaturated, by an enzyme known as delta-9 desaturase, to introduce one or more carbon-carbon double bonds. Specifically, stearate may be rapidly desaturated by a plastidial delta-9 desaturase enzyme to yield oleate (18:1). In fact, palmitate may also be desaturated to palmitoleate (16:1) by the plastidial delta-9 desaturase, but this fatty acid appears in only trace quantities (0-0.2%) in most vegetable oils.

Thus, the major products of fatty acid synthesis in the plastid are palmitate, stearate, and oleate. In most oils, oleate is the major fatty acid synthesized, as the saturated fatty acids are present in much lower proportions.

Subsequent desaturation of plant fatty acids outside the plastid in the cytoplasm appears to be limited to oleate, which may be desaturated to linoleate (18:2) and linolenate (18:3). In addition, depending on the plant, oleate may be further modified by elongation (to 20:1, 22:1, and/or 24:1), or by the addition of functional groups. These fatty acids, along with the saturated fatty acids palmitate and stearate, may then be assembled into triglycerides.

The plant delta-9 desaturase enzyme is soluble. It is located in the plastid stroma, and uses newly synthesized fatty acids esterified to ACP, predominantly stearyl-ACP, as substrates. This is in contrast to the yeast delta-9 desaturase enzyme, which is located in the endoplasmic reticular membrane (ER, or microsomal), uses fatty acids esterified to Co-A as substrates, and desaturates both the saturated fatty acids palmitate and stearate. U.S. Patent Nos. 5,723,595 and 6,706,950 relate to a plant desaturase.

The yeast delta-9 desaturase gene has been isolated from *Saccharomyces cerevisiae,* cloned, and sequenced (Stukey, J. E. et al., J. Biol. Chem. 264:16537-16544 (1989); Stukey, J. E. et al., J. Biol. Chem. 265:20144-20149 (1990)). This gene has also been used to transform the same yeast strain under conditions in which it is apparently overexpressed, resulting in increased storage lipid accumulation in the transformed yeast cells as determined by fluorescence microscopy using Nile Red as a stain for triglycerides (U.S. Pat. No. 5,057,419). The fatty acid composition was not characterized.

This reference contains a *general* discussion of using information from the isolated yeast delta-9 desaturase gene to first isolate other desaturase genes from yeast, or from other organisms, and then to re-introduce these genes into a yeast or plant under conditions. It is speculated that this could lead to high expression in order to modify the oil produced and its fatty acid composition.

Subsequently, it was reported that the yeast delta-9 desaturase gene had been introduced into tobacco leaf tissue (Polashcok, J. et al, FASEB J. 5:A1157 (1991) and was apparently expressed in this tissue. Further, this gene was expressed in tomato. *See* Wang et al., J. Agric Food Chem. 44:3399-3402 (1996); and C. Wang et al., Phytochemistry 58:227-232 (2001). While some increases in certain unsaturates and some decreases in some saturates were reported for both tobacco and tomato, tobacco and tomato are clearly not oil crops. This yeast gene was also introduced into *Brassica napus* (*see* U.S. Patent No. 5,777,201). Although a reduction in palmitate and stearate (saturates) and an increase in palmitoleate and oleate (unsaturates) was reported (*see* Tables 1 a and 1b in Example 7 of that patent), this reference is discussed in more detail towards the beginning of the Detailed Description section, below. WO 00/11012 and U.S. Patent No. 6,825,335 relate to a synthetic yeast desaturase gene for expression in a plant, wherein the gene comprises a desaturase domain and a cyt $b_5$ domain. The Background section of these references discuss fatty acid synthesis in detail.

The performance characteristics, whether dietary or industrial, of a vegetable oil are substantially determined by its fatty acid profile, that is, by the species of fatty acids present in the oil and the relative and absolute amounts of each species. While several relationships between fatty acid profile and performance characteristics are known, many remain uncertain. Notwithstanding, the type and amount of unsaturation present in a vegetable oil have implications for both dietary and industrial applications.

Standard canola oil contains about 8-12% linolenic acid, which places it in a similar category as soybean oil with respect to oxidative, and hence flavor, stability. The oxidative stability of canola oil can be improved in a number of ways, such as by hydrogenating to reduce the amount of unsaturation, adding antioxidants, and blending the oil with an oil or oils having better oxidative stability. For example, blending canola oil with low linolenic acid oils, such as sunflower, reduces the level of 18:3 and thus improves the stability of the oiL However, these treatments necessarily increase the expense of the oil, and can have other complications; for example, hydrogenation tends to increase both the level of saturated fatty acids and the amount of trans unsaturation, both of which are undesirable in dietary applications.

High oleic oils are available, but, in addition to the possible added expense of such premium oils, vegetable oils from crops bred for very high levels of oleic acid can prove unsatisfactory for industrial uses because they retain fairly high levels of polyunsaturated fatty acids, principally linoleic and/or linolenic. Such oils may still be quite usable for dietary applications, including use as cooking oils, but have inadequate oxidative stability under the more rigorous conditions found in industrial applications. Even the addition of antioxidants may not suffice to bring these oils up to the levels of oxidative stability needed for industrial applications; this is probably due to the levels of linolenic acid, with its extremely high susceptibility to oxidation, found in these oils.

Oxidative stability is important for industrial applications to extend the life of the lubricant under conditions of heat and pressure and in the presence of chemical by-products. In such applications linolenic acid, and to a lesser extent linoleic acid, are again most responsible for poor oxidative stability.

Therefore, it would be desirable to obtain a variety of *Brassica napus* which is agronomically viable and produces seed oil having a level of oxidative stability sufficient to qualify it for use in dietary applications, and which would additionally be either sufficiently stable alone, or, depending on the precise application, sufficiently responsive to antioxidants, to find use in industrial applications.

European Patent Application EP 323753, U. S. Patent No. 5,840,946, and U.S. Patent No. 5,63 8,637 are directed to rapeseed oil having an oleic content of 80-90% (by weight, of total fatty acid content) and not more than 2% erucic acid. Mutagenesis was used to improve the oleic acid content. The claims of the '946 patent further specify that the oil also has an erucic acid content ofno more than 2%, and alpha-linolenic acid content of less than 3.5%, and a saturated fatty acid content in the form of stearic and palmitic of no more than 7%. These patents relate to mutagenesis followed by selection.

U.S. Patent Nos. 5,387,758; 5,434,283; and 5,545,821 are directed to rapeseed having 2-4% combined stearic and palmitic acids (by weight), and an erucic acid content of no more than about 2% by weight. Mutagenesis was used to lower the stearic and palmitic acid content.

International Application WO 92/03919, and U.S. PatentNos. 5,668,299; 5,861,187; and 6,084,157 are directed to rapeseed seeds, plants, and oils having altered fatty acid profiles. Several such profiles are mentioned, all of which contemplate a maximum erucic acid content of about 2%, combined with palmitic acid content of from about 7% to about 12%, linoleic content of about 14% to about 20%, stearic acid content of from about 0.8% to about 1.1%, and alpha-linolenic acid content of about 7% to about 9%, as well as certain ranges of FDA saturates. These patents define saturated fatty acids and "FDA saturates" as the sum of lauric (C12:0), myristic (C14:0), palmitic (C16:0), and stearic (C18:0) acids.

International Application WO 93/06714, and U.S. Patent Nos. 6,270,828; 6,562,397; 6,680,396; and 6,689,409 are directed to canola oil and seeds with reduced glucosinolates (and thus reduced sufur), as well as an alpha-linolenic acid content of about 2% to about 7%.

U.S. Patent No. 6,169,190 relates to oil from canola seed having an oleic fatty acid content of approximately 71-77% and a linolenic acid content of less than about 3%. Oleic:linolenic ratios between 34-55 are also claimed.

U.S. Patent Nos. 6,063,947 and 5,850,026 claim oil obtained from canola seeds, related canola plants, and methods of producing the oil, wherein the oil has an oleic acid content greater than about 80% (about 86-89%), a linoleic acid content of about 2% to about 6%, an alpha-linolenic acid content of less than 2.5% (about 1-2%), and an erucic acid content of less than about 2% (after hydrolysis). These patents relate to seed-specific inhibition of microsomal oleate desaturase (a delta-12 desaturase which converts oleic acid to linoleic acid) and microsomal linoleate desaturase (a delta-15 desaturase which converts linoleic acid to alpha-linolenic acid) gene expression.

U.S. Patent No. 5,952,544 claims fragments of a plant plastid or microsomal delta-15 fatty acid desaturase enzyme, which catalyzes a reaction between carbons 15 and 16.

U.S. Patent Nos. 4,627,192 and 4,743,402 relate to sunflower seeds and sunflower oil having an oleic acid content of approximately 80-94% (relative to the total fatty acid content thereof) and a ratio of linoleic to oleic of less than about 0.09. These sunflower plants were obtained by traditional breeding techniques.

WO 2003002751 relates to the use of kinase genes and the like to alter the oil phenotype of plants.

The ability of delta-9 desaturase genes to significantly (and desirably) affect the fatty acid profile of already-beneficial oil seed crops, particularly to decrease the levels of saturated fats without adversely affecting other aspects of the plant and oil, is unpredictable.

## Brief Summary of the Invention

The subject invention provides "no sat" canola oil. The invention also relates in part to methods for reducing saturated fatty acids in certain plant seeds. These results were surprisingly achieved by the use of a delta-9 desaturase gene in canola (*Brassica*). This technology can be applied to other plants as disclosed herein. Included in the subject invention are plants, preferably canola, capable of producing such oils and seeds. The subject invention also provides seeds and oils from said plants wherein the oils have particularly advantageous characteristics and fatty acid profiles, which were not heretofore attained. The subject invention still further provides a plant-optimized delta-9 desaturase gene. In some preferred embodiments, a preferred plant comprises at least two copies of a delta-9 desaturase gene of the subject invention. Seeds produced by such plants surprisingly do not exhibit effects of gene silencing but rather have further surprising reductions in levels of total saturates.

## Brief Description of the Figures

**Figure 1** shows that a greater than 60% reduction of saturated fatty acids was achieved in *Arabidopsis.* This graph summarizes T2 and T3 seed data for a single *Arabidopsis* event.

**Figure 2** shows a reduction in "sats" of up to 60-70% in T2 *Arabidopsis* seeds from 18 additional transformants. Data illustrated in this graph was a combination of the numerical data shown in **Table 8** and earlier numerical data.

**Figure 3** shows that saturated fats were reduced by over 43% in Westar canola (and a 50% reduction was achieved when 24:0 was included).

**Figure 4A** shows a bar graph comparing total saturates of seeds from various canola plants comprising Event 36-11.19 compared to a control. **Figures 4B and 4C** present numerical data illustrated by the bar graph.

**Figure 5A** shows a bar graph comparing total saturates of seeds from various canola plants comprising Event 218-11.30 compared to a control. **Figures 5B and 5C** present numerical data illustrated by the bar graph.

**Figures 6A-F** show half-seed data from the T3 field trials. **Figures 6A and 6B** clearly show the reductions in C16:0 and increases in C16:1 in the transgenic events as compared to the nulls (events in which the transgene segregated out of the plant) and wild-type controls (non-transformed lines). **Figures 6C and 6D** clearly show the reductions in C18:0 and increases in C18:**1** in the transgenic events as compared to the nulls and wild-type controls. **Figures 6E and 6F** clearly show the reductions in C20:0 and C22:0, respectively, in the transgenic events as compared to the nulls and wild-type controls.

**Figures 6G and 6H** clearly show shifts and reductions in C16:0, and shifts and increases in C16:1 in the transgenic events, as compared to the nulls and wild-type controls. **Figures 6I and 6J** clearly show shifts and reductions in C18:0, and shifts and increases in C18:1 in the transgenic events, as compared to the nulls and wild-type controls. **Figures 6K and 6L** show similar bar graphs for C18:2 and C18:3. **Figure 6M** further illustrates reductions in total saturates, as compared to already very good Nex 710 lines. **Figure 6N** shows distributions for 1000 seeds.

**Figures 7A and 7B** illustrate data obtained using the protocol of Example 16.

**Figures 8 and 9** are pictures of two gels run with DNA from F3 plants, as discussed in Example 19.

Brief Description of the Sequences

**SEQ ID NO:1** shows the nucleic acid sequence of the open reading frame for the plant-optimized, delta-9 desaturase gene used herein.

**SEQ ID NO:2** shows the sequence of the ORF of SEQ ID NO:1 preceded by a Kozak sequence and a BamHI cloning site (residues 1-10), plus a translational terminator at the end of the ORF (residues 1379-1381).

**SEQ ID NO:3** shows the nucleic acid sequence of the delta-9 forward B primer used to amplify the delta-9 gene.

**SEQ ID NO:4** shows the nucleic acid sequence of the delta-9 reverse B primer used to amplify the delta-9 gene.

**SEQ ID NO:5** shows the amino acid sequence encoded by SEQ ID NO:1.

Detailed Description of the Invention

The subject invention provides "no sat" canola oil. The invention also relates in part to methods for reducing saturated fatty acids in certain plant seeds. These results were surprisingly achieved through the use of a delta-9 desaturase gene to surprisingly produce "no sat" levels of fatty acids in plants, preferably oil plants, and still more preferably canola (*Brassica*). The subject invention includes such plants and also provides seeds and oils from said plants wherein the oils have particularly advantageous characteristics and fatty acid profiles, which were not heretofore attained.

The *Aspergillus nidulans* microsomal delta-9-CoA desaturase gene is exemplified herein. This delta-9 desaturase is a membrane-bound enzyme and catalyzes the reaction of 16:0-CoA and 18:0-CoA to 16:1-CoA and 18:1-CoA (adding a double bond at the delta-9 location). The subject invention was further surprising in that the levels of other saturates, such as C20:0, C22:0, and C24:0, were also very surprisingly and advantageously reduced, while C16:1 and C18:1 unsaturates were increased (with little or no increases in C18:2 and C18:3, or even reductions of these relatively less stable polyunstaturates in some cases). Heretofore, it was unclear whether this would be a good enzyme (including whether the gene could be sufficiently expressed) in *Brassica* and other "good" oil seed plants, which already have a desirable (yet not optimal) fatty acid profile. (For example, it yielded only a 10% decrease in saturates in corn.)

As mentioned in the Background section, given the complex fatty acid profiles and metabolic pathways of different organisms and plants, and the different physical cell machinery thereof, even if this gene and enzyme could have *an* effect in *Brassica*, the effects could not be expected to be beneficial. As discussed in the Background section, increases in one or more types of desirable fatty acids often resulted in decreases of other desirable fatty acids, increases in undesirable fatty acids, and agronomic penalties (*i.e.*, other outright adverse effects on the modified plants). It was also surprising that the subject invention can be practiced without corresponding adverse effects to other valuable agronomic characteristics such as pod size, seed yield, seed size, oil yield, and the like. There were no adverse effects in plants homozygous for a simple transgenic insert. Some double homozygous stacks (made by crossing two transgenic events) exhibited decrease in pod number and seed set; the cause is yet unknown. However, Table 27 contains stacks (that is, apparently increased copy number events) having seed yields similar to non-transgenic controls *and also* 'no sat' composition.

Yet another reason for unpredictably arises because of differences between desaturases, and even between yeast, fungal, plant, and animal delta-9 desaturases. Differences in the desaturases can be attributed in part to differences in cell structures of the source organisms for the various desaturases. A yeast desaturase from U.S. Patent No. 5,777,201 is discussed above in the Background section. It is longer than the *Apergillus* desaturase exemplified herein (510 amino acids vs. 455 amino acids). In addition, it has only about 52% identity over about 400 amino acids (as determined by both BLAST and BestFit, a Smith-Waterman program; both done in EMBOSS). Tables 1 a and 1b of Example 7 of that patent show that the reductions in saturates achieved using the yeast desaturase were much weaker than those achieved according to the subject invention with the exemplified *Aspergillus* desaturase in canola. There are various factors that can be possible explanations for the relatively weaker performance of the yeast desaturase. For example, that protein might be inherently instable in plants (while the subject desaturase is quite apparently very stable in canola). These can also be different in other enzyme properties, such as catalytic efficiencies, substrate affinities, cofactor affinities, and the like.

Compared to the safflower desaturase of U.S. Patent Nos. 5,723,595 and 6,706,950, the safflower desaturase is shorter (396 amino acids) than the presently exemplified *Aspergillus* desaturase (455 amino acids). The safflower desaturase is also found in the plastid, while the subject *Aspergillus* desaturase is found in the ER/microsomes/cytoplasmic compartment. Furthermore, the safflower desaturase uses acyl-ACP substrates found in the plastid, while the *Aspergillus* desaturase uses acyl-CoA substrates found in the cytoplasmic compartment. Thus, for the subject invention, it was not known if a substantial portion of the pool of acyl-CoA substrates would be available to the *Aspergillus* desaturase.

Thus, it was with great surprise that the subj ect delta-9 desaturase was found to be able to yield canola plants, seeds, and oil therefrom having excellent properties, particularly for improving food qualities of the oil. Very surprisingly, a greater than 60% reduction of saturated fatty acids was achieved in Arabidopsis, and a greater than 43 % reduction of saturated fatty acids was achieved in canola. Again, it is important to note that this was achieved in a plant that already yielded one of the best fatty acid profiles of any suitable plant. This invention was also used to achieve surprising and advantageous fatty acid profiles and ratios, as shown and discussed in more detail below. Although stearic acid is considered to be a saturated fatty acid, it has been found to have cholesterol-lowering effects. Thus, relatively higher levels of stearic acid can be beneficial. Similarly, relatively higher levels of arachidonic acid can be desirable. As shown in data herein, oil from seeds of the subject invention have advantageous profiles of these two fatty acids, together with desirable levels of vaccenic acid, for example. Also shown herein is that advantageous levels of these fatty acids and/or total saturates are present in combination with desirable plant height, yield, and other beneficial characteristics in the commercial-quality plants of the subject invention (as opposed to dwarf plants, for example). Again, exemplary data for such plants of the subject invention are presented herein.

It should be noted that the subject invention is not limited to the exemplified desaturase. Various desaturases and delta-9 desaturases are available in GENBANK, and sequence alignments can be performed, using standard procedures, to observe and compare differences in the sequences of the enzymes. Enzymes similar to that exemplified herein can be used according to the subject invention.

For example, the subject *Aspergillus* desaturase has two domains. The first domain (approximately the amino-terminal two-thirds of the molecule) is the desaturase domain, and the second domain (roughly the C-terminal third of the molecule) is a cytochrome b5 domain. Residues 62-279, for example, of SEQ ID NO:5 can be aligned with residues 4-233 of fatty acid desaturase gnl|CDD|25523 pfam00487, for example. Residues 332-407 of SEQ ID NO:5 can be aligned with residues 1-74 of gnl|CDD|22935 pfam00173 (cytochrome b5 domain). Residues 17-305 of SEQ ID NO:5 can be aligned with residues 3-288 of the lipid metabolism domain of fatty acid desaturase gnl|CDD|11113 COG1398 (OLE1). Residues 301-449 of SEQ ID NO:5 can be aligned with residues 11-163 of CYB5 (cytochrome b involved in lipid metabolism) of gnl|CDD|14396 COG5274. The desaturase domain of SEQ ID NO:5 lacking the cytochrome b5 could be functional, as this is the general structure of plant *plastidial* desaturases. There is also a published presumed microsomal pine desaturase (LOCUS AF438199) which uses acyl-CoA substrates found in the cytoplasmic compartment, and it lacks the cytb5 domain. It might also be possible to swap the *Aspergillus* cytochrome b5 domain with that of another organism, even one from a plant *cytoplasmic* desaturase. These domains, or segments encoding either or both of these domains, can be used as probes to define molecules of the subject invention, as discussed in more detail below.

Thus, the genes and proteins useful according to the subject invention include not only the specifically exemplified full-length sequences, but also portions, segments and/or fragments (including internal and/or terminal deletions compared to the full-length molecules) of these sequences, variants, mutants, chimerics, and fusions thereof. Proteins used in the subject invention can have substituted amino acids so long as they retain the characteristic enzymatic activity of the proteins specifically exemplified herein. "Variant" genes have nucleotide sequences that encode the same proteins or equivalent proteins having functionality equivalent to an exemplified protein. The terms "variant proteins" and "equivalent proteins" refer to proteins having the same or essentially the same biological/functional activity as the exemplified proteins. As used herein, reference to an "equivalent" sequence refers to sequences having amino acid substitutions, deletions, additions, or insertions that improve or do not adversely affect functionality. Fragments retaining functionality are also included in this definition. Fragments and other equivalents that retain the same or similar function, as a corresponding fragment of an exemplified protein are within the scope of the subject invention. Changes, such as amino acid substitutions or additions, can be made for a variety of purposes, such as increasing (or decreasing) protease stability of the protein (without materially/substantially decreasing the functionality of the protein).

Variations of genes may be readily constructed using standard techniques for making point mutations, for example. In addition, U.S. Patent No. 5,605,793, for example, describes methods for generating additional molecular diversity by using DNA reassembly after random fragmentation. Variant genes can be used to produce variant proteins; recombinant hosts can be used to produce the variant proteins. Using these "gene shuffling" techniques, equivalent genes and proteins can be constructed that comprise any 5, 10, 15, 20, 25, 30, 35, 40, 45, 50, 55, 60 (for example) contiguous residues (amino acid or nucleotide) of any sequence exemplified herein.

Fragments of full-length genes can be made using commercially available exonucleases or endonucleases according to standard procedures. For example, enzymes such as *Bal*31 or site-directed mutagenesis can be used to systematically cut off nucleotides from the ends of these genes. Also, genes that encode active fragments may be obtained using a variety of restriction enzymes. Proteases may be used to directly obtain active fragments of these proteins.

It is within the scope of the invention as disclosed herein that the subject proteins maybe truncated and still retain functional activity. By "truncated protein" it is meant that a portion of a protein may be cleaved and yet still exhibit enzymatic activity after cleavage. Furthermore, effectively cleaved proteins can be produced using molecular biology techniques wherein the DNA bases encoding said protein are removed either through digestion with restriction endonucleases or other techniques available to the skilled artisan. After truncation, said proteins can be expressed in heterologous systems such as *Escherichia coli,* baculoviruses, plant-based viral systems, yeast and the like and then placed in insect assays as disclosed herein to determine activity. It is well-known in the art that truncated proteins can be successfully produced so that they retain functional activity while having less than the entire, full-length sequence. It is well known in the art that *B.t.* toxins can be used in a truncated (core toxin) form. *See, e.g.,* Adang et al., Gene 36:289-300 (1985), "Characterized full-length and truncated plasmid clones of the crystal protein of *Bacillus thuringiensis* subsp *kurstaki* HD-73 and their toxicity to *Manduca sexta."* There are other examples of truncated proteins that retain insecticidal activity, including the insect juvenile hormone esterase (U.S. Pat. No. 5,674,485 to the Regents of the University of California). As used herein, the term "toxin" is also meant to include functionally active truncations.

Proteins and genes for use according to the subject invention can be defined, identified, and/or obtained by using oligonucleotide probes, for example. These probes are detectable nucleotide sequences which may be detectable by virtue of an appropriate label or may be made inherently fluorescent as described in International Application No. WO 93/16094. The probes (and the polynucleotides of the subject invention) may be DNA, RNA, or PNA. In addition to adenine (A), cytosine (C), guanine (G), thymine (T), and uracil (U; for RNA molecules), synthetic probes (and polynucleotides) of the subject invention can also have inosine (a neutral base capable of pairing with all four bases; sometimes used inplace ofamixture of all four bases in synthetic probes). Thus, where a synthetic, degenerate oligonucleotide is referred to herein, and "N" or "n" is used generically, "N" or "n" can be G, A, T, C, or inosine. Ambiguity codes as used herein are in accordance with standard IUPAC naming conventions as of the filing of the subject application (for example, R means A or G, Y means C or T, etc.).

As is well known in the art, if a probe molecule hybridizes with a nucleic acid sample, it can be reasonably assumed that the probe and sample have substantial homology/similarity/identity. Preferably, hybridization of the polynucleotide is first conducted followed by washes under conditions of low, moderate, or high stringency by techniques well-known in the art, as described in, for example, Keller, G.H., M.M. Manak (1987) DNA. Probes, Stockton Press, New York, NY, pp. 169-170. For example, as stated therein, low stringency conditions can be achieved by first washing with 2x SSC (Standard Saline Citrate)/0.1% SDS (Sodium Dodecyl Sulfate) for 15 minutes at room temperature. Two washes are typically performed. Higher stringency can then be achieved by lowering the salt concentration and/or by raising the temperature. For example, the wash described above can be followed by two washings with 0.1x SSC/0.1% SDS for 15 minutes each at room temperature followed by subsequent washes with 0.1x SSC/0.1% SDS for 30 minutes each at 55° C. These temperatures can be used with other hybridization and wash protocols set forth herein and as would be known to one skilled in the art (SSPE can be used as the salt instead of SSC, for example). The 2x SSC/0.1% SDS can be prepared by adding 50 ml of 20x SSC and 5 ml of 10% SDS to 445 ml of water. 20x SSC can be prepared by combining NaCl (175.3 g/0.150 M), sodium citrate (88.2 g/0.015 M), and water, adjusting pH to 7.0 with 10 N NaOH, then adjusting the volume to 1 liter 10% SDS can be prepared by dissolving 10 g of SDS in 50 ml of autoclaved water, then diluting to 100 ml.

Detection of the probe provides a means for detemining in a known manner whether hybridization has been maintained. Such a probe analysis provides a rapid method for identifying toxin-encoding genes of the subject invention. The nucleotide segments which are used as probes according to the invention can be synthesized using a DNA synthesizer and standard procedures. These nucleotide sequences can also be used as PCR primers to amplify genes of the subject invention.

Hybridization with a given polynucleotide is a technique that can be used to identify, find, and/or define proteins and genes of the subject invention. As used herein, "stringent" conditions for hybridization refers to conditions which achieve the same, or about the same, degree of specificity of hybridization as the conditions described herein. Hybridization of immobilized DNA on Southern blots with $^{32}P$-labeled gene-specific probes can be performed by standard methods (*see, e.g.,* Maniatis, T., E.F. Fritsch, J. Sambrook [1982] Molecular Cloning: A Laboratory Manual, Cold Spring Harbor Laboratory, Cold Spring Harbor, NY). In general, hybridization and subsequent washes are carried out under conditions that allowed for detection of target sequences. For double-stranded DNA gene probes, hybridization can be carried out overnight at 20-25° C below the melting temperature (Tm) of the DNA hybrid in 6x SSPE, 5x Denhardt's solution, 0.1% SDS, 0.1 mg/ml denatured DNA. The melting temperature is described by the following formula (Beltz, G.A., K.A. Jacobs, T.H. Eickbush, P.T. Cherbas, and F.C. Kafatos [1983] Methods of Enzymology, R. Wu, L. Grossman and K. Moldave [eds.] Academic Press, New York 100:266-285):

$$Tm = 81.5^\circ C + 16.6 \, Log[Na+] + 0.41(\%G+C) - 0.61(\%formamide) - 600/\text{length of duplex in base pairs.}$$

Washes are typically carried out as follows:

1) Twice at room temperature for 15 minutes in 1x SSPE, 0.1% SDS (low stringency wash).

2) Once at Tm-20° C for 15 minutes in 0.2x SSPE, 0.1% SDS (moderate stringency wash).

For oligonucleotide probes, hybridization can be carried out overnight at 10-20° C below the melting temperature (Tm) of the hybrid in 6x SSPE, 5x Denhardt's solution, 0.1% SDS, 0.1 mg/ml denatured DNA. Tm for oligonucleotide probes was determined by the following formula: Tm (° C) = 2(number T/A base pairs) + 4(number G/C base pairs) (Suggs, S.V., T. Miyake, E.H. Kawashime, M.J. Johnson, K. Itakura, and R.B. Wallace [1981] ICN-UCLA Symp. Dev. Biol. Using Purified Genes, D.D. Brown [ed.], Academic Press, New York, 23:683-693).

Washes can be carried out as follows:

1) Twice at room temperature for 15 minutes 1x SSPE, 0.1% SDS (low stringency wash).
2) Once at the hybridization temperature for 15 minutes in 1x SSPE, 0.1% SDS (moderate stringency wash).

In general, salt and/or temperature can be altered to change stringency. With a labeled DNA fragment >70 or so bases in length, the following conditions can be used:

| | |
|---|---|
| Low: | 1 or 2x SSPE, room temperature |
| Low: | 1 or 2x SSPE, 42° C |
| Moderate: | 0.2x or 1x SSPE, 65° C |
| High: | 0.1x SSPE, 65° C. |

Duplex formation and stability depend on substantial complementarity between the two strands of a hybrid, and, as noted above, a certain degree of mismatch can be tolerated. Therefore, the probe sequences of the subject invention include mutations (both single and multiple), deletions, insertions of the described sequences, and combinations thereof, wherein said mutations, insertions and deletions permit formation of stable hybrids with the target polynucleotide of interest. Mutations, insertions, and deletions can be produced in a given polynucleotide sequence in many ways, and these methods are known to an ordinarily skilled artisan. Other methods may become known in the future.

Because of the degeneracy/redundancy of the genetic code, a variety of different DNA sequences can encode the amino acid sequences disclosed herein. It is well within the skill of a person trained in the art to create alternative DNA sequences that encode the same, or essentially the same, enzymes. These variantDNA sequences are within the scope of the subject invention.

The subject invention include, for example:

1) proteins obtained from wild type organisms;
2) variants arising from mutations;
3) variants designed by making conservative amino acid substitutions; and
4) variants produced by random fragmentation and reassembly of a plurality of different sequences that encode the subject TC proteins (DNA shuffling). *See e.g.* U.S. Patent No. 5,605,793.

The DNA sequences encoding the subject proteins can be wild type sequences, mutant sequences, or synthetic sequences designed to express a predetermined protein. DNA sequences designed to be highly expressed in plants by, for example, avoiding polyadenylation signals, and using plant preferred codons, are particularly useful.

Certain proteins and genes have been specifically exemplified herein. As these proteins and genes are merely exemplary, it should be readily apparent that the subject invention comprises use of variant or equivalent proteins (and nucleotide sequences coding for equivalents thereof) having the same or similar functionality as the exemplified proteins. Equivalent proteins will have amino acid similarity (and/or homology) with an exemplified enzyme (or active fragment thereof). Preferred polynucleotides and proteins of the subject invention can be defined in terms of narrower identity and/or similarity ranges. For example, the identity and/or similarity of the enzymatic protein can be 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, or 99% as compared to a sequence exemplified or suggested herein. Any number listed above can be used to define the upper and lower limits. For example, a protein of the subject invention can be defined as having 50-90% identity, for example, with an exemplified protein.

Unless otherwise specified, as used herein, percent sequence identity and/or similarity of two nucleic acids is determined using the algorithm of Karlin and Altschul (1990), Proc. Natl. Acad. Sci. USA 87:2264-2268, modified as in Karlin and Altschul (1993), Proc. Natl. Acad. Sci. USA 90:5873-5877. Such an algorithm is incorporated into the NBLAST and XBLAST programs of Altschul et al. (1990), J. Mol. Biol. 215:402-410. BLAST nucleotide searches are performed with the NBLAST program, score = 100, wordlength=12. Gapped BLAST can be used as described in Altschul et al. (1997), Nucl. Acids Res. 25:3389-3402. When utilizing BLAST and Gapped BLAST programs, the default parameters of the respective programs (NBLAST and XBLAST) are used. *See* NCBI/NIH website.

To obtain gapped alignments for comparison purposes, the AlignX function of Vector NTI Suite 8 (InforMax, Inc., North Bethesda, MD, U.S.A.), can be used employing the default parameters. Typically these would be a Gap opening penalty of 15, a Gap extension penalty of 6.66, and a Gap separation penalty range of 8. Two or more sequences can be aligned and compared in this manner or using other techniques that are well-known in the art. By analyzing such alignments, relatively conserved and non-conserved areas of the subject polypeptides can be identified. This can be useful for, for example, assessing whether changing a polypeptide sequence by modifying or substituting one or more amino acid residues can be expected to be tolerated.

The amino acid homology/similarity/identity will typically (but not necessarily) be highest in regions of the protein that account for its activity or that are involved in the determination of three-dimensional configurations that are ultimately responsible for the activity. In this regard, certain amino acid substitutions are acceptable and can be expected to be tolerated. For example, these substitutions can be in regions of the protein that are not critical to activity. Analyzing the crystal structure of a protein, and software-based protein structure modeling, can be used to identify regions of a protein that can be modified (using site-directed mutagenesis, shuffling, etc.) to actually change the properties and/or increase the functionality of the protein.

Various properties and three-dimensional features of the protein can also be changed without adversely affecting the activity/functionality of the protein. Conservative amino acid substitutions can be expected to be tolerated/to not adversely affect the three-dimensional configuration of the molecule. Amino acids can be placed in the following classes: non-polar, uncharged polar, basic, and acidic. Conservative substitutions whereby an amino acid of one class is replaced with another amino acid of the same type fall within the scope of the subject invention so long as the substitution is not adverse to the biological activity of the compound. The following list provides examples of amino acids belonging to each class.

| Class of Amino Acid | Examples of Amino Acids |
|---|---|
| Nonpolar | Ala, Val, Leu, Ile, Pro, Met, Phe, Trp |
| Uncharged Polar | Gly, Ser, Thr, Cys, Tyr, Asn, Gln |
| Acidic | Asp, Glu |
| Basic | Lys, Arg, His |

In some instances, non-conservative substitutions can also be made. The critical factor is that these substitutions must not significantly detract from the functional/biological/enzymatic activity of the protein.

To obtain high expression of heterologous genes in plants, for example, it may be preferred to reengineer said genes so that they are more efficiently expressed in plant cells. Sequences can be designed for optimized expression in plants, generally, or they can be desigened for optimized expression in a specific type of plant. Canola is one such plant where it may be preferred to re-design the heterologous gene(s) prior to transformation to increase the expression level thereof in said plant. Therefore, an additional step in the design of genes encoding a fungal protein, for example, is reengineering of a heterologous gene for optimal expression in a different type of organism. Guidance regarding the production of synthetic genes that are optimized for plant expression can be found in, for example, U.S. Patent No. 5,380,831. A sequence optimized for expression in plants is exemplified herein as **SEQ ID NO:1** (which encodes the exemplified protein, as shown in **SEQ ID NO:5).**

As used herein, reference to "isolated" polynucleotides and/or proteins, and "purified" proteins refers to these molecules when they are not associated with the other molecules with which they would be found in nature. Thus, reference to "isolated" and/or "purified" signifies the involvement of the "hand of man" as described herein. For example, a fungal polynucleotide (or "gene") of the subject invention put into a plant for expression is an "isolated polynucleotide." Likewise, a protein of the subject invention when produced by a plant is an "isolated protein."

A "recombinant" molecule refers to a molecule that has been recombined. When made in reference to a nucleic acid molecule, the term refers to a molecule that is comprised of nucleic acid sequences that are joined together by means of molecular biological techniques. The term "recombinant" when made in reference to a protein or a polypeptide refers to a protein molecule that is produced using one or more recombinant nucleic acid molecules.

The term "heterologous" when made in reference to a nucleic acid sequence refers to a nucleotide sequence that is ligated to, or is manipulated to become ligated to, a nucleic acid sequence to which it is not joined in nature, or to which it is joined at a different location in nature. The term "heterologous" therefore indicates that the nucleic acid molecule has been manipulated using genetic engineering, *i.e.* by human intervention. Thus, a gene of the subj ect invention can be operably linked to a heterologous promoter (or a "transcriptional regulatory region" which means a nucleotide sequence capable of mediating or modulating transcription of a nucleotide sequence of interest, when the transcriptional regulatory region is operably linked to the sequence of interest). Preferred heterologous promoters can be plant promoters. A promoter and/or a transcriptional regulatory region and a sequence of interest are "operably linked" when the sequences are functionally connected so as to permit transcription of the sequence of interest to be mediated or modulated by the transcriptional regulatory region. In some embodiments, to be operably linked, a transcriptional regulatory region may be located on the same strand as the sequence of interest. The transcriptional regulatory region may in some embodiments be located 5' of the sequence of interest. In such embodiments, the transcriptional regulatory region maybe directly 5' of the sequence of interest or there may be intervening sequences between these regions. The operable linkage of the transcriptional regulatory region and the sequence of interest may require appropriate molecules (such as transgenic activator proteins) to be bound to the transcriptional regulatory region, the invention therefore encompasses embodiments in which such molecules are provided, either *in vitro* or *in vivo.*

There are a number of methods for obtaining the proteins for use according to the subject invention. For example, antibodies to the proteins disclosed herein can be used to identify and isolate other proteins from a mixture. Specifically, antibodies may be raised to the portions of the proteins that are most constant and most distinct from other proteins. These antibodies can then be used to specifically identify equivalent proteins with the characteristic activity by immunoprecipitation, enzyme linked immunosorbent assay (ELISA), or immuno-blotting. Antibodies to the proteins disclosed herein, or to equivalent proteins, or to fragments of these proteins, can be readily prepared using standard procedures. Such antibodies are an aspect of the subject invention.

A protein "from" or "obtainable from" any of the subj ect isolates referred to or suggested herein means that the protein (or a similar protein) can be obtained from the exemplified isolate or some other source, such as another fungal or bacterial strain, or a plant (for example, a plant engineered to produce the protein). "Derived from" also has this connotation, and includes polynucleotides (and proteins) obtainable from a given type of fungus or bacterium, for example, wherein the polynucleotide is modified for expression in a plant, for example. One skilled in the art will readily recognize that, given the disclosure of a fungal gene and protein, a plant can be engineered to produce the protein. Antibody preparations, nucleic acid probes (DNA and RNA, for example), and the like maybe prepared using the polynucleotide and/or amino acid sequences disclosed herein and used to screen and recover other protein genes from other (natural) sources.

Oils of the subject invention retain a high degree of oxidative stability but contain lower levels of saturated fatty acids and higher levels of unsaturated fatty acids. Preferred oils of the subject invention have less than 3.5% total saturated fatty acid content, oleic content of at least 75% (and preferably and surprisingly less than 80%), and apoly unsaturated fatty acid content of less than 20% (and more preferably less than 15%, still more preferably less than 10%, and even more preferably less than 9%). The subject invention can also be used to achieve canola seed having total saturated fatty acid content (C:14, C:16, C:18, C:20, C:22, and C:24) of not more than (and preferably less than) 2.5% of the total fatty acid content, preferably with the oleic acid ranges as mentioned above). 18:2 and 18:3 levels, which contribute to oil instability, are not increased or are preferably reduced (for food applications). End points for ranges for any of these particular fatty acids, any combinations thereof, and particularly for either one or both of the C18 polyunsaturates, can be obtained from any of the Figures and Tables provided herein.

The subject invention can be used to provide agronomically elite canola seed that results in a refined/deodorized oil with less than 3.5% total saturates. Oil derived from these plants can be used to formulate various end products, or they can be used as stand-alone frying oil for "no sat" (or "low sat") products.

Unless indicated otherwise, the saturated fatty acid content of a given collection of canola seeds can be determined by standard procedures wherein the oil is removed from the seeds by crushing the seeds and is extracted as fatty acid methyl esters following reaction with methanol and sodium hydroxide. The resulting ester is then analyzed for fatty acid content by gas liquid chromatography using a capillary column which allows separation on the basis of the degree of unsaturation and chain length. This analysis procedure is described in, for example, J. K. Daun et al., J. Amer. Oil Chem. Soc. 60: 1751-1754 (1983).

The fatty acid composition of canola seed was determined as described below for either "half-seed" analysis, "single/whole seed" analysis, or "bulk seed" analyses. For "half-seed" analyses, a portion of cotyledonary tissue from the embryo was removed and analyzed; the remaining seed was then saved, and could be germinated if desired. Although the half-seed technique can be somewhat unreliable in selecting stable genetically controlled fatty acid mutations (and subsequent breeding and crosses), the subject invention demonstrates that preferred genes can be introduced and used to create stable lines. Unlike uncharacterized mutations, it is well known in the art that a gene can be introduced and stably maintained in plants. Thus, the analysis set forth herein demonstrates the utility of the subject genes and that canola oil having the indicated characteristics can be attained.

"No saturates" (*i.e.,* No Sat) levels of fatty acids were reached in seeds from transgenic lines derived from commercial Nexera 710 (canola) germplasm. The No Sat level is defined as less than 3.5% combined saturates. In addition, reduced saturates were seen in both the Westar canola line, and another Crucifer (*Arabidopsis*) with the same transformation construct. Notably, saturate levels in single seeds were down to 2.6 to 2.7% for some seeds. The subject invention can also be used to produce seeds with 2.5% or less total saturates. This is important because oil processing can add~0.5-1% to the total saturate "score," meaning that the processed oil product can still measurably reach the FDA-defined No Sat level using standard testing procedures. Having this level of tolerance not only permits for some levels of contamination (by higher saturate seeds) of testing equipment (especially if the plant operator does a poor job of keeping seed batches distinct), but also permits for some level of variation in field growth conditions (such as high temperatures, which tend to create more saturates) and cross-pollination by pollen drifting from unimproved canola in adjacent fields (which dilutes desirable genes).

The U.S. Food and Drug Administration defines "saturated fat" as "A statement of the number of grams of saturated fat in a serving defined as the sum of all fatty acids containing no double bonds." 21 CFR 101.9(c)(2)(i). Unless otherwise specified, this is the definition used herein for "total saturates" and "total saturated fat." A serving of a food product is considered to have "no saturated fat" if the product "contain[s] less than 0.5 gram of total fat in a serving." 21 CFR 101.9(c)(2)(i). "Total fat" is defined as "A statement of the number of grams of total fat in a serving defined as total lipid fatty acids and expressed as triglycerides." 21 CFR 101.9(c)(2). "Serving sizes" for various types of foods are defined in 21 CFR 101.12(b), which defines a serving of oil as 1 tablespoon or 15 ml. As used herein, this is understood to mean 14 grams. Thus, "no sat" canola oil (or canola oil comprising no saturated fat) is defined herein as canola oil having less than 0.5 grams of total saturated fat in a serving (14 grams of canola oil comprising 14 grams of fat). Stated another way, "no sat" canola oil comprises less than 3.57% total saturates (0.5 grams of total saturates divided by 14 grams of total fat). Unless specified otherwise, all percent fatty acids herein are percent by weight of the oil of which the fatty acid is a component.

As shown herein, the subj ect invention can surprisingly be used to obtain oil from canola, seeds wherein said oil comprises less than 3.57% total saturates. Oil can be obtained from the subject seeds using procedures that are well-known in the art, as mentioned in the preceding paragraphs, and the oil can be assayed for content using well-known techniques, including the techniques exemplified herein. Unless otherwise specified, analysis that was used to generate half-seed oils data and field oils data used a base-catalyzed transesterification reaction (AOCS Ce 2-66, alternative method). The protocol is similar to the saponification/acid esterification protocol described herein, except the saponification/acid esterification protocol measure total lipids, of which the majority are the same fatty acids from triacylglycerides detected by the base-catalyzed transesterification reaction.

In the commercial Nexera 710 germplasm, levels of 18:3 fatty acids, those that contribute to oxidative instability, were relatively unchanged. Thus, the subject invention not only provides plants, seeds, and oils with lower saturated fat, but also plants, seeds, and oils that very surprisingly maintain other beneficial characteristics. That is, the plants and genes of the subj ect invention can surprisingly be used without adversely affecting other advantageous characteristics of the plants.

In preferred embodiments, the subject invention provides plants comprising more than one expressed copy of a delta-9 desaturase gene of the subject invention. Results presented herein show that expressing multiple copies of this gene surprisingly improved the fatty acid profile of canola plants (saturated fat levels were greatly reduced). This is surprising in part because the art was heretofore unpredictable regarding the expression of multiple copies of the same gene. "Gene silencing" is one known phenomenon that teaches against using multiple copies (inserted at different locations in the genome, for example) of a heterologous gene. It is also not ideal to attempt to obtain multiple transformation events. Thus, there was no motivation to produce plants comprising more than one (two, three, four, and the like) delta-9 desaturase event. There was also no expectation that such plants would actually have improved characteristics.

Two examples of Cruciferous plants are specifically exemplified herein: *Brassica napus* (canola) and *Arabidopsis.* However, as is known in the art, other *Brassica* species and other Crucifers can be used for, for example, breeding and developing desired traits in canola and the like. Other such plants that can thus be used according to the subject invention include *Brassica rapa, Brassica juncea, Brassica carinata, Brassica nigra, Brassica oleracea, Raphanus sativus,* and *Sinapis alba.* Soybeans, soybean plants, and soybean oil can also be tested for improvement according to the subject invention.

In preferred embodiments, delta-9 desaturase genes of the subject invention are optimized for plant expression. Thus, the subject invention also provides a plant-optimized delta-9 desaturase gene. Optimization exemplified herein included introducing preferred codons and a Kozak translational initiator region, and removing unwanted sequences. The gene was driven by the beta-phaseolin promoter (a strong dicot seed storage protein promoter).

Promoters for which expression coincides with oil synthesis (*e.g.* ACP, elongase) can be used to further reduce saturates, as expression occurs earlier than for storage proteins. (Prior tobacco constructs used the nos 3' UTR, and prior corn constructs used the constitutive maize Ubiquitin-1 promoter and nos 3' UTR) Other dicot seed promoters can be used according to the subject invention, including vicilin, lectin, cruciferin, glycinin, and conglycinin promoters, plant seed promoters disclosed in US20030005485 A1, elongase promoters in US20030159173 A1, and the ACP promoter in U.S. Patent No. 5,767,363. *See also,* for example, US6100450A (seed specific, expesses in embryo, column 8 line 8); US20030159173A1 (section 0044 seed specific promoter; examples are USP, hordein, ACP, napin, FatB3, and FatB4); WO9218634A1 (introduction discusses seed-specific promoters from other patents pages 1 through 7; WO0116340A1 (page 7 line 13 provides a definition of a "seed specific" promoter, which typically expresses at less than 5% in other tissues; page 10 lines 19-29 discusses seed storage proteins like albumins, globulins, vicilin and legumin-like proteins, non-storage oleosins, promoters associated with fatty acid metabolism like ACP, saturases, desaturases, elongases); WO2003014347A2 (promoter definition p23-25: preferably 2x greater for seed-specific); US20030233677A1 (section 0033 provides "seed promoter" examples [napin, ACCase, 2S albumin, phaseolin, oleosin, zein, glutelin, starch synthase, starch branching enzyme]); WO2003092361A2 (page 15 provides a definition for "promoter"; the top of page 17 provides promoter examples and patent references (storage proteins only) including zeins, 7S storage proteins, Brazil nut protein, phe-free protein, albumin, beta-conglycinin, 11S, alpha-hordothionin, arcelin, lectins, glutenin); US20030148300 A1 (see Claim 8, including the napin promoter, the phaseolin promoter, the soybean trypsin inhibitor promoter, the ACP promoter, stearoyl-ACP desaturase promoter, the soy 7S promoter, the oleosin promoter, the conglycinin promoter, oleosin promoters, embryogenesis-abundant protein promoters, embryo globulin promoters, arcelin 5, the napin promoter, and the acid chitinase promoter); U.S. Patent No. 5,777,201 (column 6, lines 30-50, constitutive promoters, seed- and/or developmentally regulated promoters *e.g.* plant fatty acid lipid biosynthesis genes [ACPs, acyltransferases, desaturases, lipid transfer proteins] or seed promoters [napin, cruciferin, conglycinin, lectins] or inducible promoters [light, heat, wound inducers]).

The plastids of higher plants are an attractive target for genetic engineering. Chloroplast (a type of plastid) transformation has been achieved and is advantageous. *See e.g.* U.S. Patent Nos. 5,932,479; 6,004,782; and 6,642,053. *See also* U.S. Patent Nos. 5,693,507 and 6,680,426. Advantages of transformation of the chloroplast genome include: potential environmental safety because transformed chloroplasts are only maternally inherited and thus are not transmitted by pollen out crossing to other plants; the possibility of achieving high copy number of foreign genes; and reduction in plant energy costs because importation of proteins into chloroplasts, which is highly energy dependent, is reduced.

Plant plastids (chloroplasts, amyloplasts, elaioplasts, etioplasts, chromoplasts, *etc.*) are the major biosynthetic centers that, in addition to photosynthesis, are responsible for producing many industrially important compounds such as amino acids, complex carbohydrates, fatty acids, and pigments. Plastids are derived from a common precursor known as a proplastid; thus, the plastids in a given plant species all have the same genetic content.

Plastids of most plants are maternally inherited. Consequently, unlike heterologous genes expressed in the nucleus, heterologous genes expressed in plastids are not disseminated in pollen. Therefore, a trait introduced into a plant plastid will not be transmitted to wild-type relatives. This offers an advantage for genetic engineering of plants for tolerance or resistance to natural or chemical conditions, such as herbicide tolerance, as these traits will not be transmitted to wild-type relatives.

The plastid genome (plastome) of higher plants is a circular double-stranded DNA molecule of 120-160 kb which may be present in 1,900-50,000 copies per leaf cell (Palmer, 1991). In general, plant cells contain 500-10,000 copies of a small 120-160 kilobase circular genome, each molecule of which has a large (approximately 25 kb) inverted repeat. Thus, it is possible to engineer plant cells to contain up to 20,000 copies of a particular gene of interest; this can potentially result in very high levels of foreign gene expression.

Oils of the subject invention are applicable for, and can be specially tailored for, industrial as well as various food uses. Aside from cooking oil, itself, the subject invention also includes "no sat" products such as potato chips and the like (*see* U.S. Patent No. 6,689,409, which claims a fried food composition comprising potatoes and a canola oil; the subject invention, however, can be used to improve the compositions described in the ’409 patent).

Plants of the subject invention can be crossed with other plants to achieve various desirable combinations of characteristics and traits. Even further improvements can be made by crossing the subject plants, using known breeding technique and other advantageous sources of germplasm such as other canola lines having additional or other beneficial traits and characteristics. Another example would be crosses with a line having a plastidial delta-9 desaturase.

Thus, the subject invention can be used to achieve less than 3.5% total saturated fatty acids in commercial oil under variable environmental conditions (and less than 3% total saturated fatty acids in seed oil in breeder seed). This can be accomplished with no reduction in the quality and quantity of storage proteins, with no increase in indigestible fiber in canola meal, and no negative impact on seed yield (or other desirable agronomic traits) per acre.

Following is a list of the common names of fatty acids, as used herein, together with their number of carbon atoms and double bonds. Saturated fats have zero double bonds.

**Table 1.**

| Name | Number of Carbon Atoms Per Molecule | Number of Double Bonds Per Molecule |
|---|---|---|
| Lauric | 12 | 0 |
| Myristic | 14 | 0 |
| Palmitic | 16 | 0 |
| Palmitoleic | 16 | 1 |
| Stearic | 18 | 0 |

(continued)

| Name | Number of Carbon Atoms Per Molecule | Number of Double Bonds Per Molecule |
|---|---|---|
| Oleic* | 18 | 1 |
| Vaccenic** | 18 | 1 |
| Linoleic | 18 | 2 |
| Alpha-Linolenic | 18 | 3 |
| Arachidonic | 20 | 0 |
| Eicosenoic (or Arachidic) | 20 | 1 |
| Behenic | 22 | 0 |
| Erucic | 22 | 1 |
| Lignoceric | 24 | 0 |
| Nervonic | 24 | 1 |

* = double bond at delta-9 position
** = double bond at delta-11 position

All patents, patent applications, provisional applications, and publications referred to or cited herein are incorporated by reference in their entirety to the extent they are not inconsistent with the explicit teachings of this specification.

Unless specifically indicated or implied, the terms "a", "an", and "the" signify "at least one" as used herein.

Following are examples that illustrate procedures for practicing the invention. These examples should not be construed as limiting. All percentages are by weight and all solvent mixture proportions are by volume unless otherwise noted.

Example 1 - Delta-9 desaturase gene rebuilding

A delta-9 desaturase gene of the subject invention was redesigned for plant expression through a combination of changing *Aspergillus nidulans* sequence to plant-preferred translational codons, introducing unique restriction enzyme sites, and removing unwanted sequences and some secondary structure. The redesigned gene was synthesized by Operon, Inc. The sequence of the open reading frame for this polynucleotide is provided here as **SEQ ID NO:1**. The sequence of the ORF preceded by a Kozak sequence and a BamHI cloning site (caps), plus a translational terminator at the end of the ORF (caps), is provided in **SEQ ID NO:2.**

Example 2 - Delta-9 desaturase plant transformation vector construction

The BamHI-BstEll gene fragment was cloned into a vector between the Pv beta-phaseolin promoter and Pv beta-phaseolin 3' UTR (pPhas-UTR). This construct was named pOIL. The promoter-gene-UTR fragment was excised from pOIL by digestion with NotI, blunted, and cloned into the blunt PmeI site of vector pOEA1. The final vector was named pPD9-OEA1.

Example 3 - Plant transformation with pPD9-OEA1

Plasmid vector pPD9-OEA1 was transformed into *Agrobacterium tumefaciens* [strain C58GV3101 (C58C1RifR) pMP90 (GmR). Koncz and Schell, *Mol. Gen. Genet* (1986)]. The delta 9-desaturase plants were then obtained by *Agrobacterium tumefaciens* mediated plant transformation

*Arabidopsis* was transformed with the "dip method," a procedure well known in the art. Plants were selfed, and dried seed was collected for FAME (fatty acid methyl ester) analysis.

The protocol used for canola transformation was as described by Katavic [Katavic, Campbell, L., Friesen, L., Palmer, D., Keller, W., and Taylor, D.C. (1996), "Agrobacterium-mediated genetic transformation of selected high erucic acid B. napus cultivars," 4th Ganadian Plant Tissue Culture and Genetic Engineering Conference, Saskatoon, SK, June 1-4,1996], with modifications for DAS's Nexera line. Hypocotyl sections were isolated from 6-day-old seedlings *of B. napus,* cv Westar or Nexera 710 and were cultured on callus initiation medium prior to transformation. On the day of transformation, the hypocotyls were coincubated with an *Agrobacterium* culture containing the plasmid pPD9-OEA1 with the trait gene such that a fragment of plasmid DNA including the delta 9-desaturase gene was incorporated into the cell chromosome. After a co-cultivation period, the hypocotyls were transferred to callus initiation medium containing glufosinate ammonium as the selection agent. Healthy, resistant callus tissue was obtained and repeatedly transferred to fresh selection medium for approximately 12 to 16 weeks. Plants were regenerated and transferred to Convirons growth chambers. Plants were selfed to obtain seed. If transgenic plants were sterile, they were crossed with pollen from unmodified Nexera 710 lines. Dry seed was harvested for FAME analysis.

Example 4 - Canola Event Sorting Process, and Summary of Canola Results

Unless otherwise specified, the following procedures were used to obtain the Nex 710/Delta-9 canola seeds, the data for which is presented in subsequent Examples.

In general, there were four main steps for developing, selecting, or sorting events: sorting initial transgenic events, sorting T1 and T2 seed, sorting T1 or T2 plants, and sorting events by field performance. Transformed callus was first regenerated to T0 plants.

For the initial sorting, 107 putative transgenics were screened by agronomics and by southern blotting (simple or complex, i.e., more than 3 copies). Multiple seeds per event were screened. T1 seed saturates were determined, C16:1/C16:0 ratios were determined (to infer catalytic efficiency), and segregation of biochemical phenotypes were determined. Based on these data, and seed availability (timing, amount), a limited subset of seed was advanced.

For sorting T1 and T2 seed (a few events were advanced by one generation), half-seed analysis was conducted, and possible homozygotes were identified. Based on this data, half-seeds from segregating populations were selected for greenhouse growth.

The next main step was sorting T1 or T2 plants (a few events were advanced one generation). Southerns were conducted to determine transgene integration complexity. Zygosity was also determined by INVADER assays (Third Wave Technologies, Inc.). These data were used to select seed for field trials.

For T1 greenhouse studies, 30 seeds per event were subjected to half-seed analysis. Nex 710 canola was used as a commercial check. All individuals within the event were zygosity sampled to determine allele copy number for Delta-9 and PAT. This was followed by PCR and indoleacetamide hydrolase (IAAH; a negative scoreable or screenable marker) analysis. All individuals within the event were leaf painted to determine the PAT segregation ratio. Southern analysis was then conducted on those individuals approaching a "no sat" profile, positive IAAH, and homozygous for PAT and Delta-9.

These were then used for T2 analysis (100 seeds from each of the above plants were half-seed analyzed). INVADER was used to verify copy number and to see if the event was segregating for D9 and PAT. Leaf painting was used to see if lines are segregating for PAT. 10 seed bulk fatty acid data was collected from plants based on half-seed data, INVADER results, LP, and IAAH-positive.

The fourth main step was sorting events by field performance (plant T2 or T3 seed, analyze T2 or T3 plants, then T3 or T4 seed). There was a wide sampling of transgenic events. Agronomics, Southerns, and zygosity were analyzed. Batch seed oils analysis was also conducted. Based on these data, events were selected for crossing to increase gene dosage.

The following selection criteria was used to advance lines to field studies. 224 lines (including nulls) from 23 events (6 reps/entry) were evaluated in replicated nurseries at 3 locations. 6 T3 and 17 T2 events were planted. Selection of lines/event going to field was based on insert number and half-seed analysis followed by a 10-seed bulk fatty acid analysis of seed from each plant. The percent total sat range of selected lines was in the approximate range of 3.3-4.5%. The following T3 events were selected for further development:

**Table 2A.**

| Event | Copy Number | # of Lines Field Tested |
|---|---|---|
| 218-11.30 | 2 D9:2 PAT | 45 |
| 36-11.19 | 2 D9:2 PAT | 7 |
| 31a-3.30.01 | 1 D9:1 PAT | 15 |
| 146-11.19 | 3 D9:1 + partial PAT | 23 |
| 159a-11.19 | 2 D9:2 PAT | 19 |
| 69-11.19 | 2 D9:2 PAT | 20 |

The following T2 events were selected for further development:

**Table 2B.**

| Event | Copy Number | # of Lines Field Tested |
|---|---|---|
| 146-11.19 | nd (not determined) | 6 |
| 149-11.30 | nd | 8 |
| 15-11.19 | nd | 4 |

(continued)

| Event | Copy Number | # of Lines Field Tested |
|---|---|---|
| 224-11.30 | 3 D9:2.5 PAT | 10 |
| 226-11.30 | nd | 4 |
| 230-11.30 | nd | 2 |
| 250-11.19 | nd | 5 |
| 267-11.19 | nd | 5 |
| 284-11.19 | nd | 8 |
| 309-11.30 | nd | 2 |
| 32-11.30 | nd | 3 |
| 324-11.30 | nd | 8 |
| 43-11.19 | nd | 5 |
| 43b-11.30 | 2 D9:2 PAT | 10 |
| 57-11.30 | nd | 5 |
| 68-11.30 | 2 D9:2 PAT | 8 |
| 96a-6.15 | nd | 2 |

Field tests were conducted as follows. Agronomic assessments were taken as discussed in a subsequent example to confirm that no agronomic penalty was associated with the Delta-9 (D9). 15 INVADER leaf tissue samples were collected from 28 of the most promising T3 lines (plus sib-nulls) for further copy number verification and to determine if lines were segregating for PAT. The D9 lines were chosen based on having less than 3.5% total saturates.

10 Southern tissue samples were taken from the 3 most promising T2 lines, which were chosen based on having less than 3.5% total saturates. All tissue-sampled plants were self-pollinated. Fatty acid analysis was determined based on 10 seed bulk from selfed plants (455 samples), and 1 gram of bulk seed sample from OP rows (1445).

The "best" T4 events are as follows:

**Table 3A.**

| Event | Copy Number | # of Lines Field Tested |
|---|---|---|
| 218-11.30 | 2 D9:2 PAT | 9 |
| 36-11.19 | 2 D9:2 PAT | 2 |
| 146-11.19 | 3 D9:1+partial PAT | 4 |
| 159a-11.19 | 2 D9:1 PAT | 1 |
| 69-11.19 | 2 D9:2 PAT | 3 |

The "best" T3 events are as follows:

**Table 3B.**

| Event | Copy Number | # of Lines Field Tested |
|---|---|---|
| 149-11.30 | nd (not determined) | 3 |
| 43b-11.30 | 2 D9:2 PAT | 1 |
| 57-11.30 | nd | 2 |
| 284-11.19 | nd | 2 |

Generally, no major agronomic penalty associated with Delta-9 was observed, and some lines exhibited an approximately 10% increase in seed weight. Agronomic results are discussed in more detail below in **Example 18.** General observations regarding distributions of individual fatty acid components are discussed below in **Examples 11-13.** Summaries of mean TSAT data, % changes in TSATs, and % changes for certain fatty acid components for events 218-11.30,36-11.19, and 69-11.19 are presented in **Tables 4-7.** Generally, ~30% to ~40% reductions in TSATs were observed, relative to the sib-null and wild type. However, even further improvements are discussed in more detail below and can be made with further crosses, for example.

**Table 4. Mean TSAT For All Lines from Event 218-11.30 Across 3 Sites**

| | Event | N | C16:0 %Total | C16:1 %Total | C18:0 % Total | C18:1 %Total | C18:2 % Total | C18:3 %Total | %Total Saturates |
|---|---|---|---|---|---|---|---|---|---|
| Selfs | Null | 94 | 3.75 | 0.38 | 1.67 | 76.57 | 11.65 | 2.50 | 6.62 |
| | #218-11.30 | 217 | 3.10 | 1.40 | 0.68 | 79.11 | 10.89 | 2.29 | 4.37 |
| | | **%Wt** | **86%** | **385%** | **38%** | **101%** | **105%** | **98%** | **66%** |
| | | **%Null** | **83%** | **365%** | **40%** | **103%** | **93%** | **92%** | **66%** |
| Open Pollinated | | | | | | | | | |
| | Null | 3 | 3.70 | 0.47 | 1.65 | 78.07 | 10.65 | 2.40 | 6.45 |
| | Wt Control | 88 | 3.60 | 0.36 | 1.79 | 78.19 | 10.37 | 2.34 | 6.60 |
| | #218-11.30 | 123 | 3.07 | 1.23 | 0.74 | 80.17 | 10.15 | 2.22 | 4.42 |
| | | **%Wt** | **85%** | **338%** | **41%** | **103%** | **98%** | **95%** | **67%** |
| | | **%Null** | **83%** | **263%** | **45%** | **103%** | **95%** | **92%** | **68%** |

**Table 5. % Changes in TSATS C16:0, C16:1, C18:0 And C18:1 VS Nulls and WT Control Nex 710**

| Event | Line | 16:0 | C16: 1 | C18: 0 | C18:1 | TSAT | % TSAT **Null** | vs **WT** | % C16: 0 VsN | % C16:1 Vs N | % C18:0 Vs N |
|---|---|---|---|---|---|---|---|---|---|---|---|
| 218-11.30 (TS) | 1361 | 2.87 | 1.31 | 0.64 | 80.23 | 4.04 | 39 | 37 | 22 | 317 | 61 |
| 218-11.30 (TS) | 1319 | 2.88 | 1.41 | 0.62 | 79.53 | 4.09 | 38 | 37 | 20 | 248 | 62 |
| 218-11.30 (TS) | 1304 | 2.95 | 1.33 | 0.60 | 79.16 | 4.10 | 38 | 36 | 18 | 230 | 63 |
| 218-11.30 (TS) | 1500 | 2.95 | 1.31 | 0.63 | 79.58 | 4.11 | 38 | 36 | 18 | 224 | 61 |
| 218-11.30 (TS) | 1405 | 3.04 | 1.40 | 0.60 | 80.44 | 4.17 | 37 | 35 | 16 | 245 | 63 |
| 218-11.30 (TS) | 1370 | 3.02 | 1.38 | 0.66 | 80.24 | 4.24 | 36 | 34 | 17 | 240 | 59 |
| 218-11.30 (TS) | 1369 | 3.03 | 1.30 | 0.65 | 79.44 | 4.25 | 36 | 34 | 16 | 220 | 59 |
| 218-11.30 (T) | 1370 | 2.96 | 1.20 | 0.77 | 80.28 | 4.31 | 31 | 33 | 18 | 196 | 53 |
| 218-11.30 (T) | 1405 | 3.01 | 1.19 | 0.71 | 78.65 | 4.31 | 31 | 33 | 17 | 193 | 56 |
| **218-11.30 (N)** | **1299** | **3.62** | **0.41** | **1.61** | **78.10** | **6.26** | . | . | . | . | . |
| **218-11.30 (NS)** | **1299** | **3.70** | **0.32** | **1.65** | **77.56** | **6.52** | . | . | . | . | . |
| **Nex 710** | **.** | **3.58** | **0.35** | **1.75** | **77.87** | **6.45** | . | . | . | . | . |

**Table 6. % Changes in TSATS C16:0, C16:1, C18:0 And C18:1 VS Nulls and WT Control Nex 710**

| Event | Line | C16: 0 | C16: 1 | C18: 0 | C18:1 | TSAT | % TSAT | **WT** | % C16:0 VsN | % C16:1 VsN | % C18:0 VsN |
|---|---|---|---|---|---|---|---|---|---|---|---|
| 36-11.19 (T) | 1099 | 2.93 | 1.23 | 0.77 | 78.85 | 4.30 | 32 | 33 | 16 | 322 | 55 |
| **36-11.19 (N)** | **1127** | **3.49** | **0.29** | **1.70** | **77.14** | **6.36** | **.** | **.** | **.** | **.** | **.** |

(continued)

| Event | Line | C16:0 | C16:1 | C18:0 | C18:1 | TSAT | % TSAT | **WT** | % C16:0 VsN | % C16:1 VsN | % C18:0 VsN |
|---|---|---|---|---|---|---|---|---|---|---|---|
| **Nex 710** | **.** | **3.58** | **0.35** | **1.75** | **77.87** | **6.45** | **.** | **.** | **.** | **.** | **.** |

**Table 7. % Changes in TSATS C16:0, C16:1, C18:0 And C18:1 VS Nulls and WT Control Nex 710**

| Event | Line | C16:0 | C16:1 | C18:0 | C18:1 | TSAT | % TSAT | **WT** | % C16:0 Vs N | % C16:1 Vs N | % C18:0 Vs N |
|---|---|---|---|---|---|---|---|---|---|---|---|
| 69-11.19 (T) | 1538 | 3.05 | 1.05 | 0.71 | 80.70 | 4.21 | 34 | 35 | 15 | 223 | 59 |
| 69-11.19 (T) | 1529 | 3.02 | 1.02 | 0.72 | 80.61 | 4.24 | 33 | 34 | 16 | 213 | 58 |
| 69-11.19 (T) | 1534 | 3.09 | 1.02 | 0.73 | 80.45 | 4.29 | 32 | 34 | 14 | 213 | 57 |
| **69-11.19 (N)** | **1604** | **3.58** | **0.33** | **1.72** | **78.45** | **6.34** | · | · | · | · | · |
| **Nex 710** | **.** | **3.58** | **0.35** | **1.75** | **77.87** | **6.45** | · | · | · | · | · |

Example 5 - Molecular characterization of plants

Leaf samples were taken for DNA analysis to verify the presence of the transgenes by PCR and Southern analysis, and occasionally to confirm expression of PAT protein by ELISA.

5A. Protocol for PCR analysis for delta-9 desaturase. The following two primers were used:

Delta-9 forward B:

5' TGA GTT CAT CTC GAG TTC ATG 3' (**SEQ ID NO:3**)

Delta-9 reverse B:

5' GAT CCA ACA ATG TCT GCT CC 3' (**SEQ ID NO:4**)

This primer pair yields a~1380 bp fragment after amplifying the delta-9 gene.

The following cycling protocol was used in this screen with an MJ Tetrad thermal cycler:

1. 94° C, 2 minutes
2. 94° C, 1 minutes
3. 50° C, 2 minutes
4. 72° C, 3 minutes, + 5 seconds / cycle extension
5. repeat Steps 2-4 25 times
6. 4° C until ready for analysis, or at least 2 minutes

5B. Protocol for the extraction of plant genomic DNA for Southern analysis. The DNeasy Plant Maxi Kit from Qiagen was used. The protocol in the booklet was used with the following changes to the elution part. Buffer AE was diluted 1:10 with DNA grade water (Fisher No. BP561-1). Two elutions were performed using 0.75ml of the diluted AE buffer pre-warmed to 65° C. DNA was precipitated with isopropanol and washed with 70% ethanol. The DNA pellet was resuspended in 100 μl of 1X TE buffer. DNA concentration was quantitated. 6 μg of DNA was aliquoted and adjusted to a final volume of 40 μl. Samples were stored at -20° C.

5C. FAME analysis (Direct FAME Synthesis from Seeds with Methanolic $H_2SO_4$

The protocol for FAME analysis was as follows.

GC Specs

Gas Chromatograph: Hewlett-Packard 6890 with dual injection ports and dual flame ionization detectors.
Data System: HP Chemstation, Leap Technologies, Carrboro, NC 27510, PAL System.
Column: J&W capillary column, DB-23, 60M x 0.25mm i.d. with 0.15 microfilm thickness, maximum operating temperature 250° C. Catalog number: 122-2361.
Temperature profile: Equilibration time: 1 minute. Initial temperature: 50° C. Initial time: 3 minutes. Increase rate: 40° C/minute. Final temperature: 240° C. Final time: 7.25 minute.

FAME procedures for Arabidopsis. Add 100 μl (50 μg) **15:0 Standard** into a clean 16x125 mm glass tube (Internal Standard stock solution: 500 μg/ml of C15:0 TAG in 2:1 chloroform:isopropanol). Dry Standard under nitrogen in evaporation water bath at 55° C.

When dry add 2 ml 1N methanolic $H_2SO_4$ with 2% DMP (for 100 ml: 95,22 ml methanol, 2.772 ml $H_2SO_4$, 2 ml DMP = 2,2-dimethoxypropane). Heat tube to 85° C.

Weigh ~5 mg Arabidopsis seeds into clean 16x125 mm glass tube and record exact weight.

To destroy lipases, add hot meth. $H_2SO_4$ with Standard to tube with seeds, incubate for 15 minutes at 85° C.

Cool vial down to ~50° C, then crush seeds with glass pestle in mini grinder.

Transfer sample back into tube and incubate for at least one hour under nitrogen at 85° C. Cool vial on ice. Add first 0.5 ml 0.9% NaCl, then 250 μl **17:0 Standard** in hexane (0.1335 mg/ml methyl ester stock solution). Vortex, centrifuge at 1000g for 5 minutes.

Transfer 100-200 μl with Pasteur pipette into 1.5 ml vial with conical insert (0.5 ml).

Inject 5 μl into GC.

FAME procedures for Canola. Add 100 μl (50 μg) **15:0 Standard** into a clean 16x125 mm glass tube (Internal Standard stock solution: 500 μg/ml of C15:0 TAG in 2:1 chloroform:isopropanol). Dry Standard under nitrogen in evaporation water bath at 55° C.

When dry add 2 ml 1N methanolic $H_2SO_4$ with 2% DMP (for 100ml.: 95,22 ml methanol, 2.772 ml $H_2SO_4$, 2 ml DMP = 2,2-dimethoxypropane). Heat tube to 85° C.

Weigh one canola seed in clean tube and record exact weight.

To destroy lipases, add seed sample to tube containing Standard with hot methanol $H_2SO_4$, incubate for 15 minutes at 85° C.

Cool vials down to ~50° C, then crush seeds with glass pestle.

Incubate for at least 1 hour under $N_2$ at 85° C.

Cool vial on ice. Add first 0.5 ml 0.9% NaCl, then 250 μl **17:0 Standard** in hexane (0.1335 mg/ml methyl ester stock solution). Vortex, centrifuge at 1000g for 5 minutes.

Transfer 100-200 μl with Pasteur pipette into 1.5 ml vial with conical insert (0.5 ml).

Inject 5 μl into GC.

Example 6 - Arabidopsis Results

Initial results are illustrated in **Figure 1,** showing that a greater than 60% reduction of saturated fatty acids was achieved. Also, more 16:1 (5.9% for example) than 16:0 (4.4%) was achieved.

No Sat Oil via Δ9-CoA-Desaturase Approach

*FAME analysis*

T2 seeds from approximately 18 additional transformants were analyzed. This data (see **Table 8** and **Figure 2**) show a reduction in "sats" of up to 60-70%. This is an even stronger reduction in saturated fatty acids than the initial data (see **Figure 1**) indicated. It is important to note that the T2 generation is still segregating; thus, even better performing lines in following generations are expected. This point is true for all T1, T2, T3, and other initial generations (including canola lines) as reported elsewhere herein, until the trait is fixed and the line is homozygous for the transgene. (Stable lines and plants where the traits are fixed were produced and are described in subsequent Examples.)

| | 16:0 | 16:1 | 18:0 | 18:1 | 18:2 | 18:3 | 20:0 | 20:1? | ? | ? | ? | 22:0 | 22:1 | 24:0 | Tot Sats |
|---|---|---|---|---|---|---|---|---|---|---|---|---|---|---|---|
| **WT1** | 7.8 | 0.2 | 3.2 | 11.2 | 27.3 | 21.4 | 2.8 | 20.1 | 2.3 | 0.7 | 0.0 | 0.4 | 2.4 | 0.1 | 14.4 |
| **TF-21** | 7.2 | 0.3 | 3.3 | 14.4 | 27.9 | 18.0 | 2.5 | 21.4 | 2.1 | 0.5 | 0.0 | 0.4 | 2.0 | 0.0 | 13.3 |
| **WT1-3** | 7.2 | 0.2 | 3.0 | 12.1 | 26.5 | 21.9 | 2.6 | 20.8 | 2.3 | 0.7 | 0.0 | 0.4 | 2.3 | 0.0 | 13.2 |
| **WT1-2** | 6.9 | 0.2 | 2.9 | 13.7 | 27.5 | 20.9 | 2.5 | 20.2 | 2.1 | 0.6 | 0.0 | 0.4 | 2.2 | 0.0 | 12.7 |
| **TF-10** | 5.5 | 3.0 | 2.0 | 19.5 | 28.7 | 18.4 | 1.3 | 17.9 | 1.6 | 0.4 | 0.0 | 0.3 | 1.3 | 0.0 | 9.1 |
| **TF-18** | 6.1 | 1.9 | 1.4 | 18.0 | 29.0 | 19.0 | 0.9 | 19.6 | 1.9 | 0.5 | 0.0 | 0.2 | 1.5 | 0.0 | 8.7 |
| **TF-13** | 4.7 | 2.2 | 1.6 | 19.9 | 27.9 | 19.1 | 1.3 | 19.2 | 1.8 | 0.4 | 0.0 | 0.3 | 1.5 | 0.0 | 7.9 |
| **TF-22** | 4.6 | 2.6 | 1.6 | 20.2 | 27.9 | 18.6 | 1.3 | 19.4 | 1.7 | 0.4 | 0.0 | 0.3 | 1.5 | 0.0 | 7.8 |
| **TF-12** | 5.5 | 2.1 | 1.2 | 18.6 | 28.4 | 20.1 | 0.8 | 19.4 | 1.8 | 0.5 | 0.0 | 0.2 | 1.5 | 0.0 | 7.6 |
| **TF-17** | 3.4 | 1.1 | 1.9 | 19.5 | 27.5 | 17.3 | 1.8 | 22.8 | 2.0 | 0.5 | 0.0 | 0.5 | 1.8 | 0.0 | 7.5 |
| **TF-14** | 5.0 | 2.1 | 1.4 | 18.8 | 27.3 | 21.4 | 1.0 | 19.2 | 1.7 | 0.5 | 0.0 | 0.0 | 1.6 | 0.0 | 7.4 |
| **TF-8** | 3.2 | 0.9 | 1.6 | 19.0 | 25.3 | 18.7 | 1.8 | 24.7 | 2.0 | 0.5 | 0.0 | 0.0 | 2.2 | 0.0 | 6.6 |
| **TF-19** | 4.1 | 2.7 | 1.0 | 20.7 | 28.4 | 19.9 | 0.8 | 18.8 | 1.7 | 0.4 | 0.0 | 0.2 | 1.4 | 0.0 | 6.0 |
| **TF-20** | 4.4 | 2.8 | 0.6 | 20.2 | 28.8 | 19.7 | 0.5 | 19.3 | 1.7 | 0.4 | 0.0 | 0.1 | 1.4 | 0.0 | 5.7 |
| **TF-7** | 5.0 | 2.9 | 0.0 | 20.8 | 27.5 | 25.8 | 0.0 | 15.3 | 1.3 | 0.0 | 0.0 | 0.0 | 1.4 | 0.0 | 5.0 |
| **TF-6** | 3.4 | 1.6 | 0.7 | 22.4 | 27.9 | 17.9 | 0.7 | 21.4 | 1.9 | 0.4 | 0.0 | 0.0 | 1.7 | 0.0 | 4.8 |
| **TF-11** | 3.4 | 2.7 | 0.6 | 22.4 | 28.8 | 19.7 | 0.5 | 18.5 | 1.6 | 0.4 | 0.0 | 0.2 | 1.3 | 0.0 | 4.7 |

**Table 8.**

Values are from a single sample prep and GC run (not averages)
TF-21 behaves as a wild-type (non-transformed) plant; possible explanations include gene silencing or non-transgenic escape (inadequate selection with glufosinate herbicide)
"?" indicates that identity of the peak on the GC chromatogram is questionable, or unknown

Example 7 - Westar data

Protocols similar to those described in Example 5C were applied to canola lines derived from well-known "Westar" canola. As illustrated in **Figure 3,** the indicated saturated fats were reduced by over 43%, and a 50% reduction was achieved when 24:0 was included.

Example 8 - Exemplary Nexera 710 data

Protocols similar to those described elsewhere herein were applied to canola lines derived from well-known "Nexera 710" commercially elite canola. Total saturates were calculated used methodology discussed herein and as specified below.

Total saturates are derived from the sum of 16:0 + 18:0 + 20:0 + 22:0 + 24:0 fatty acids. Some notable saturate levels in single seeds are presented in Table 9. Oil profiles are presented as mol% values. The mol% value incorporates the formula weight of each specific fatty acid into the calculation. It uses the mass of a given fatty acid species (peak area, or the same value used to directly calculate % fatty acid), divided by the formula weight for that fatty acid species.

**Table 9.**

| Seeds | Saturate Level |
|---|---|
| Event 5 11.19 seed #6 | 3.1% |
| Event 5 11.19 #8 | 2.7% |
| Event 113a 11.19 #4 | 3.4% |
| Event 113a 11.19 #8 | 3.2% |
| Event 147 11.19 #3 | 3.0% |
| Event 147 11.19 #7 | 2.6% |
| Event 36a 11.19 seed | 2.7% |

(continued)

| Table 9. | |
|---|---|
| **Seeds** | **Saturate Level** |
| Event (9)3 11.30 | 3.3% |

Profiles from the seeds with the lowest total saturates were analyzed (seeds 113a 11.19 #4, 113a 11.19 #8, 5 11.19 #6 and 5 11.19 #8). Unmodified Nexera 710 germplasm values came from the same FAME analysis run. Plants were grown in the Convirons growth chamber, so actual mol% values may differ from field grown seed. In general, transgenic plants show a reduction in 16:0, 18:0 and 20:0, and increases in 16:1. 18:0 levels generally fell from an average of 1.4% (upper 2.08%, lower 0.81%) in Nexera 710 to 0.1% average (upper 0.6%, lower 0%) in select transgenic material. Also, 16:0 levels fell from an average of 4.6% (upper 5.12% to lower 4%) to 3.0% (upper 3.41% to lower 2.63%). Likewise, the 20:0 levels dropped from 0.5% average in Nexera 710 to 0% in the selected transgenics. The 16:1 levels were undetectable in Nexera 710, increasing to an average of 2.3 % (upper 2.71% to lower 1.72%) in transgenics. The average 18:3 levels were slightly increased in the small transgenic population, but the range of values overlapped with the unmodified Nexera 710 samples. These results can be summarized as follows:

| Table 10. | | |
|---|---|---|
| **Fatty Acid** | **Nexera 710** | **Select Transgenic Material** |
| 20:0 | 0.5% average | 0% |
| 18:0 | 1.4% average (upper 2.08%, lower 0.81%) | 0.1% average (upper 0.6%, lower 0%) |
| 16:0 | 4.6% average (upper 5.12% to lower 4%) | 3.0% average (upper 3.41% to lower 2.63%) |
| 16:1 | Undetectable | 2.3% average (upper 2.71% to lower 1.72%) |

Example 9 - Further Canola Data

Protocols similar to those described elsewhere herein were applied to additional canola lines derived from well-known "Nexera 710" commercially elite canola. Total saturates and the weight percent of the individual types of fatty acids, as indicated below, were calculated using methodology discussed herein.

**Figures 4A-C and 5A-C** show representative results, from Events 36-11.19 and 218-11.30 respectively, that demonstrate reduced saturated fatty levels that are obtainable by practicing the subject invention. By making further manipulations according to the subject invention, the saturated fat levels exemplified here can be even further reduced. All of this data were obtained from selfed transgenic canola plants as indicated.

In summary, for Event 36-11.19, T2 half seed analysis from greenhouse-grown plants had total saturates as low as 2.57%. Total saturates for T3 whole seeds, from greenhouse-grown plants, were as low as 3.66%. Results are shown graphically in **Figure 4A** (numerical data are in **Figures 4B and 4C**). For Event 218-11.30 greenhouse-grown plants, T2 half seed analysis revealed total saturates to be as low as 2.71%. T3 whole seeds had total saturates as low as 3.37%. Results are shown graphically in **Figure 5A** (numerical data are in **Figures 5B and 5C**). For reference, NATREON has 6.5% total saturates, on average, under field conditions.

By making further improvements according to the subject invention (such as additional rounds of selfing, crosses with other superior lines, increasing desaturase gene copy number [either by additional transformation, by further breeding crosses, and the like], changing timing of desaturase expression, and mutagenesis), even greater levels of reduction of total saturated fat levels can be achieved.

Example 10 - Analysis of Further Canola Data - Percent reduction of total saturated fats

The data presented in Example 9 can be used in various calculations to illustrate various aspects of the subject invention. For example, percent reduction of total saturated fats can be calculated by first dividing the total saturates of a given plant by the total saturates of the control line, and then subtracting from 100%. Examples of such reductions, provided by the subject invention, are illustrated below. Results can be approximated by rounding to the closest whole (non-decimal) number.

**Table 11.**

| Event (& generation) | Total Sats (TS) | Control TS | % Reduction |
|---|---|---|---|
| 218-11.30 (T2) | 2.71 | 6.36 | 57.4% |
| 36-11.19 (T2) | 2.57 | 6.44 | ~60% |

Any number shown on any of the graphs, figures, tables, or otherwise discussed herein can be used as an endpoint to define the metes and bounds of the subject invention. Likewise, any calculations using any of these numbers, such as those shown above and those discussed in more detail below, can be used to define the metes and bounds of the subject invention. Tables 12-14 show further representative results and calculations, for Lines with Events 218-11.30, 36-11.19, and 69-11.19.

**Table 12. Event 218-11.30 HS Selections for Crossing**

| Line | C16:0 | C16:1 | C18:0 | C18:1 | TSAT | % ↓TSAT VS Null | WT | % ↓C16:0 Vs N | % ↑C16:1 Vs N | % ↓C18:0 Vs N |
|---|---|---|---|---|---|---|---|---|---|---|
| 2193HS50 | 2.05 | 1.54 | 0.33 | 81.34 | 2.66 | 54 | 53 | 43 | 611 | 75 |
| 2193HS9 | 2.27 | 1.76 | 0.27 | 75.02 | 2.81 | 51 | 50 | 37 | 709 | 79 |
| 2183HS22 | 2.31 | 1.31 | 0.29 | 81.16 | 2.87 | 50 | 49 | 36 | 505 | 78 |
| 2193HS23 | 2.29 | 1.47 | 0.28 | 77.17 | 2.89 | 50 | 49 | 36 | 577 | 79 |
| 2195(N) | 3.60 | 0.22 | 1.32 | 76.25 | 5.75 | . | . | . | . | . |
| Nex 710 | 3.33 | 0.18 | 1.51 | 77.39 | 5.61 | . | . | . | . | . |

**Table 13. Event 36-11.19 HS Selections for Crossing**

| Line | C16:0 | C16:1 | C18:0 | C18:1 | TSAT | % ↓TSAT VS Null | WT 48 | % ↓C16:0 Vs N | % tC16:1 Vs N | % ↓C18:0 VsN |
|---|---|---|---|---|---|---|---|---|---|---|
| 1099HS3 | 1.95 | 1.76 | 0.52 | 81.92 | 2.92 | 51 | | 45 | 1018 | 61 |
| 1099HS8 | 2.19 | 2.05 | 0.40 | 77.81 | 2.97 | 50 | 47 | 39 | 1201 | 70 |
| 1099HS17 | 2.11 | 1.73 | 0.42 | 80.16 | 2.99 | 50 | 47 | 41 | 1000 | 69 |
| 1099HS11 | 2.13 | 1.73 | 0.45 | 79.30 | 3.00 | 49 | 47 | 40 | 998 | 66 |
| 1099HS43 | 2.18 | 1.72 | 0.41 | 77.88 | 3.01 | 49 | 46 | 39 | 991 | 69 |
| 1127(N) | 3.57 | 0.16 | 1.34 | 74.21 | 5.92 | . | . | . | . | . |
| Nex 710 | 3.33 | 0.18 | 1.51 | 77.39 | 5.61 | . | . | . | . | . |

**Table 14. Event 69-11.19 HS Selections for Crossing**

| Line | C16:0 | C16:1 | C18:0 | C18:1 | TSAT | % ↓TSAT Null | VS WT | % ↓C16:0 Vs N | % ↑C16:1 Vs N | % ↓C18:0 Vs N |
|---|---|---|---|---|---|---|---|---|---|---|
| 1538HS23 | 1.88 | 2.20 | 0.34 | 79.56 | 2.64 | 55 | 53 | 49 | 1101 | 74 |
| 1538HS26 | 2.18 | 1.60 | 0.29 | 77.55 | 2.81 | 52 | 50 | 41 | 776 | 78 |
| 1538HS4 | 2.20 | 1.71 | 0.35 | 80.17 | 2.90 | 51 | 48 | 41 | 831 | 73 |
| 1538HS36 | 2.17 | 1.49 | 0.37 | 78.24 | 2.93 | 50 | 48 | 41 | 713 | 72 |
| 1604(N) | 3.70 | 0.18 | 1.29 | 78.12 | 5.88 | . | . | . | . | . |
| Nex 710 | 3.33 | 0.18 | 1.51 | 77.39 | 5.61 | . | . | . | . | . |

Example 11 - Analysis of Further Canola Data - Detailed fatty acid profiles

Again, **Figures 4A-C and 5A-C** show some representative results that show fatty acid profiles of various plants having events 218-11.30 and 36-11.19. Generally, these results demonstrate that not only are the 16:0 and 18:0 levels greatly reduced (with a resulting increase in corresponding unsaturated levels), but the 20:0, 22:0, and 24:0 levels are also advantageously, and surprisingly and unexpectedly, reduced. In some cases, 18:2 and 18:3 levels can also be reduced, which enhances the oxidative stability of the improved oil. Furthermore, any of the ratios suggested above (such as 16:0-16:1, 18:0-18:1, and, for example, 18:0-[20:0+22:0+24:0]) can be used to define advantageous results of practicing the subj ect invention. Combined percent reductions in total C20:0 + C22:0 + C24:0 are also surprisingly achieved according to the subject invention. Thus, the subject invention provides plants have advantageous and improved fatty acid profiles, as exemplified herein. By making further improvements according to the subject invention, even better reductions in saturates, increases in "no sats," and better ratios can be achieved.

For example, various calculations, using the following data from **Figure 5C** and **Figure 4B,** can be used to illustrate accomplishments of the subject invention. Sample data from **Figure 5C** and **Figure 4B** are presented in the following Table. The amount of each indicated fatty acid is indicated for each event and in parentheses for the relevant control plant(s).

**Table 15.**

| Event (& generation) | C20:0 (control) | C22:0 (control) | C24:0 (control) |
|---|---|---|---|
| 218-11.30 (T2) | 0.11 (0.62) | 0.12 (0.32) | 0.03 (0.14) |
| 36-11.19 (T3) | 0.32 (0.64) | 0.15 (0.42) | 0.04 (0.21) |

Looking at the 218-11.30 event, the total contribution to saturates by the C20:0, C22:0, and C24:0 components is 1.08% in the control, but these components are advantageously decreased to 0.26% in a canola line of the subject invention. This represents an over 4-fold decrease in these saturates. Likewise, each component can be considered individually. Again looking at the 218-11.30 event, the C20:0 component is 0.62% in the control/wild-type, while it is reduced about 5.6 times in the plant line of the subject invention (down to 0.11%). The C22:0 component is reduced about 2 2/3 times: 0.12% in the d-9 desaturase plant line, which is down from 0.32% in the control line that lacks the desaturase gene. C24:0 is reduced about 4 2/3 times, from 0.14% down to 0.03 %.

For Event 36 (or 36-11.19), two lines have C20:0, C22:0, and C24:0 content of 0.32, 0.15, and 0.04, and 0.30,0.14, and 0.07, respectively. Compared to the control having 0.64,0.42, and 0.21 respectively, these lines have about half the C20:0, and at least about a three-fold reduction in C22:0 and C24:0. The first line mentioned above actually exhibits an over 5X reduction in C24:0.

It will quickly become apparent that a great number of similar calculations can be made for any of the other lines of the subject invention, for any of these preferred fatty acid components. These illustrations should not be construed as limiting, and any such novel reductions and ratios can be used to define the subject invention.

Example 12 - Further Half-Seed Data of Subsequent Generations

Further half-seed FAME analysis is set forth in **Table 16.** This Figure shows total saturates as low as 2.64% in a T3 generation and 2.66% in a T4 generation. **Table 17** shows the copy number of D-9 desaturase genes present in the respective lines (see Sample ID in **Table 16** and ID column in **Table 17**). Effects of copy number are discussed in more detail below in Examples 14 and 19.

Table. 16.

| | | | HALF-SEED FAME ANALYSIS - % of Total Oil | | | | | | | |
|---|---|---|---|---|---|---|---|---|---|---|
| EVENT | Generation | Sample ID: | C12:0 | C14:0 | C16:0 | C16:1 | C18:0 | C18:1 | C18:2 | C18:3 |
| 69-11.19 (HL) | T3 | 03TGH01538HS23 | nd | 0.04 | 1.88 | 2.20 | 0.34 | 79.56 | 9.43 | 3.29 |
| 218-11.30 (HL) | T4 | 03TGH02193HS50 | nd | 0.05 | 2.05 | 1.54 | 0.33 | 81.34 | 9.85 | 2.77 |
| 69-11.19 (HL) | T3 | 03TGH01538HS26 | nd | 0.05 | 2.18 | 1.60 | 0.29 | 77.55 | 11.78 | 4.01 |
| 218-11.30 (HL) | T4 | 03TGH02193HS9 | nd | nd | 2.27 | 1.76 | 0.27 | 75.02 | 14.44 | 3.08 |
| 218-11.30 (HL) | T4 | 03TGH02193HS22 | 0.01 | 0.05 | 2.31 | 1.31 | 0.29 | 81.16 | 10.30 | 2.72 |
| 218-11.30 (HL) | T4 | 03TGH02193HS23 | 0.01 | 0.06 | 2.29 | 1.47 | 0.28 | 77.17 | 13.57 | 3.15 |
| 69-11.19 (HL) | T3 | 03TGH01538HS4 | nd | 0.03 | 2.20 | 1.71 | 0.35 | 80.17 | 10.19 | 3.07 |
| 36-11.19 (HL) | T3 | 03TGH01099HS3 | nd | 0.04 | 1.95 | 1.76 | 0.52 | 81.92 | 8.61 | 3.03 |
| 69-11.19 (HL) | T3 | 03TGH01538HS36 | nd | 0.04 | 2.17 | 1.49 | 0.37 | 78.24 | 11.54 | 3.39 |
| 218-11.30 (HL) | T4 | 03TGH02193HS2 | nd | 0.06 | 2.31 | 1.60 | 0.30 | 77.39 | 13.23 | 2.98 |
| 69-11.19 (HL) | T3 | 03TGH01538HS40 | nd | 0.03 | 2.26 | 1.46 | 0.38 | 79.75 | 10.51 | 3.44 |
| 36-11.19 (HL) | T3 | 03TGH01099HS8 | nd | 0.05 | 2.19 | 2.05 | 0.40 | 77.81 | 12.22 | 2.86 |
| 36-11.19 (HL) | T3 | 03TGH01099HS17 | nd | 0.06 | 2.11 | 1.73 | 0.42 | 80.16 | 10.37 | 2.73 |
| 36-11.19 (HL) | T3 | 03TGH01099HS11 | nd | 0.06 | 2.13 | 1.73 | 0.45 | 79.30 | 10.98 | 3.04 |
| 36-11.19 (HL) | T3 | 03TGH01099HS43 | nd | 0.06 | 2.18 | 1.72 | 0.41 | 77.88 | 12.09 | 3.06 |
| 218-11.30 (HL) | T4 | 03TGH02194HS37 | nd | 0.06 | 2.25 | 1.34 | 0.44 | 83.00 | 8.52 | 2.50 |
| 218-11.30 (HL) | T4 | 03TGH02194HS27 | nd | 0.08 | 2.31 | 1.26 | 0.41 | 79.74 | 11.23 | 3.22 |
| 218-11.30 (HL) | T4 | 03TGH02194HS17 | nd | 0.06 | 2.30 | 1.34 | 0.40 | 79.75 | 11.15 | 3.19 |
| 218-11.30 (HL) | T4 | 03TGH02194HS2 | nd | 0.06 | 2.25 | 1.22 | 0.44 | 81.50 | 9.84 | 2.79 |
| 218-11.30 (HL) | T4 | 03TGH02194HS15 | nd | 0.07 | 2.35 | 1.32 | 0.40 | 78.85 | 12.00 | 3.29 |
| 218-11.30 (N) | T4 | 03TGH02195HS3 | 0.01 | 0.06 | 3.60 | 0.23 | 1.37 | 76.94 | 12.52 | 2.69 |
| 218-11.30 (N) | T4 | 03TGH02195HS9 | nd | 0.05 | 3.33 | 0.17 | 1.35 | 77.76 | 12.01 | 2.86 |
| 218-11.30 (N) | T4 | 03TGH02195HS13 | nd | 0.06 | 3.76 | 0.21 | 1.13 | 76.48 | 13.23 | 2.62 |
| 218-11.30 (N) | T4 | 03TGH02195HS16 | 0.01 | 0.05 | 3.31 | 0.20 | 1.35 | 75.89 | 13.05 | 3.82 |
| 218-11.30 (N) | T4 | 03TGH02195HS19 | nd | 0.06 | 4.02 | 0.27 | 1.43 | 74.20 | 14.43 | 2.90 |
| 36-11.19 (N) | T3 | 03TGH01127HS1 | nd | 0.05 | 3.46 | 0.18 | 1.42 | 74.06 | 14.50 | 3.69 |
| 36-11.19 (N) | T3 | 03TGH01127HS2 | nd | 0.06 | 3.53 | 0.14 | 1.30 | 73.14 | 15.34 | 3.37 |
| 36-11.19 (N) | T3 | 03TGH01127HS4 | nd | 0.06 | 3.74 | 0.16 | 1.29 | 71.92 | 16.49 | 3.38 |
| 36-11.19 (N) | T3 | 03TGH01127HS8 | nd | 0.04 | 3.59 | 0.14 | 1.28 | 75.09 | 13.69 | 2.67 |

Table. 16.

| | | | HALF-SEED FAME ANALYSIS - % of Total Oil | | | | | | | |
|---|---|---|---|---|---|---|---|---|---|---|
| EVENT | Generation | Sample ID: | C12:0 | C14:0 | C16:0 | C16:1 | C18:0 | C18:1 | C18:2 | C18:3 |
| 36-11.19 (N) | T3 | 03TGH01127HS18 | nd | 0.04 | 3.55 | 0.18 | 1.41 | 76.85 | 12.00 | 2.67 |
| WT Control | | M94S010HS6 | nd | 0.06 | 3.36 | 0.17 | 1.45 | 76.60 | 12.52 | 3.36 |
| WT Control | | M94S010HS12 | nd | 0.06 | 3.19 | 0.21 | 1.58 | 74.56 | 13.88 | 4.31 |
| WT Control | | M94S010HS15 | nd | 0.03 | 3.37 | 0.16 | 1.62 | 78.68 | 10.07 | 3.26 |
| WT Control | | M94S010HS17 | nd | 0.05 | 3.21 | 0.19 | 1.54 | 77.37 | 12.21 | 2.97 |
| WT Control | | M94S010HS18 | nd | 0.07 | 3.54 | 0.18 | 1.37 | 79.73 | 9.91 | 2.35 |
| 69-11.19(N) | T3 | 03TGH01604HS2 | nd | 0.06 | 3.77 | 0.15 | 1.19 | 80.15 | 9.04 | 2.60 |
| 69-11.19(N) | T3 | 03TGH01604HS6 | nd | 0.05 | 3.74 | 0.24 | 1.22 | 75.57 | 13.27 | 3.23 |
| 69-11.19(N) | T3 | 03TGH01604HS8 | nd | 0.05 | 3.66 | 0.11 | 1.35 | 80.52 | 8.74 | 2.64 |
| 69-11.19(N) | T3 | 03TGH01604HS9 | nd | 0.04 | 3.88 | 0.21 | 1.32 | 77.33 | 11.40 | 2.67 |
| 69-11.19(N) | T3 | 03TGH01604HS15 | nd | 0.05 | 3.48 | 0.21 | 1.37 | 77.05 | 12.00 | 3.38 |

Table 16 (continued).

| | | | HALF-SEED FAME ANALYSIS - % of Total Oil | | | | | | | |
|---|---|---|---|---|---|---|---|---|---|---|
| EVENT | C20:0 | C20:1 | C20:2 | C22:0 | C22:1 | C24:0 | C24:1 | TOTSAT | Selected | Leaf Paint Data |
| 69-11.19 (HL) | 0.18 | 0.81 | 0.03 | 0.10 | nd | 0.10 | 0.05 | 2.64 | Selected | Resistant |
| 218-11.30 (HL) | 0.19 | 0.82 | 0.04 | 0.04 | nd | nd | nd | 2.66 | Selected | Resistant |
| 69-11.19 (HL) | 0.20 | 0.84 | 0.06 | 0.08 | nd | 0.02 | 0.01 | 2.81 | Selected | Resistant |
| 218-11.30 (HL) | 0.18 | 0.78 | 0.10 | 0.05 | nd | 0.04 | 0.04 | 2.81 | Selected | Resistant |
| 218-11.30 (HL) | 0.15 | 0.77 | 0.04 | 0.06 | nd | 0.02 | 0.02 | 2.87 | Selected | Resistant |
| 218-11.30 (HL) | 0.17 | 0.73 | 0.06 | 0.06 | nd | 0.02 | nd | 2.89 | Selected | Resistant |
| 69-11.19 (HL) | 0.18 | 0.80 | 0.05 | 0.06 | nd | 0.09 | 0.05 | 2.90 | Selected | Resistant |
| 36-11.19 (HL) | 0.26 | 0.85 | 0.06 | 0.11 | nd | 0.04 | nd | 2.92 | Selected | Resistant |
| 69-11.19 (HL) | 0.19 | 0.85 | 0.08 | 0.12 | nd | 0.04 | nd | 2.93 | Selected | Resistant |
| 218-11.30 (HL) | 0.17 | 0.84 | 0.06 | 0.06 | nd | 0.05 | nd | 2.95 | Selected | Resistant |
| 69-11.19 (HL) | 0.21 | 0.84 | 0.04 | 0.08 | nd | nd | 0.03 | 2.96 | Selected | Resistant |
| 36-11.19 (HL) | 0.22 | 0.77 | 0.08 | 0.11 | nd | 0.01 | 0.02 | 2.97 | Selected | Resistant |
| 36-11.19 (HL) | 0.23 | 0.83 | 0.08 | 0.13 | nd | 0.04 | 0.09 | 2.99 | Selected | Resistant |
| 36-11.19 (HL) | 0.22 | 0.79 | 0.06 | 0.12 | nd | 0.02 | 0.07 | 3.00 | Selected | Resistant |

Table 16 (continued).

| | | | HALF-SEED FAME ANALYSIS - % of Total Oil | | | | | | | |
|---|---|---|---|---|---|---|---|---|---|---|
| EVENT | C20:0 | C20:1 | C20:2 | C22:0 | C22:1 | C24:0 | C24:1 | TOTSAT | Selected | Leaf Paint Data |
| 36-11.19 (HL) | 0.21 | 0.82 | 0.10 | 0.13 | nd | 0.01 | 0.07 | 3.01 | Selected | Resistant |
| 218-11.30 (HL) | 0.20 | 0.83 | 0.04 | 0.07 | nd | nd | nd | 3.03 | Selected | Resistant |
| 218-11.30 (HL) | 0.18 | 0.77 | 0.04 | 0.06 | nd | 0.01 | nd | 3.04 | Selected | Resistant |
| 218-11.30 (HL) | 0.19 | 0.76 | 0.05 | 0.07 | nd | 0.02 | nd | 3.05 | Selected | Resistant |
| 218-11.30 (HL) | 0.20 | 0.82 | 0.05 | 0.09 | nd | 0.03 | nd | 3.06 | Selected | Resistant |
| 218-11.30 (HL) | 0.18 | 0.75 | 0.04 | 0.06 | nd | 0.02 | nd | 3.08 | Selected | Resistant |
| 218-11.30 (N) | 0.52 | 1.16 | 0.05 | 0.23 | nd | 0.12 | nd | 5.90 | Selected null | Susceptible |
| 218-11.30 (N) | 0.42 | 1.21 | 0.05 | 0.18 | nd | 0.07 | 0.03 | 5.39 | Selected null | Susceptible |
| 218-11.30 (N) | 0.42 | 1.21 | 0.06 | 0.22 | nd | 0.08 | nd | 5.67 | Selected null | Susceptible |
| 218-11.30 (N) | 0.42 | 1.13 | 0.05 | 0.18 | nd | 0.07 | 0.03 | 5.39 | Selected null | Susceptible |
| 218-11.30 (N) | 0.52 | 1.23 | 0.06 | 0.27 | nd | 0.11 | 0.05 | 6.40 | Selected null | Susceptible |
| 36-11.19 (N) | 0.47 | 1.17 | 0.07 | 0.23 | 0.01 | 0.07 | 0.05 | 5.71 | Selected null | Susceptible |
| 36-11.19 (N) | 0.53 | 1.36 | 0.09 | 0.29 | nd | 0.13 | 0.07 | 5.84 | Selected null | Susceptible |
| 36-11.19 (N) | 0.50 | 1.35 | 0.08 | 0.27 | 0.02 | 0.09 | 0.06 | 5.96 | Selected null | Susceptible |
| 36-11.19 (N) | 0.59 | 1.45 | 0.07 | 0.36 | 0.04 | 0.15 | 0.09 | 6.01 | Selected null | Susceptible |
| 36-11.19 (N) | 0.61 | 1.41 | 0.08 | 0.35 | nd | 0.14 | 0.07 | 6.10 | Selected null | Susceptible |
| WT Control | 0.45 | 1.13 | 0.05 | 0.18 | nd | 0.06 | 0.06 | 5.54 | Selected null | Susceptible |
| WT Control | 0.41 | 0.97 | 0.06 | 0.15 | nd | nd | 0.04 | 5.39 | Selected null | Susceptible |
| WT Control | 0.51 | 1.09 | 0.07 | 0.22 | nd | 0.05 | nd | 5.80 | Selected null | Susceptible |
| WT Control | 0.46 | 1.17 | 0.07 | 0.18 | nd | 0.06 | nd | 5.50 | Selected null | Susceptible |
| WT Control | 0.52 | 1.31 | 0.05 | 0.29 | nd | 0.06 | 0.02 | 5.85 | Selected null | Susceptible |
| 69-11.19(N) | 0.51 | 1.39 | 0.04 | 0.29 | nd | 0.12 | nd | 5.93 | Selected null | Susceptible |
| 69-11.19(N) | 0.46 | 1.22 | 0.06 | 0.21 | nd | 0.08 | 0.08 | 5.74 | Selected null | Susceptible |
| 69-11.19(N) | 0.53 | 1.29 | 0.05 | 0.28 | nd | 0.09 | 0.10 | 5.96 | Selected null | Susceptible |
| 69-11.19(N) | 0.54 | 1.36 | 0.07 | 0.31 | 0.02 | 0.08 | 0.07 | 6.16 | Selected null | Susceptible |
| 69-11.19(N) | 0.45 | 1.12 | 0.06 | 0.21 | nd | 0.05 | 0.04 | 5.61 | Selected null | Susceptible |

**Table 17.**

| | | | | southern copy # | |
|---|---|---|---|---|---|
| **ID** | **Project** | **Event** | **Generation** | **D-9** | **PAT** |
| **03TGH02193HS50** | TG03D9-62 | **218-11.30 (HL)** | **T4** | 1 | 1.5 or 2 |
| **03TGH02193HS9** | TG03D9-62 | **218-11.30 (HL)** | **T4** | 1 | 1.5 or 2 |
| **03TGH02193HS22** | TG03D9-62 | **218-11.30 (HL)** | **T4** | 1 | 1.5 or 2 |
| **03TGH02193HS23** | TG03D9-62 | **218-11.30 (HL)** | **T4** | 1 | 1.5 or 2 |
| **03TGH02193HS2** | TG03D9-62 | **218-11.30 (HL)** | **T4** | 1 | 1.5 or 2 |
| **03TGH02194HS37** | TG03D9-62 | **218-11.30 (HL)** | **T4** | 1 | 1.5 or 2 |
| **03TGH02194HS27** | TG03D9-62 | **218-11.30 (HL)** | **T4** | 1 | 1.5 or 2 |
| **03TGH02194HS17** | TG03D9-62 | **218-11.30 (HL)** | **T4** | 1 | 1.5 or 2 |
| **03TGH02194HS2** | TG03D9-62 | **218-11.30 (HL)** | **T4** | 1 | 1.5 or 2 |
| **03TGH02194HS15** | TG03D9-62 | **218-11.30 (HL)** | **T4** | 1 | 1.5 or 2 |
| **03TGH02195HS9** | **TG03D9-62** | **218-11.30 (N)** | **T4** | 0 | 0 |
| **03TGH02195HS13** | **TG03D9-62** | **218-11.30 (N)** | **T4** | 0 | 0 |
| **03TGH02195HS16** | **TG03D9-62** | **218-11.30 (N)** | **T4** | 0 | 0 |
| **M94S010HS15** | TG03D9-62 | **WT Control** | | 0 | 0 |
| **M94S010HS15** | TG03D9-62 | **WT Control** | | 0 | 0 |
| **03TGH01099HS3** | **TG03D9-62** | **36-11.19 (HL)** | **T3** | 2 | 1.5 |
| **03TGH01099HS8** | **TG03D9-62** | **36-11.19 (HL)** | **T3** | 2 | 1.5 |
| **03TGH01099HS17** | **TG03D9-62** | **36-11.19 (HL)** | **T3** | 2 | 1.5 |
| **03TGH01099HS11** | **TG03D9-62** | **36-11.19 (HL)** | **T3** | 2 | 1.5 |
| **03TGRG1099HS43** | **TG03D9-62** | **36-11.19 (HL)** | **T3** | 2 | 1.5 |
| **03TGH01127HS2** | **TG03D9-62** | **36-11.19 (N)** | **T3** | 0 | 0 |
| **03TGH01127HS4** | **TG03D9-62** | **36-11.19 (N)** | **T3** | 0 | 0 |
| **03TGH01127HS8** | **TG03D9-62** | **36-11.19 (N)** | **T3** | 0 | 0 |
| **03TGH01538HS23** | TG03D9-62 | **69-11.19 (HL)** | **T3** | 2 | 1.5 |
| **03TGH01538HS26** | TG03D9-62 | **69-11.19 (HL)** | **T3** | 2 | 1.5 |
| **03TGH01538HS4** | TG03D9-62 | **69-11.19 (HL)** | **T3** | 2 | 1.5 |
| **03TGH01538HS36** | TG03D9-62 | **69-11.19 (HL)** | **T3** | 2 | 1.5 |
| **03TGH01538HS40** | **TG03D9-62** | **69-11.19 (HL)** | **T3** | 2 | 1.5 |
| **03TGH01604HS6** | **TG03D9-62** | **69-11.19(N)** | **T3** | 0 | 0 |
| **03TGHD16D4HS8** | **TG03D9-62** | **69-11.19(N)** | **T3** | 0 | 0 |
| **03TGH01604HS9** | **TG03D9-62** | **69-11.19(N)** | **T3** | 0 | 0 |
| **M94S010HS15** | TG03D9-62 | **WT Control** | | 0 | 0 |
| **M94S010HS15** | TG03D9-62 | **WT Control** | | 0 | 0 |

Example 13 - Further Analysis of Half-Seed Data from Example 12, and Further Data Showing Fatty Acid Shifts, Increases in Unsaturates, and Decreases in Saturates

Half-seed data from the T3 field trials was plotted to illustrate various comparisons of the fatty acid contents to the "total sat" data. **Figures 6A and 6B** clearly show the reductions in C16:0 and increases in C16:1 in the transgenic events as compared to the nulls (events with a non-functional insert) and wild-type controls (non-transformed lines). **Figures 6C and 6D** clearly show the reductions in C18:0 and increases in C18:1 in the transgenic events as compared to the nulls and wild-type controls. **Figures 6E and 6F** clearly show the reductions in C20:0 and C22:0, respectively, in the transgenic events as compared to the nulls and wild-type controls.

Similar results, using data obtained as discussed in Example 4, are also illustrated with bar graphs. **Figures 6G and 6H** clearly show shifts and reductions in C16:0, and shifts and increases in C16:1 in the transgenic events, as compared to the nulls and wild-type controls. **Figures 6I and 6J** clearly show shifts and reductions in C18:0, and shifts and increases in C18:1 in the transgenic events, as compared to the nulls and wild-type controls. **Figures 6K and 6L** show similar bar graphs for C18:2 and C18:3.

The plots discussed above and **Figure 6M** also clearly illustrate the very surprising reduction in total saturates, as compared to already very good Nex 710 lines. **Figure 6N** shows distributions for 1000 seeds.

Example 14 - Decreasing saturated fat levels with multiple Delta-9 desaturase genes

Results from greenhouse increases and field trials suggest that there is a relationship between the reduction in total saturates and Aspergillus delta-9 desaturase copy number.

14A: FAME Analysis Protocol for Event Sorting and Effect of Trans gene Copy Number

Sample preparation

1. Obtain weight of individual seeds and place into plastic mother plate.
2. Add 2, 1/8" balls to each well
3. Take mother plate to liquid handler Hamilton:

   add 400 µL of heptane with IS (C11:0) and surrogate (C15:0 FAEE) then add 100 µL of sodium methoxide (.5N)

4. Cap inserts with strips caps and Geno-grind for 5 minutes (1x @ 500)
5. Replace strip cap
6. Add plate lid with a rubber mat (extra sealing). Tape the lid with black electrical tape and remove bottom of mother plate to expose vials
7. Place the plate onto vortex/heater for 15 min. at 37°C / 60 rpm (the vortex wells are filled with sand)
8. Centrifuge plate @ 3500 rpm for 2 min.
9. Place the bottom lid back and remove lid/strip cap. Transfer 350 µl of top layer into the daughter plate using Hamilton.
10. Then add 400 µL of heptane with IS and surrogate to the extraction plate.
11. Repeat steps 5 to 10 two more times (for a total of 3 transfers)
12. Keep the extract plate on the Hamilton after last transfer. Transfer 50 µl of extract into glass insert mounted in aluminum block containing 450 µl heptane with C11
13. Inject on GC

GC/FID analysis

GC parameters:
Injection 1 ul splitless per sample

Column-

DB23, 15 meters, 0.25 mm I.D. and 0.15 µm film thickness

GC parameters-

Oven temperature program-
70°C hold for 2.15min (splitless)
70°C - 150° @ 25°C min.,
150°C -180° @ 5°C min.,
180-C - 220° @ 25°C min.,
220°C hold for 2 min.
Injector temp. - 230°C
Detector temp. - 240°C
Make-up gas - Nitrogen @ 25mL/ min.
FID fuel - air @ 400mL/ min.,
hydrogen @ 40mL/ min
front injector: purge time 1 min.
purge flow 35 ml/min
back injector: purge time 2.15 min.
purge flow 35 ml/min.
front inlet pressure: 1.0 mL/min - constant flow
back inlet pressure: 1.0 mL/min - constant flow
Run time - 14.95 minutes,
Flow rates - helium constant flow @ 1 mL/ min

Acquisition sequence

96 samples are splited between front and back column to minimize the build on the liner. The sample list is built with 5 injection methods corresponding to the type of sample being injected. The first 5 samples injected are always:

1. Matrix
2. Matrix
3. Standard1
4. Canola Positive Control
5. Reagent Blank
6-27. Canola samples
33. Standard2
34-54. Canola samples
55. Standard3

Each list comprises 3 events of 16 samples (48 samples).

Standard contains 25 ppm FAMES total distributed as follow:

**Table 18.**

| FAMEs | Concentration (ppm) | Added to calibration |
|---|---|---|
| C14:0 | 0.25 | Yes |
| C16:0 | 1 | Yes |
| C18:0 | 0.75 | Yes |
| C18:1 | 15 | Yes |
| C18:2 | 3 | Yes |
| C18:3 | 1.25 | Yes |
| C20:0 | 0.75 | Yes |
| C20:1 | 0.25 | Yes |
| C22:0 | 0.75 | Yes |
| C22:1 | 1.25 | No |
| C24:0 | 0.75 | Yes |

14B: Preliminary Southern Blot Analysis to Approximate Copy Number

**[0167]** The DNA preparation protocol used for these purposes was as follows. Approximately 6 micrograms of DNA was digested with HindIII, and digested DNA was run on a 0.75% agarose gel. Blotting onto positively charged nylon membrane and hybridization followed typical protocols (Maniatis, Roche Applied Science, Inc.). The probe consisted of a DIG-labeled (kit from Roche Applied Science, Inc.) PCR product derived from the Aspergillus delta-9 desaturase gene. Washes were done twice for 5 minutes in room-temperature 2XSSC/0.1% SDS, then twice in 65⁺C 0.1XSSC/0.1% SDS. Hybridized bands were visualized with the DIG-Luminescent Detection Kit according to manufacturer's guidelines (Roche Applied Science, Inc.). Hybridizing bands were counted, and transgenic samples were initially described as 'Simple' if they displayed 1 to 3 bands, or "Complex" if more than 3 bands.

14C: Comparison of Transgene Copy Number and Reduction in Saturated Fatty Acids in Transgenic Events

The following definitions apply to the Table:

| | |
|---|---|
| # **GC/FID analysis** | number of individual seeds that has been analyzed |
| # **seed C16:1 WT ratio <10%** | number of seeds with a ratio C16:1/C16:0*100 less than 10% |
| # **seed C16:1 intermediate ratio** | number of seeds with a ratio C16:1/C16:0*100 more than 10% (interpreted as hemizygote based on FAME phenotype) |
| # **seed C16:1 highest ratio** | number of seed with the highest ratio C16:1/C16:0*100 (interpreted as homozygote based on FAME phenotype) |
| **Ratio Sat in WT** | ratio of saturated FA in the wild type seed for that particular event. If not available (interpreted as complex event based on FAME phenotype), the null average of 7.5% was used.<br>WT: individual seeds whose sat:unsat ratio is <10%, which is similar to Null event seeds |
| **Ratio Sat in transgenic** | ratio of saturated FA in the homozygote seed for that particular event. If not available (complex event) used the average. |
| **Sat reduction (%)** | (saturated FA in wild type - saturated FA in transgenic) / saturated FA in wild type of that particular event. If saturated FA in wild type is not available (complex event) used the null average (7.5%). |
| **S** | 'Simple' event, 1 to 3 transgene copies |
| **PS** | Probably a 'Simple' event |
| **C** | 'Complex' event with more than 3 transgene copies |
| **N** | 'Negative', no transgenes detected |

**Table 19.**

| Plant ID | Preliminary Southern Analysis | # seeds for GC FAME analysis | # seed with C16:1 WT ratio <10% | # seed with C16:1 inter-mediat e ratio | # seed with C16:1 highest ratio | Ratio sat in WT | Ratio sat in transgenic | Ratio sat reduction |
|---|---|---|---|---|---|---|---|---|
| 152-11.30 | C | 16 | 2 | 12 | 2 | 9.6 | 3.4 | 64.6 |
| 230-11.30 | C | 16 | 2 | 13 | 1 | 9.8 | 3.5 | 64.4 |
| 235-11.19 | C | 16 | 0 | 16 | 0 | 7.5 | 3.4 | 54.9 |
| 75-11.30 | S | 16 | 5 | 9 | 2 | 9.0 | 4.1 | 54.6 |
| 147-11.19 | C | 15 | 3 | 8 | 4 | 8.1 | 3.8 | 53.4 |
| 68-11.30 | C | 16 | 1 | 15 | 0 | 9.1 | 4.4 | 51.5 |

(continued)

**Table 19.**

| Plant ID | Preliminary Southern Analysis | # seeds for GC FAME analysis | # seed with C16:1 WT ratio <10% | # seed with C16:1 inter-mediat e ratio | # seed with C16:1 highest ratio | Ratio sat in WT | Ratio sat in transgenic | Ratio sat reduction |
|---|---|---|---|---|---|---|---|---|
| 222-11.30 | C | 36 | 3 | 11 | 2 | 8.5 | 4.2 | 50.8 |
| 57-11.30 | C | 16 | 5 | 10 | 1 | 8.4 | 4.2 | 50.0 |
| 284-11.19 | S | 16 | 3 | 8 | 5 | 7.1 | 3.5 | 49.9 |
| 108-11.30 | S | 16 | 0 | 16 | 0 | 7.5 | 3.9 | 48.5 |
| 151b-11.30 | S | 16 | 3 | 9 | 4 | 7.1 | 3.7 | 48.3 |
| 87b-11.19 | C | 15 | 7 | 5 | 3 | 7.7 | 4.1 | 47.6 |
| 171-11.30 | C | 16 | 0 | 16 | 0 | 7.5 | 4.0 | 46.5 |
| 43b-11.30 | S | 16 | 1 | 14 | 1 | 6.9 | 3.7 | 46.4 |
| 32-11.30 | pS | 16 | 1 | 12 | 3 | 9.5 | 5.1 | 46.1 |
| 145-11.19 | S | 16 | 0 | 16 | 0 | 7.5 | 4.1 | 45.6 |
| 232-11.30 | C | 16 | 0 | 16 | 0 | 7.5 | 4.1 | 45.1 |
| 96a-6.15 | pS | 16 | 2 | 11 | 3 | 9.1 | 5.0 | 45.0 |
| 115-11.30 | C | 16 | 9 | 4 | 4 | 8.1 | 4.5 | 44.5 |
| 250-11.19 | S | 16 | 7 | 7 | 2 | 6.2 | 3.5 | 43.5 |
| 52-(2)-11.30 | pS | 16 | 0 | 16 | 0 | 7.5 | 4.3 | 43.1 |
| 226-11.30 | C | 16 | 1 | 12 | 3 | 5.8 | 3.3 | 42.8 |
| 224-11.30 | C | 16 | 2 | 10 | 4 | 6.8 | 3.9 | 42.7 |
| 149-11.30 | S | 16 | 7 | 7 | 2 | 6.0 | 3.5 | 42.6 |
| 309-11.30 | C | 16 | 5 | 7 | 4 | 6.8 | 4.0 | 41.5 |
| 159a-11.19 | S | 16 | 8 | 8 | 0 | 8.0 | 4.7 | 41.3 |
| 114-11.30 | C | 16 | 0 | 16 | 0 | 7.5 | 4.5 | 40.4 |
| 5-11.19 | C | 16 | 1 | 15 | 0 | 7.3 | 4.4 | 40.0 |
| 294-11.19 | C | 16 | 0 | 16 | 0 | 7.5 | 4.5 | 40.0 |
| (9)-3-11.30 | S | 16 | 6 | 7 | 3 | 7.9 | 4.8 | 39.8 |
| 210-11.19 | S | 16 | 7 | 4 | 4 | 7.8 | 4.8 | 39.1 |
| 15-11.19 | pS | 16 | 6 | 10 | 0 | 7.5 | 4.6 | 38.7 |
| 162a-11.30 | S | 16 | 10 | 6 | 0 | 7.9 | 4.9 | 38.6 |
| 72-11.19 | S | 16 | 13 | 3 | 0 | 7.0 | 4.3 | 37.8 |
| 324-11.30 | S | 16 | 1 | 9 | 6 | 6.1 | 3.8 | 37.2 |
| 162c-11.30 | S | 16 | 2 | 14 | 0 | 7.4 | 4.7 | 37.2 |

(continued)

**Table 19.**

| Plant ID | Preliminary Southern Analysis | # seeds for GC FAME analysis | # seed with C16:1 WT ratio <10% | # seed with C16:1 inter-mediat e ratio | # seed with C16:1 highest ratio | Ratio sat in WT | Ratio sat in transgenic | Ratio sat reduction |
|---|---|---|---|---|---|---|---|---|
| 102-11.19 | pS | 16 | 4 | 10 | 2 | 7.6 | 4.8 | 36.1 |
| 126-11.30 | S | 16 | 4 | 9 | 3 | 8.3 | 5.3 | 35.7 |
| 322-11.30 | S | 16 | 1 | 9 | 6 | 5.7 | 3.8 | 32.6 |
| 249 (294)-11.30 | S | 16 | 2 | 10 | 4 | 8.0 | 5.5 | 31.9 |
| 330-11.19 | pS | 16 | 3 | 12 | 1 | 6.2 | 4.2 | 31.8 |
| 138-11.30 | pS | 15 | 5 | 7 | 3 | 7.5 | 5.1 | 31.8 |
| 35b-11.30 | S | 16 | 1 | 12 | 3 | 7.1 | 4.9 | 31.5 |
| 218-11.30 | S | 16 | 0 | 16 | 0 | 7.5 | 5.2 | 30.9 |
| 146- 11.19 | S | 16 | 2 | 8 | 6 | 6.4 | 4.4 | 30.9 |
| 175-11.30 | S | 16 | 6 | 8 | 2 | 7.2 | 5.1 | 28.7 |
| 69-11.19 | S | 16 | 4 | 9 | 3 | 7.0 | 5.0 | 28.3 |
| 311-11.30 | C | 16 | 0 | 16 | 0 | 7.5 | 5.4 | 28.3 |
| 162-.11.19 | pS | 16 | 9 | 7 | 0 | 6.6 | 4.8 | 27.4 |
| 350-11.19 | pS | 16 | 3 | 12 | 1 | 6.7 | 4.9 | 27.0 |
| 36-11.19 | S | 15 | 1 | 9 | 5 | 6.8 | 5.0 | 26.8 |
| 320-11.30 | C | 16 | 7 | 9 | 0 | 7.1 | 5.3 | 24.9 |
| 245-11.30 | C | 16 | 0 | 16 | 0 | 7.5 | 5.7 | 24.7 |
| 326-11.19 | S | 16 | 5 | 1 | 10 | 5.7 | 4.5 | 20.6 |
| 213-11.30 | S | 16 | 8 | 8 | 0 | 6.2 | 5.1 | 18.0 |
| 26-11.30 | pS | 16 | 8 | 7 | 1 | 7.8 | 7.4 | 5.0 |
| 209-11.19 | S | 16 | 16 | 0 | 0 | 7.9 | 7.9 | 0.0 |
| 5a-6.15 | N | 16 | 16 | 0 | 0 | 9.2 | 9.2 | 0.0 |
| 14a--6.15 | N | 13 | 13 | 0 | 0 | 8.2 | 8.2 | 0.0 |
| 14b-6.15 | N | 16 | 16 | 0 | 0 | 8.0 | 8.0 | 0.0 |
| 63-6.15 | N | 16 | 16 | 0 | 0 | 9.3 | 9.3 | 0.0 |
| 99a-6.15 | N | 16 | 16 | 0 | 0 | 9.2 | 9.2 | 0.0 |
| 99c-6.15 | N | 16 | 16 | 0 | 0 | 8.2 | 8.2 | 0.0 |
| 87a-11.9 | N | 16 | 16 | 0 | 0 | 7.9 | 7.9 | 0.0 |
| 35a-11.30 | N | 16 | 16 | 0 | 0 | 7.1 | 7.1 | 0.0 |
| 43a-11.30 | N | 15 | 15 | 0 | 0 | 7.4 | 7.4 | 0.0 |
| 76-11.30 | N | 16 | 16 | 0 | 0 | 7.5 | 7.5 | 0.0 |

The "Ratio of Saturate Reduction" was used to rank events because it generally used seed from the same transgenic event. This direct comparison helps reduce variability between plants caused by tissue culture and growing plants at different times.

The above data shows an apparent gene dosage effect; more copies of the transgene tend to cause a more effective reduction in saturated fatty acids. For example, there are 57 "non-control" plants represented above. These 57 plants can be divided into three groups of 19 plants. The top set of plants (exhibiting the best reductions in saturates and the lowest levels of saturates) have 11 of 19 "complex" events (more than 3 copies of the desaturase gene). This set contained 8 of 19 events characterized as 'Simple' or 'probably Simple.' The middle set of 19 plants had only 6 of 19 "complex" events (13 of 19 "simple" or "probably simple" events). Still further, the third set of 19 plants (showing, relatively, the least reductions in saturates) contained only 3 complex events, with 16 of the 19 events being "simple" or "probably simple." Thus, plants with cells having more than 3 copies of the desaturase appear to show a better reduction in saturates than plants with cells having only 1 copy of the gene.

Example 15 - Segregation of Oleic and Vaccenic Acids

Vaccenic acid is a C18:1 with the double bond in the delta-11 position. Vaccenic acid is formed by elongating delta-9 C16:1 outside of the plastid. The following is important because other analytical methods discussed herein combined oleic and vaccenic acid peaks together into a single percent composition that was labeled as "oleic." That is, there was no separation of the two unless otherwise indicated. By subtracting out the vaccenic acid contribution, it is presently demonstrated that the percent contribution of oleic acid is, preferably and advantageously (and surprisingly), maintained at less than 80% while still achieving a reduction (to "no sat" or "low sat" levels) of overall) total saturates. Two types of analyses were used to demonstrate this, as set forth in the following two Examples.

Example 16 - Canola Delta-9 Seed Extraction and Analysis for Vaccenic Acid SOP

**Figures 7A and 7B** illustrate data obtained using the following protocol.

Sample preparation (same as before)

1. Obtain weight of individual seeds and place into plastic mother plate.
2. Add 2, 1/8" balls to each well
3. Take mother plate to liquid handler Hamilton
add 400 μL of heptane with IS (C11:0) and surrogate (C15:0 FAEE) then add 100 μL of sodium methoxide (.5N)
4. Cap inserts with strips caps and Geno-grind for 5 minutes (1x @ 500)
5. Replace strip cap
6. Add plate lid with a rubber mat (extra sealing). Tape the lid with black electrical tape and remove bottom of mother plate to expose vials
7. Place the plate onto vortex/heater for 15 min. at 37°C / 60 rpm (the vortex wells are filled with glass beads)
8. Centrifuge plate @ 3500 rpm for 2 min.
9. Place the bottom lid back and remove lid/strip cap. Transfer 350 μl of top layer into the daughter plate using Hamilton.
10. Then add 400 μL of heptane with IS and surrogate to the extraction plate.
11. Repeat steps 5 to 10 two more times (for a total of 3 transfers)
12. Keep the extract plate on the Hamilton after last transfer. Transfer 50 μl of extract into glass insert mounted in aluminum block containing 450 μl heptane with IS C11
13. Inject on GC

GC/FID analysis (different from FAMEs profile)

GC parameters:
Injection 1 μl splitless per sample
Column-
BPX 70 from SGE, 15 meters, 0.25 mm LD. and 0.25 μm film thickness

GC parameters-

Oven temperature program-
70°C hold for 2,15min (splitless)
70°C - 140° @ 25°C- min,
140°C hold for 14 min
140°C -180° @ 10°C- min.,
180°C hold for 3 min
180°C - 220° @ 25°C- min.,
220°C hold for 3 min.
Injector temp. - 230°C
Detector temp. - 240°C
Make-up gas - Nitrogen @ 25mL/min.
FID fuel - air @ 400mL/ min.,
hydrogen @ 40mL/ min
front injector: purge time 1 min.\
purge flow 35 ml/min
back injector: purge time 2.15 min.
purge flow 35 ml/min.
front inlet pressure: 1.0 mL/min - constant flow
back inlet pressure: 1.0 mL/min - constant flow
Run time - 30.55 minutes,
Flow rates - helium constant flow @ 1 mL/ min

Acquisition sequence

96 samples are splited between front and back column to minimize the build on the liner. The sample list is built with 5 injection methods corresponding to the type of sample being injected. The first 5 samples injected are always:

1. Matrix
2. Matrix
3. Standard1
4. Reagent Blank
5-32. Canola samples
33. Standard2
34-54. Canola samples
55. Standard3

Each list comprises 6 events of 8 samples (1 seed = 1 sample) (48 samples).

Standard contains 200 ppm FAMES total distributed as follow:

Table 20.

| FAMEs | Concentration (ppm) | Added to calibration |
|---|---|---|
| C14:0 | 2 | yes |
| C16:0 | 8 | yes |
| C18:0 | 6 | yes |
| C18:1 | 120 | yes |
| C18:2 | 24 | yes |
| C18:3 | 10 | yes |
| C20:0 | 6 | yes |
| C20:1 | 2 | yes |

(continued)

| Table 20. | | |
|---|---|---|
| FAMEs | Concentration (ppm) | Added to calibration |
| C22:0 | 6 | yes |
| C22:1 | 10 | No |
| C24:0 | 6 | Yes |

Example 17 - Analysis of Vaccenic Acid Contribution Using Gas Chromatography/Mass Spectrometry/Time of Flight

**Table 21** shows data obtained using the following protocol. In **Table 21,** for the T4, percent lipid was not reduced; it was maintained at 40.8% in the transgenic line (the same as the control line).

**Table 21.**

| Event | Generation | D-9 | PAT | | C14:0 | C16:0 | C16:1 | C18:0 | C18:1 | Vacc 18:1 | C18:2 | C18:3 |
|---|---|---|---|---|---|---|---|---|---|---|---|---|
| 69-11.19 (HL) | T3 | 2 | 1.5 | average | 0* | 2.3 | 1.6 | 0.4 | 78.6 | 3.1 | 9.2 | 3.4 |
| 69-11.19(N) | T3 | 0 | 0 | average | 0.0 | 3.7 | 0.3 | 1.9 | 75.2 | 3.2 | 10.1 | 3.2 |
| | | | | | | | | | | | | |
| 218-11.30 (HL) | T4 | 1 | 1.5 or 2 | average | 0.0 | 2.8 | 1.2 | 0.7 | 78.2 | 2.9 | 9.9 | 3.1 |
| 218-11.30 (N) | T4 | 0 | 0 | average | 0.0 | 3.9 | 0.3 | 1.8 | 74.4 | 3.1 | 10.8 | 3.3 |

**Table 21. (continued)**

| Event | Generation | D-9 | PAT | | C20:0 | C20:1 | C22:0 | C24:0 | Percent Lipid | Seed weight | Total oil (mg) | % saturated FA |
|---|---|---|---|---|---|---|---|---|---|---|---|---|
| 69-11.19 (HL) | T3 | 2 | 1.5 | average | 0.2 | 0.8 | 0.1 | 0.1 | 35.1 | 5.3 | 1.9 | 3.2 |
| 69-11.19(N) | T3 | 0 | 0 | average | 0.7 | 1.2 | 0.4 | 0.2 | 42.6 | 4.0 | 1.7 | 6.9 |
| | | | | | | | | | | | | |
| 218-11.30 (HL) | T4 | 1 | 1.5 or 2 | average | 0.3 | 0.8 | 0.1 | 0.0 | 40.8 | 4.2 | 1.7 | 4.0 |
| 218-11.30 (N) | T4 | 0 | 0 | average | 0.7 | 1.2 | 0.4 | 0.2 | 40.8 | 4.0 | 1.6 | 6.9 |

*: if less than detection limit by processing method the default was 0

Acquisition and processing using HP Chemserver package

Instruments description

Time Of Flight mass spectrometer Pegasus III from Leco interfaced with a gas chromatograph HP 6890.

Combi Pal autoinjector from CTC Analytics technology mounted on the HP 6890 with a 10 $\mu$l syringe.

GC method

GC parameters:
Injection I to 3 μl splitless per sample

Column-

SolGel Wax, 30 meters, 0.25 mm I.D. and 0.25μm film thickness

GC parameters-

Oven temperature program-
70°C - 175° @ 25°C$^{-}$ min., (splitless)
175°C hold for 25 min.
175°C - 230° @ 50°C$^{-}$ min.,
230°C hold for 3 min.
Injector temp. - 230°C
Transfer line. - 300°C
back injector: purge time 30 seconds.
purge flow 20 ml/min.
back inlet pressure: 2 mL/min - constant flow
Run time - 33.3 minutes,
Flow rates - helium constant flow @ 2 mL/ min
Mass spectrometer
Mass selection:
Collected mass from 50 to 600 amu
Filament bias: -70 V
Ion source: 225°C

Detector:

Detector voltage: 1600 V
Acquisition rate: 10 spectra/sec
Solvent delay 100 seconds

Fragmentation

ChromaTOF Software compiling fragmentation and deconvoluting co migrating peaks for better separation and interpretation.

Identification of fatty acids is performed by retention time and fragmentation match based on Standard solution (see below) injected in the same conditions and/or good match with NIST/EPA/NIH database included in software described above.

Vaccenoic acid methyl ester also known as cis 11-octadecenoic acid methyl ester was identified from the canola seed extract by running a standard made from Vaccenic acid from Sigma (CAS:506-17-2). The retention time is 1207 seconds and produce a 853 match with standard and 814 with library describe above.

Composition of the standard injected:

| Table 22. | | |
|---|---|---|
| FAMEs | Concentration (ppm) | Added to calibration |
| C14:0 | 2 | Yes |
| C16:0 | 8 | Yes |
| C18:0 | 6 | Yes |
| C18:1 | 120 | Yes |
| C18:2 | 24 | Yes |
| C18:3 | 10 | Yes |
| C20:0 | 6 | Yes |
| C20:1 | 2 | Yes |
| C22:0 | 6 | Yes |
| C22:1 | 10 | No |
| C24:0 | 6 | Yes |

Vaccenoate methyl ester process:

The methylated product was obtain after methylation of 100 mg of the acid:

- 100 mg dissolve in 5 ml of MeOHCl 0.5 N (Supelco) in a 30 ml glass vial
- heated 1 hour at 70°C under nitrogen
- after cooling at room temperature added 5 ml of water containing 0.9% NaCl
- partitioned the ester in hexane in three consecutive hexane extractions (15 ml)
- neutralize the acid residue by mixing the organic phase with 15 ml of water containing HNaCO3 at 2.5%
- evaporate the organic layer under N2 to obtain an oily transparent liquid at RT corresponding to a vaccenoate methyl ester

Example 18 - Achieving "No Sats" While Maintaining Superior Agronomic Traits

Agronomic measurements were made at the various field sites, compazing the transgenics and controls. The conclusion was that overall, the transgenic plants behaved like and exhibited similar traits (aside from much-improved saturate levels) the Nex 710 controls. Thus, the subject transgene(s) has no consistent negative effect on plant health. A summary of the traits and rating system is provided in **Table 23.** Various criteria were used at various field site locations.

**Tables 24-27** numerically illustrate some representative results. The following abbreviations are used in those figures:

| | |
|---|---|
| DTF = Days to Flower | EOF = Days to End of Flower |
| HT = Height | SC = Sterile Counts |
| DTM = Days to Maturity | LSV = Late Season Vigor |
| LOD = Lodging | SD WT = Seed Weight |

**Table 24** shows agronomic data for lines from Event #218-11.30. **Table 25** shows agronomic data for lines from Event #36-11.19. **Table 26** shows agronomic data for lines from Event 69-11.19.The foregoing demonstrates that the subject invention, *i.e*. achieving "no sat" canola, can be practiced, surprisingly, without adversely affecting other important agronomic traits.

**Table 23.**

| Field Ratings | | | |
|---|---|---|---|
| Rating | Timing | Scale | Details |
| Vigor/Establishment | 3-4 leaf | 1 to 5 | 1=excellent emergence, healthy stand; 5=very poor emergence and/or seedling health |

(continued)

| Field Ratings | | | |
|---|---|---|---|
| Rating | Timing | Scale | Details |
| Herbicide tolerance 1 | 4-7 days after sraying | 0 to 100% | 1-5 barely detectable, 6-10 detectable by trained eye, 11-15 noticeable by a grower, 15+ likely a grower complaint, 100=all plants in plot are dead |
| Herbicide tolerance 2 | 10-14 days after spraying | 0 to 100% | same as details for Herbicide tolerance 1 |
| Days to flower (DTF) | rated every 2-3 days | # days after seeding | 10 % of plants have at least 1 flower open |
| Days to end of flower (EOF) | rated every 2-3 days | # days after seeding | 95% of plants in plot have finished flowering |
| Height | late pod fill | cm | height of perfectly erect plants, gather bunch of plants from centre of plot and stretch up to measure |
| Days to maturity (DTM) | rated every 2-3 days | # days after seeding | 30% pod turn, or optimal time of commercial swathing. However, several pods per plot (from middle of the main raceme) should be opened to determine whether seed maturity correlates to pod colour (ie. Some varieties may appear green, but seeds are mature.) |
| Lodging resistance (LODGE_RES) | at maturity | 1 to 5 | 1=perfectly erect, 5=horizontal |
| Rating Yield | Timing | Scale grams per plot | Details |
| | | | and weigh system should be used to sample standardize yield for varying moisture content; if system is not available, samples should be dired to constant weight prior to recording yield. |
| Late Season Vigor (1-5): Sterile Counts Notes | just before maturity Flowering throughout season | 100 % bloom | A visual assessment of the general agronomic *performance* of the line (i.e. potential yielding ability of the line, branching pattern, silique length and size) was used. A rating of 1-5 was used with 1 = best and 5 being the worst in agronomic appearance counting ten plants/ plot |
| | | | note any plot or plots with poor emergence or plant stand, flooding, or any other factors that might affect accuracty of ratings. |

**Table 24. Agronomic Data Summary of Best Performing Lines from Event #218-11.30**

| | | | | | | | | | | 1000 SEED WT | |
|---|---|---|---|---|---|---|---|---|---|---|---|
| Line | Event | DTM | EOF | HT | SC | DTM | LSV | LOD | SD WT | % NULL | % NEX 710 |
| 1361 (TS) | #218-11.30 | 45 | 70 | 100 | 0 | 90 | 2 | 2 | 3.20 | 98 | 93 |
| 1319(TS) | #218-11.30 | 44 | 70 | 98 | 0 | 90 | 2 | 1 | 3.23 | 98 | 94 |
| 1304(TS) | #218-11.30 | 46 | 72 | 110 | 0 | 90 | 2 | 1 | 3.45 | 105 | 101 |
| 1500(TS) | #218-11.30 | 45 | 71 | 103 | 0 | 91 | 3 | 1 | 3.12 | 95 | 91 |
| 1405(TS) | #218-11.30 | 45 | 72 | 109 | 0 | 91 | 2 | 1 | 3.05 | 93 | 89 |
| 1370(TS) | #218-11.30 | 44 | 70 | 106 | 0 | 90 | 2 | 1 | 3.41 | 104 | 99 |
| 1369(TS) | #218-11.30 | 44 | 70 | 102 | 0 | 91 | 2 | 1 | 3.33 | 102 | 97 |
| 1370(T) | #218-11.30 | 44 | 70 | 106 | 0 | 90 | 2 | 1 | 3.41 | 104 | 99 |
| 1405(T) | #218-11.30 | 45 | 72 | 109 | 0 | 91 | 2 | 1 | 3.05 | 93 | 89 |
| 1299(N) | #218-11.30 | 43 | 69 | 98 | 0 | 90 | 1 | 1 | 3.28 | . | 96 |

(continued)

| Line | Event | DTM | EOF | HT | SC | DTM | LSV | LOD | SD WT | 1000 SEED WT % NULL | % NEX 710 |
|---|---|---|---|---|---|---|---|---|---|---|---|
| Nex 710 | . | 42 | 67 | 100 | 0 | 88 | 2 | 1 | 3.43 | . | . |

Table 25. Agronomic Data Summary of Best Performing Lines from Event 36-11.19

| Line | Event | DTF | EOF | HT | SC | DTM | LSV | LOD | SD WT | 1000 SEED WT % NULL | % NEX 710 |
|---|---|---|---|---|---|---|---|---|---|---|---|
| 1099(T) | 36-11.19 | 45 | 70 | 106 | 0 | 90 | 2 | 1 | 3.78 | 98 | 110 |
| 1127(N) | 36-11.19 | 44 | 68 | 102 | 0 | 89 | 2 | 1 | 3.85 | . | 112 |
| Nex 710 | . | 42 | 67 | 100 | 0 | 88 | 2 | 1 | 3.43 | . | . |

Table 26. Agronomic Data Summary of Best Performing Lines from Event 69-11.19

| Line | Event | DTF | EOF | HT | SC | DTM | LSV | LOD | SD WT | 1000 SEED WT | % NEX 710 |
|---|---|---|---|---|---|---|---|---|---|---|---|
| 1538 | 69-11.19 | 45 | 72 | 103 | 0 | 91 | 4 | 1 | 3.17 | 94 | 92 |
| 1529 | 69-11.19 | 45 | 71 | 112 | 0 | 91 | 3 | 1 | 3.41 | 101 | 99 |
| 1534 | 69-11.19 | 46 | 71 | 109 | 0 | 91 | 3 | 1 | 3.24 | 96 | 94 |
| 1604(N) | 69-11.19 | 43 | 68 | 102 | 0 | 89 | 2 | 1 | 3.38 | . | 99 |
| Nex 710 | . | 42 | 67 | 100 | 0 | 88 | 2 | 1 | 3.43 | . | . |

Example 19 - Dose Effect; Further Lowering of Saturates by Insertion of Multiple Copies of δ-9 Desaturase Genes

This Example further shows that "stacking" multiple copies of δ-9 desaturase genes has a surprising dose effect, which can be used to even further reduce saturates in oil seed plants such as canola. The following is greenhouse data measured using FAME procedures unless otherwise indicated.

**Table 27** reports F3 10 seed bulk fatty acid data from native Nex 710, the simple events (218, 36, 69), and from each of the F3 seed packages tracing back to F2 plants that were selected on the basis of InVader assays. (The last two columns of Table 27 show approximate average total saturates for the respective plants, and approximate average reduction in total saturates, as compared to the Nex 710 control.) At that generation, Southern data was not used. Thus, based on saturate expression and InVader assays, a selection of suspected stacks and suspected parental siblings as well as nulls was made to be reconfirmed by growing out F3 plants and re-testing using Southems.

For example, while not statistically analyzed, samples from the 41 "stack" lines have an average total saturates of 3.5%. The 21 "single" lines have an average total saturates of 3.84%.

**Table 28** contains a summary of the suspected F3 stacks, suspected parental siblings, and nulls that were replanted for continuation of copy number and zygosity. Lines named TDN0400141, TDN0400142, TDN0400145, TDN0400155, TDN0400158, and TDN0400160 were suspected to be homozygous stacks. Lines TDN0400189, TDN0400143, TDN0400197, TDN0400167, and TDN0400184 were suspected to be parental siblings out of the stacks. Lines TDN0400198, TDN0400199 were advanced as nulls selfed out of the stacks. TDN0400202, TDN0400204, and TDN0400208 are the simple events. This material is also currently in the field or recently harvested. Thus, field data is not yet available.

**Figures 8 and 9** are pictures of two gels run with DNA from F3 plants. Similar issues with isolating DNA for Southern analysis were encountered at this step, so not all 9 of the plants submitted appeared in gels. Based on the gels, lines TDN0400141, TDN0400142, and possibly TDN0400158 (only 2 plants) are homozygous stacks. TDN0400145 is still segregating for event 36, and TDN0400155 is still segregating for event 69. Line TDN0400160 appears to have an odd segregation of bands which may indicate that it is segregating for all 3 simple events. Follow-up will be conducted TDN0400160. Plant #8 of from this cross has a low sat level of 2.6% which is consistent with being a triple stack of high zygosity. Additional molecular analysis could confirm this. Not all of the lines suspected to be parental siblings appear in the gels (Lines TDN0400184, TDN0400189, and TDN0400197 highlighted in the Event column of **Table 29,** discussed below). Based on the single plant fatty acid data, it appears that the 9 plants from TDN0400184 (suspected of being a sibbed-out simple event 218) have as low sat levels as the suspected stacks. That is, the trend in the data is that stacks consistently show a reduction in saturates compared to "sibbed-out" events (single transgenic events recovered from crosses of two transgenic events).

**Table 29** contains 10 seed bulk fatty acid data from Nex 710, the simple events, and each single F4 plant. Nine plants of each suspected stack, null, simple event, and the like were regrown, tissue sampled for molecular analysis, and kept through to seed set for fatty acid analysis.

Table 27.

| Name | Source | C12:0 | C14:0 | C16:0 | C16:1 | C18:0 | C18:1 | C18:2 | C18:3 | C20:0 | C20:1 | C20:2 |
|---|---|---|---|---|---|---|---|---|---|---|---|---|
| TDN0400210 | NEX 710 | 0.0 | 0.0 | 4.1 | 0.3 | 1.4 | 76.5 | 11.6 | 2.9 | 0.6 | 1.5 | 0.1 |
| TDN0400211 | NEX 710 | 0.0 | 0.1 | 4.4 | 0.3 | 1.5 | 73.9 | 13.5 | 2.9 | 0.7 | 1.5 | 0.1 |
| TDN0400212 | NEX 710 | 0.0 | 0.0 | 4.3 | 0.3 | 1.6 | 76.2 | 11.5 | 2.8 | 0.7 | 1.5 | 0.0 |
| TDN0400201 | TDN04-123 | 0.0 | 0.0 | 3.0 | 1.5 | 0.5 | 79.3 | 11.0 | 2.8 | 0.3 | 0.9 | 0.0 |
| TDN0400202 | TDN04-123 | 0.0 | 0.0 | 3.0 | 1.6 | 0.5 | 79.3 | 10.9 | 2.8 | 0.3 | 0.9 | 0.0 |
| TDN0400203 | TDN04-123 | 0.0 | 0.0 | 2.8 | 1.6 | 0.4 | 79.3 | 10.8 | 3.1 | 0.2 | 0.8 | 0.0 |
| TDN0400204 | TDN04-128 | 0.0 | 0.1 | 2.7 | 1.9 | 0.5 | 78.8 | 11.0 | 3.0 | 0.3 | 0.8 | 0.0 |
| TDN0400205 | TDN04-128 | 0.0 | 0.0 | 2.7 | 1.9 | 0.5 | 80.3 | 9.8 | 2.8 | 0.3 | 0.8 | 0.0 |
| TDN0400206 | TDN04-128 | 0.0 | 0.0 | 2.8 | 1.9 | 0.6 | 79.4 | 10.6 | 2.7 | 0.3 | 0.8 | 0.0 |
| TDN0400207 | TDN04-132 | 0.0 | 0.1 | 2.6 | 1.8 | 0.5 | 79.4 | 10.8 | 2.7 | 0.3 | 0.9 | 0.0 |
| TDN0400208 | TDN04-132 | 0.0 | 0.1 | 2.5 | 1.9 | 0.6 | 79.4 | 10.7 | 2.5 | 0.3 | 1.0 | 0.0 |
| TDN0400209 | TDN04-132 | 0.0 | 0.1 | 2.7 | 1.9 | 0.6 | 79.7 | 10.6 | 2.4 | 0.3 | 0.9 | 0.0 |
| TDN0400198 | TDN04-133/P-116 | 0.0 | 0.0 | 4.0 | 0.3 | 1.2 | 75.3 | 12.8 | 3.4 | 0.5 | 1.5 | 0.1 |
| TDN0400199 | TDN04-134/P11 | 0.0 | 0.0 | 4.2 | 0.3 | 1.2 | 73.7 | 14.2 | 3.4 | 0.5 | 1.5 | 0.1 |
| TDN0400200 | TDN04-135/P125 | 0.0 | 0.0 | 4.0 | 0.3 | 1.5 | 75.6 | 12.6 | 3.1 | 0.6 | 1.5 | 0.1 |
| TDN0400136 | TDN04-133/P14 | 0.0 | 0.0 | 2.4 | 2.2 | 0.4 | 79.9 | 9.7 | 3.0 | 0.3 | 0.9 | 0.1 |
| TDN0400137 | TDN04-133/P25 | 0.0 | 0.0 | 2.4 | 2.1 | 0.6 | 79.8 | 9.2 | 3.1 | 0.4 | 1.1 | 0.1 |
| TDN0400138 | TDN04-133/P29 | 0.0 | 0.0 | 2.5 | 2.3 | 0.4 | 81.5 | 8.5 | 2.6 | 0.3 | 0.8 | 0.0 |
| TDN0400139 | TDN04-133/P37 | 0.0 | 0.0 | 2.3 | 2.4 | 0.4 | 80.1 | 9.9 | 3.0 | 0.6 | 0.8 | nd |
| TDN0400140 | TDN04-133/P40 | 0.0 | 0.0 | 2.4 | 2.1 | 0.5 | 81.4 | 8.3 | 2.9 | 0.3 | 0.9 | 0.0 |
| TDN0400141 | TDN04-133/P70 | 0.0 | 0.0 | 2.2 | 2.4 | 0.3 | 80.3 | 9.3 | 3.2 | 0.3 | 0.9 | 0.0 |
| TDN0400142 | TDN04-133/P114 | 0.0 | 0.0 | 2.2 | 2.4 | 0.3 | 79.9 | 9.8 | 3.2 | 0.3 | 0.9 | nd |
| TDN0400143 | TDN04-133/P121 | 0.0 | 0.0 | 2.8 | 1.7 | 0.6 | 79.6 | 10.7 | 2.5 | 0.3 | 0.9 | 0.0 |
| TDN0400144 | TDN04-133/P122 | 0.0 | 0.0 | 2.7 | 1.9 | 0.5 | 80.6 | 9.5 | 2.8 | 0.3 | 0.9 | 0.0 |
| TDN0400145 | TDN04-133/P129 | 0.0 | 0.0 | 2.3 | 2.1 | 0.4 | 81.2 | 9.1 | 2.7 | 0.1 | 0.9 | nd |
| TDN0400146 | TDN04-133/P139 | 0.0 | 0.0 | 2.8 | 1.7 | 0.6 | 79.7 | 10.1 | 3.2 | 0.3 | 0.9 | nd |
| TDN0400147 | TDN04-133/P1 | 0.0 | 0.0 | 2.7 | 2.0 | 0.5 | 80.9 | 8.8 | 2.7 | 0.4 | 1.0 | 0.0 |
| TDN0400148 | TDN04-133/P11 | 0.0 | 0.0 | 2.6 | 2.2 | 0.8 | 79.7 | 9.1 | 3.0 | 0.4 | 1.0 | nd |
| TDN0400149 | TDN04-133/P54 | 0.0 | 0.0 | 2.5 | 2.0 | 0.5 | 82.2 | 8.0 | 2.5 | 0.3 | 0.9 | nd |
| TDN0400150 | TDN04-133/P57 | 0.0 | 0.0 | 2.8 | 1.7 | 0.5 | 79.0 | 11.1 | 2.9 | 0.3 | 0.9 | 0.0 |
| TDN0400151 | TDN04-133/P67 | 0.0 | 0.0 | 2.1 | 2.2 | 0.4 | 80.4 | 9.4 | 3.0 | 0.3 | 1.0 | 0.0 |

Table 27.

| Name | Source | C12:0 | C14:0 | C16:0 | C16:1 | C18:0 | C18:1 | C18:2 | C18:3 | C20:0 | C20:1 | C20:2 |
|---|---|---|---|---|---|---|---|---|---|---|---|---|
| **TDN0400152** | TDN04-133/P76 | 0.0 | 0.0 | 2.5 | 1.7 | 0.5 | 81.2 | 8.6 | 3.2 | 0.3 | 1.0 | 0.0 |
| **TDN0400153** | TDN04-133/P77 | 0.0 | 0.0 | 2.7 | 1.6 | 0.5 | 81.6 | 8.8 | 2.7 | 0.3 | 1.0 | 0.0 |
| **TDN0400154** | TDN04-134/P-27 | 0.0 | 0.0 | 2.5 | 2.1 | 0.4 | 82.0 | 7.9 | 2.7 | 0.3 | 0.9 | 0.0 |
| **TDN0400155** | TDN04-134/P-32 | 0.0 | 0.0 | 2.4 | 2.3 | 0.4 | 79.6 | 10.1 | 3.4 | 0.1 | 0.8 | nd |
| **TDN0400156** | TDN04-134/P-33 | 0.0 | 0.0 | 2.8 | 2.0 | 0.5 | 80.0 | 9.5 | 3.0 | 0.3 | 0.9 | nd |
| **TDN0400157** | TDN04-134/P-34 | 0.0 | 0.0 | 2.8 | 1.9 | 0.6 | 80.3 | 9.5 | 2.7 | 0.3 | 0.9 | 0.0 |
| **TDN0400158** | TDN04-134/P-38 | 0.0 | 0.0 | 2.2 | 2.4 | 0.3 | 80.1 | 9.4 | 3.4 | 0.3 | 0.8 | 0.0 |
| **TDN0400159** | TDN04-134/P-42 | 0.0 | 0.0 | 2.6 | 2.1 | 0.5 | 79.7 | 10.0 | 3.1 | 0.3 | 0.9 | 0.0 |
| **TDN0400160** | TDN04-134/P-48 | 0.0 | 0.0 | 2.2 | 2.3 | 0.3 | 81.9 | 8.5 | 3.0 | 0.0 | 0.8 | nd |
| **TDN0400161** | TDN04-134/P-52 | 0.0 | 0.0 | 2.5 | 2.2 | 0.4 | 80.6 | 9.2 | 3.1 | 0.0 | 0.8 | 0.0 |
| **TDN0400162** | TDN04-134/P-57 | 0.0 | 0.0 | 2.2 | 2.4 | 0.3 | 80.3 | 9.0 | 3.2 | 0.3 | 0.9 | 0.0 |
| **TDN0400163** | TDN04-134/P-77 | 0.0 | 0.0 | 2.6 | 1.8 | 0.5 | 80.0 | 9.8 | 3.2 | 0.3 | 0.9 | 0.0 |
| **TDN0400164** | TDN04-134/P-82 | 0.0 | 0.0 | 2.7 | 1.9 | 0.5 | 79.6 | 9.8 | 3.3 | 0.3 | 0.9 | 0.0 |
| **TDN0400165** | TDN04-134/P-98 | 0.0 | 0.0 | 2.8 | 1.9 | 0.6 | 77.2 | 11.2 | 3.7 | 0.4 | 1.0 | 0.1 |
| **TDN0400166** | TDN04-134/P-118 | 0.0 | 0.0 | 2.5 | 2.3 | 0.4 | 80.6 | 9.3 | 2.8 | 0.3 | 0.8 | nd |
| **TDN0400167** | TDN04-134/P-119 | 0.0 | 0.0 | 2.6 | 1.9 | 0.5 | 80.5 | 9.4 | 2.8 | 0.3 | 0.9 | nd |
| **TDN0400168** | TDN04-134/P-142 | 0.0 | 0.0 | 2.4 | 2.2 | 0.5 | 80.4 | 9.1 | 3.0 | 0.2 | 0.9 | nd |
| **TDN0400169** | TDN04-134/P-40 | 0.0 | 0.0 | 3.2 | 1.3 | 0.7 | 79.0 | 10.6 | 2.8 | 0.4 | 1.0 | 0.0 |
| **TDN0400170** | TDN04-134/P-41 | 0.0 | 0.0 | 2.5 | 2.0 | 0.4 | 81.0 | 8.9 | 2.9 | 0.3 | 0.9 | 0.0 |
| **TDN0400171** | TDN04-134/P-60 | 0.0 | 0.0 | 2.5 | 2.0 | 0.4 | 80.3 | 9.5 | 3.1 | 0.3 | 0.9 | 0.0 |
| **TDN0400172** | TDN04-134/P-66 | 0.0 | 0.1 | 3.1 | 1.2 | 0.7 | 79.8 | 10.1 | 3.0 | 0.3 | 1.0 | 0.0 |
| **TDN0400173** | TDN04-135/P-23 | 0.0 | 0.0 | 2.6 | 2.2 | 0.5 | 80.6 | 9.3 | 2.7 | 0.3 | 0.8 | 0.0 |
| **TDN0400174** | TDN04-135/P-24 | 0.0 | 0.0 | 2.5 | 2.5 | 0.5 | 81.2 | 8.9 | 2.7 | 0.0 | 0.7 | 0.0 |
| **TDN0400175** | TDN04-135/P-25 | 0.0 | 0.0 | 2.2 | 2.3 | 0.4 | 82.0 | 8.2 | 2.5 | 0.3 | 0.8 | 0.0 |
| **TDN0400176** | TDN04-135/P-36 | 0.0 | 0.1 | 2.3 | 2.3 | 0.4 | 81.2 | 8.6 | 2.7 | 0.3 | 0.9 | 0.0 |
| **TDN0400177** | TDN04-135/P-41 | 0.0 | 0.0 | 2.2 | 2.1 | 0.7 | 83.2 | 7.1 | 2.3 | 0.4 | 0.9 | 0.0 |
| **TDN0400178** | TDN04-135/P-45 | 0.0 | 0.0 | 2.4 | 2.2 | 0.6 | 81.8 | 7.8 | 2.7 | 0.4 | 0.9 | 0.0 |
| **TDN0400179** | TDN04-135/P-47 | 0.0 | 0.0 | 2.3 | 2.5 | 0.5 | 81.8 | 8.0 | 2.5 | 0.3 | 0.8 | 0.0 |
| **TDN0400180** | TDN04-135/P-48 | 0.0 | 0.0 | 2.3 | 2.3 | 0.6 | 83.2 | 6.8 | 2.2 | 0.4 | 0.9 | 0.0 |
| **TDN0400181** | TDN04-135/P-64 | 0.0 | 0.1 | 2.2 | 2.0 | 0.6 | 81.7 | 8.7 | 2.3 | 0.4 | 0.9 | 0.0 |
| **TDN0400182** | TDN04-135/P-86 | 0.0 | 0.1 | 2.3 | 2.4 | 0.5 | 80.2 | 9.5 | 2.7 | 0.3 | 0.8 | 0.0 |
| **TDN0400183** | TDN04-135/P-108 | 0.0 | 0.0 | 2.3 | 2.1 | 0.5 | 82.0 | 8.2 | 2.5 | 0.3 | 0.9 | 0.0 |

**Table 27.**

| Name | Source | C12:0 | C14:0 | C16:0 | C16:1 | C18:0 | C18:1 | C18:2 | C18:3 | C20:0 | C20:1 | C20:2 |
|---|---|---|---|---|---|---|---|---|---|---|---|---|
| **TDN0400184** | TDN04-135/P-112 | 0.0 | 0.1 | 2.4 | 2.3 | 0.4 | 81.3 | 8.9 | 2.7 | 0.3 | 0.8 | 0.0 |
| **TDN0400185** | TDN04-135/P-120 | 0.0 | 0.1 | 2.5 | 1.9 | 0.5 | 81.3 | 9.1 | 2.7 | 0.3 | 0.8 | 0.0 |
| **TDN0400186** | TDN04-135/P-123 | 0.0 | 0.1 | 2.8 | 2.1 | 0.7 | 79.8 | 10.1 | 2.3 | 0.4 | 0.8 | 0.0 |
| **TDN0400187** | TDN04-135/P-124 | 0.0 | 0.1 | 2.7 | 2.9 | 0.4 | 80.7 | 8.3 | 2.7 | 0.3 | 0.7 | nd |
| **TDN0400188** | TDN04-135/P-13 | 0.0 | 0.1 | 2.4 | 1.8 | 0.5 | 80.1 | 9.9 | 2.8 | 0.3 | 0.9 | 0.0 |
| **TDN0400189** | TDN04-135/P-31 | 0.0 | 0.0 | 2.7 | 1.6 | 0.6 | 80.3 | 10.1 | 2.8 | 0.3 | 0.9 | 0.0 |
| **TDN0400190** | TDN04-135/P-46 | 0.0 | 0.1 | 2.7 | 2.1 | 0.7 | 78.9 | 9.6 | 2.6 | 0.4 | 1.2 | 0.0 |
| **TDN0400191** | TDN04-135/P-51 | 0.0 | 0.0 | 2.1 | 1.5 | 0.8 | 83.1 | 7.9 | 2.1 | 0.4 | 1.0 | 0.1 |
| **TDN0400192** | TDN04-135/P-55 | 0.0 | 0.0 | 2.3 | 1.6 | 0.7 | 82.0 | 8.6 | 2.5 | 0.3 | 0.9 | 0.0 |
| **TDN0400193** | TDN04-135/P-56 | 0.0 | 0.1 | 2.7 | 1.6 | 0.6 | 79.5 | 10.9 | 2.6 | 0.3 | 0.9 | 0.0 |
| **TDN0400194** | TDN04-135/P-57 | 0.0 | 0.1 | 2.7 | 1.3 | 0.8 | 80.4 | 10.1 | 2.4 | 0.4 | 1.0 | 0.0 |
| **TDN0400195** | TDN04-135/P-74 | 0.0 | 0.0 | 3.0 | 1.5 | 0.6 | 79.7 | 10.2 | 2.8 | 0.3 | 0.8 | 0.0 |
| **TDN0400196** | TDN04-135/P-75 | 0.0 | 0.0 | 2.9 | 1.7 | 0.6 | 80.0 | 10.0 | 2.7 | 0.3 | 0.8 | 0.0 |
| **TDN0400197** | TDN04-135/P-88 | 0.0 | 0.1 | 2.7 | 1.6 | 0.5 | 79.1 | 10.8 | 3.1 | 0.3 | 0.9 | 0.0 |

**Table 27 (continued).**

| Name | C22:0 | C22:1 | C24:0 | C24:1 | TOTSAT | Seed Weight | F2 Invader + Southern | Pedigree | | |
|---|---|---|---|---|---|---|---|---|---|---|
| **TDN0400210** | 0.4 | nd | 0.0 | 0.0 | 6.6 | 7.1 | | Nex 710 | | |
| **TDN0400211** | 0.4 | nd | 0.1 | 0.1 | 7.1 | 7.3 | | Nex 710 | | |
| **TDN0400212** | 0.4 | 0.0 | 0.1 | 0.0 | 7.2 | 7.7 | | Nex 710 | 6.96 | |
| **TDN0400201** | 0.0 | nd | 0.1 | nd | 3.9 | 8.2 | | 69-11.19 | | |
| **TDN0400202** | 0.0 | nd | 0.1 | nd | 3.9 | 7.6 | | 69-11.19 | | |
| **TDN0400203** | 0.1 | nd | 0.1 | nd | 3.8 | 7.3 | | 69-11.19 | 3.84 | 0.4005729 |
| **TDN0400204** | 0.1 | nd | 0.2 | 0.0 | 3.7 | 9.1 | | 218-11.30 | | |
| **TDN0400205** | 0.0 | nd | 0.1 | nd | 3.6 | 9.2 | | 218-11.30 | | |
| **TDN0400206** | 0.0 | 0.0 | 0.1 | 0.0 | 3.9 | 9.2 | | 218-11.30 | 3.75 | 0.4139063 |
| **TDN0400207** | 0.0 | 0.0 | 0.2 | nd | 3.7 | 10.7 | | 36-11.19 | | |
| **TDN0400208** | 0.1 | nd | 0.1 | nd | 3.7 | 9.8 | | 36-11.19 | | |
| **TDN0400209** | 0.0 | nd | 0.2 | nd | 3.8 | 8.5 | | 36-11.19 | 3.72 | 0.4191146 |
| **TDN0400198** | 0.3 | nd | 0.0 | 0.0 | 6.2 | 10.2 | null | 69-11.19/36-11.19(null) | 6.16 | |

Table 27 (continued).

| Name | C22:0 | C22:1 | C24:0 | C24:1 | TOTSAT | Seed Weight | F2 Invader + Southern | Pedigree | | |
|---|---|---|---|---|---|---|---|---|---|---|
| TDN0400199 | 0.3 | 0.0 | 0.1 | 0.1 | 6.4 | 8.6 | null | 69-11.19/218-11.30(null) | 6.43 | |
| TDN0400200 | 0.3 | nd | 0.1 | 0.0 | 6.5 | 11.3 | null | 218-11.30/36-11.19(null) | 6.52 | |
| TDN0400136 | 0.0 | nd | 0.2 | nd | 3.4 | 5.3 | stack | 69-11.19/36-11.19 | | 0.45 |
| TDN0400137 | 0.0 | nd | 0.3 | nd | 3.7 | 3 | stack | 69-11.19/36-11.19 | | 0.40 |
| TDN0400138 | 0.1 | 0.0 | 0.2 | nd | 3.5 | 7.6 | stack | 69-11.19/36-11.19 | | 0.43 |
| TDN0400139 | 0.0 | nd | 0.2 | nd | 3.5 | 4.2 | stack | 69-11.19/36-11.19 | | 0.43 |
| TDN0400140 | 0.1 | nd | 0.2 | nd | 3.5 | 2.4 | stack | 69-11.19/36-11.19 | | 0.43 |
| TDN0400141 | 0.1 | nd | 0.2 | nd | 3.1 | 1.3 | stack | 69-11.19/36-11.19 | | 0.50 |
| TDN0400142 | 0.0 | nd | 0.2 | nd | 3.0 | 4.8 | stack | 69-11.19/36-11.19 | | 0.51 |
| TDN0400143 | 0.1 | nd | 0.2 | nd | 4.0 | 8.5 | stack | 69-11.19/36-11.19 | | 0.35 |
| TDN0400144 | 0.0 | nd | 0.2 | nd | 3.7 | 8.5 | stack | 69-11.19/36-11.19 | | 0.40 |
| TDN0400145 | 0.0 | nd | 0.2 | nd | 3.1 | 5.8 | stack | 69-11.19/36-11.19 | | 0.51 |
| TDN0400146 | 0.1 | nd | 0.2 | nd | 4.0 | 8.9 | stack | 69-11.19/36-11.19 | | 0.36 |
| TDN0400147 | 0.1 | nd | 0.2 | nd | 3.9 | 6.6 | single | 69-11.19/36-11.19 | | 0.37 |
| TDN0400148 | 0.0 | nd | 0.3 | nd | 4.2 | 2.8 | single | 69-11.19/36-11.19 | | 0.32 |
| TDN0400149 | 0.1 | nd | 0.2 | nd | 3.6 | 6 | single | 69-11.19/36-11.19 | | 0.43 |
| TDN0400150 | 0.0 | nd | 0.2 | nd | 3.9 | 9.7 | single | 69-11.19/36-11.19 | | 0.38 |
| TDN0400151 | 0.1 | nd | 0.2 | nd | 3.2 | 1.7 | single | 69-11.19/36-11.19 | | 0.48 |
| TDN0400152 | 0.0 | nd | 0.2 | nd | 3.6 | 1.6 | single | 69-11.19/36-11.19 | | 0.42 |
| TDN0400153 | 0.0 | nd | 0.2 | nd | 3.7 | 6.4 | single | 69-11.19/36-11.19 | | 0.40 |
| TDN0400154 | 0.1 | 0.0 | 0.3 | nd | 3.5 | 4.1 | stack | 69-11.19/218-11.30 | | 0.45 |
| TDN0400155 | 0.0 | 0.0 | 0.2 | nd | 3.1 | 3.2 | stack | 69-11.19/218-11.30 | | 0.51 |
| TDN0400156 | 0.0 | nd | 0.2 | nd | 3.8 | 2.7 | stack | 69-11.19/218-11.30 | | 0.40 |
| TDN0400157 | 0.1 | 0.0 | 0.2 | nd | 4.0 | 7.7 | stack | 69-11.19/218-11.30 | | 0.37 |
| TDN0400158 | 0.1 | nd | 0.2 | nd | 3.1 | 1.4 | stack | 69-11.19/218-11.30 | | 0.52 |
| TDN0400159 | 0.0 | nd | 0.2 | nd | 3.6 | 6.4 | stack | 69-11.19/218-11.30 | | 0.44 |
| TDN0400160 | 0.0 | nd | 0.2 | 0.0 | 2.8 | 1.2 | stack | 69-11.19/218-11.30 | | 0.56 |
| TDN0400161 | 0.0 | nd | 0.2 | nd | 3.3 | 4.5 | stack | 69-11.19/218-11.30 | | 0.49 |
| TDN0400162 | 0.1 | nd | 0.3 | nd | 3.2 | 3 | stack | 69-11.19/218-11.30 | | 0.49 |
| TDN0400163 | 0.0 | nd | 0.2 | nd | 3.5 | 1.9 | stack | 69-11.19/218-11.30 | | 0.45 |
| TDN0400164 | 0.1 | nd | 0.2 | nd | 3.8 | 2.4 | stack | 69-11.19/218-11.30 | | 0.40 |
| TDN0400165 | 0.1 | 0.0 | 0.2 | nd | 4.1 | 1.1 | stack | 69-11.19/218-11.30 | | 0.36 |

**Table 27 (continued).**

| Name | C22:0 | C22:1 | C24:0 | C24:1 | TOTSAT | Seed Weight | F2 Invader + Southern | Pedigree | |
|---|---|---|---|---|---|---|---|---|---|
| TDN0400166 | 0.0 | nd | 0.2 | nd | 3.5 | 6.1 | stack | 69-11.19/218-11.30 | 0.45 |
| TDN0400167 | 0.1 | nd | 0.2 | nd | 3.8 | 7.2 | stack | 69-11.19/218-11.30 | 0.41 |
| TDN0400168 | 0.0 | nd | 0.2 | nd | 3.4 | 1.6 | stack | 69-11.19/218-11.30 | 0.47 |
| TDN0400169 | 0.2 | nd | 0.2 | nd | 4.6 | 6.7 | single | 69-11.19/218-11.30 | 0.28 |
| TDN0400170 | 0.1 | nd | 0.2 | nd | 3.5 | 1.8 | single | 69-11.19/218-11.30 | 0.45 |
| TDN0400171 | 0.0 | nd | 0.2 | nd | 3.4 | 5 | single | 69-11.19/218-11.30 | 0.47 |
| TDN0400172 | 0.1 | nd | 0.2 | nd | 4.5 | 8.5 | single | 69-11.19/218-11.30 | 0.30 |
| TDN0400173 | 0.0 | 0.0 | 0.2 | nd | 3.6 | 7.3 | stack | 218-11.30/36-11.19 | 0.44 |
| TDN0400174 | 0.1 | nd | 0.2 | nd | 3.3 | 6.9 | stack | 218-11.30/36-11.19 | 0.49 |
| TDN0400175 | 0.0 | nd | 0.2 | 0.0 | 3.2 | 6.1 | stack | 218-11.30/36-11.19 | 0.50 |
| TDN0400176 | 0.0 | 0.0 | 0.2 | nd | 3.4 | 4.5 | stack | 218-11.30/36-11.19 | 0.48 |
| TDN0400177 | 0.1 | nd | 0.2 | 0.0 | 3.5 | 5.9 | stack | 218-11.30/36-11.19 | 0.46 |
| TDN0400178 | 0.1 | nd | 0.2 | nd | 3.7 | 3.8 | stack | 218-11.30/36-11.19 | 0.44 |
| TDN0400179 | 0.0 | nd | 0.3 | nd | 3.4 | 4.2 | stack | 218-11.30/36-11.19 | 0.47 |
| TDN0400180 | 0.1 | nd | 0.3 | nd | 3.7 | 2.8 | stack | 218-11.30/36-11.19 | 0.43 |
| TDN0400181 | 0.0 | nd | 0.2 | nd | 3.5 | 6.4 | stack | 218-11.30/36-11.19 | 0.46 |
| TDN0400182 | 0.1 | nd | 0.2 | 0.0 | 3.5 | 8.3 | stack | 218-11.30/36-11.19 | 0.47 |
| TDN0400183 | 0.0 | nd | 0.2 | nd | 3.5 | 6.7 | stack | 218-11.30/36-11.19 | 0.47 |
| TDN0400184 | 0.1 | nd | 0.2 | nd | 3.4 | 9 | stack | 218-11.30/36-11.19 | 0.48 |
| TDN0400185 | 0.0 | nd | 0.1 | nd | 3.4 | 10.1 | stack | 218-11.30/36-11.19 | 0.47 |
| TDN0400186 | 0.0 | nd | 0.1 | 0.0 | 4.0 | 8.3 | stack | 218-11.30/36-11.19 | 0.38 |
| TDN0400187 | 0.0 | nd | 0.2 | 0.0 | 3.7 | 5.2 | stack | 218-11.30/36-11.19 | 0.43 |
| TDN0400188 | 0.1 | nd | 0.2 | 0.0 | 3.6 | 5.8 | single | 218-11.30/36-11.19 | 0.44 |
| TDN0400189 | 0.0 | nd | 0.1 | nd | 3.7 | 8.1 | single | 218-11.30/36-11.19 | 0.43 |
| TDN0400190 | 0.0 | nd | 0.2 | nd | 4.0 | 0.1 | single | 218-11.30/36-11.19 | 0.38 |
| TDN0400191 | 0.2 | nd | 0.2 | nd | 3.7 | 5.4 | single | 218-11.30/36-11.19 | 0.43 |
| TDN0400192 | 0.0 | nd | 0.2 | 0.0 | 3.5 | 6.4 | single | 218-11.30/36-11.19 | 0.46 |
| TDN0400193 | 0.0 | 0.0 | 0.2 | nd | 3.8 | 7.6 | single | 218-11.30/36-11.19 | 0.41 |
| TDN0400194 | 0.1 | nd | 0.1 | 0.0 | 4.1 | 7.5 | single | 218-11.30/36-11.19 | 0.36 |
| TDN0400195 | 0.1 | nd | 0.1 | nd | 4.2 | 9.4 | single | 218-11.30/36-11.19 | 0.36 |
| TDN0400196 | 0.1 | nd | 0.1 | nd | 4.1 | 7.3 | single | 218-11.30/36-11.19 | 0.37 |
| TDN0400197 | 0.1 | nd | 0.2 | 0.0 | 3.9 | 8 | single | 218-11.30/36-11.19 | 0.40 |

**Table 28,**

| **Name** | **Pedigree** | Source | Generation | **C16:0** | **C16:1** | **C18:0** | **C18:1** | **C18:2** | **C18:3** | **TOTSAT** | Follow-up done to confirm: | Seed (g) |
|---|---|---|---|---|---|---|---|---|---|---|---|---|
| **TDN0400211** | Nex710 | NEX 710 | | 4.37 | 0.34 | 1.46 | 73.94 | 13.53 | 2.91 | 7.08 | | |
| **TDN0400202** | 69-11.19 | TDN04-123 | T7 | 2.97 | 1.59 | 0.47 | 79.3 | 10.86 | 2.85 | 3.86 | | |
| **TDN0400204** | 218-11.30 | TDN04-128 | T6 | 2.66 | 1.9 | 0.49 | 78.85 | 10.98 | 3.01 | 3.73 | | |
| **TDN0400208** | 36-11.19 | TDN04-132 | T6 | 2.51 | 1.95 | 0.58 | 79.45 | 10.73 | 2.52 | 3.69 | | 10.2 |
| **TDN0400198** | 69-11.19/36-11.19 (null) | TDN04-133/P-116 | F3 | 4.05 | 0.29 | 1.2 | 75.27 | 12.77 | 3.42 | 6.16 | | 8.6 |
| **TDN0400199** | 69-11.19/216-11.30 (nu!i) | TDN04-134/P11 | F3 | 4.16 | 0.29 | 1.24 | 73.67 | 14.17 | 3.4 | 6.43 | | 1.3 |
| **TDN0400141** | 69-11.19/36-11.19 | TDN04-133/P70 | F3 | 2.24 | 2.36 | 0.32 | 80.3 | 9.3 | 3.23 | 3.1 | homo Stack | 4.8 |
| **TDN0400142** | 69-11.19/36-11.19 | TDN04-133/P114 | F3 | 2.15 | 2.38 | 0.3 | 79.89 | 9.77 | 3.17 | 3.02 | homo Stack | 5.8 |
| **TDN0400145** | 69-11.19/36-11.19 | TDN04-133/P129 | F3 | 2.34 | 2.09 | 0.37 | 81.23 | 9.12 | 2.7 | 3.07 | homo Stack | 3.2 |
| **TDN0400155** | 69-11.19/218-11.30 | TDN04-134/P-32 | F3 | 2.38 | 2.31 | 0.42 | 79.55 | 10.15 | 3.4 | 3.12 | homo Stack | 1.4 |
| **TDN0400158** | 69-11.19/218-11.30 | TDN04-134/P-38 | F3 | 2.2 | 2.36 | 0.29 | 80.14 | 9.44 | 3.36 | 3.05 | homo Stack | 1.2 |
| **TDN0400160** | 69-11.19/218-11.30 | TDN04-134/P-48 | F3 | 2.23 | 2.27 | 0.29 | 81.88 | 8.47 | 2.97 | 2.81 | homo Stack | 8.1 |
| **TDN0400189** | 218-11.30/36-11.19 | TDN04-135/P-31 | F3 | 2.69 | 1.65 | 0.55 | 80.3 | 10.07 | 2.78 | 3.7 | homo 36 | 8.5 |
| **TDN0400143** | 69-11.19/36-11.19 | TDN04-133/P121 | F3 | 2.82 | 1.66 | 0.62 | 79.6 | 10.65 | 2.46 | 4.04 | homo 69 | 8.0 |
| **TDN0400197** | 218-11.30/36-11.19 | TDN04-135/P-88 | F3 | 2.75 | 1.64 | 0.54 | 79.11 | 10.78 | 3.06 | 3.89 | homo 218 | 7.2 |
| **TDN0400167** | 69-11.19/218-11.30 | TDN04-134/P-119 | F3 | 2.64 | 1.94 | 0.51 | 80.54 | 9.4 | 2.83 | 3.8 | homo 218 | 9.0 |
| **TDN0400184** | 298-11.30/36-11.19 | TDN04-135/P-112 | F3 | 2.4 | 2.26 | 0.42 | 81.28 | 8.89 | 2.7 | 3.4 | homo 218 | |

**Table 29.**

| Name | Generation | Source Pop | Event Name | QA Lab ID | C12:0 | C14:0 | C16:0 | C16:1 |
|---|---|---|---|---|---|---|---|---|
| Nex 710 | DH | Polo/SVO95-09 | Natreon | 05-147-0001 | 0.0 | 0.1 | 3.5 | 0.2 |
| Nex 710 | DH | Polo/SVO95-09 | Natreon | 05-147-0002 | 0.0 | 0.1 | 3.7 | 0.2 |
| Nex 710 | DH | Polo/SVO95-09 | Natreon | 05-147-0003 | 0.0 | 0.1 | 3.7 | 0.2 |
| Nex 710 | DH | Polo/SVO95-09 | Natreon | 05-147-0004 | 0.0 | 0.1 | 3.6 | 0.2 |
| Nex 710 | DH | Polo/SVO95-09 | Natreon | 05-147-0005 | 0.0 | 0.1 | 3.7 | 0.2 |
| Nex 710 | DH | Polo/SVO95-09 | Natreon | 05-147-0006 | 0.0 | 0.0 | 3.7 | 0.2 |
| Nex 710 | DH | Polo/SVO95-09 | Natreon | 05-147-0007 | 0.0 | 0.1 | 3.7 | 0.3 |
| Nex 710 | DH | Polo/SVO95-09 | Natreon | 05-147-0008 | 0.0 | 0.0 | 3.7 | 0.2 |
| Nex 710 | DH | Polo/SVO95-09 | Natreon | 05-147-0009 | 0.0 | 0.1 | 3.8 | 0.2 |
| TDN0400141-1 | F4 | 03TGH01538/03TGH01099 | 69.11.19::36.11.19 | 05-147-0010 | 0.0 | 0.0 | 2.3 | 1.8 |
| TDN0400141-2 | F4 | 03TGH01538/03TGH01099 | 69.11.19::36.11.19 | 05-147-0011 | 0.0 | 0.0 | 2.3 | 2.3 |
| TDN0400141-3 | F4 | 03TGH01538/03TGH01099 | 69.11.19::36.11.19 | 05-147-0012 | 0.0 | 0.0 | 2.2 | 2.3 |
| TDN0400141-4 | F4 | 03TGH01538/03TGH01099 | 69.11.19::36.11.19 | 05-147-0013 | 0.0 | 0.1 | 2.2 | 2.2 |
| TDN0400141-5 | F4 | 03TGH01538/03TGH01099 | 69.11.19::36.11.19 | 05-147-0014 | 0.0 | 0.0 | 2.2 | 2.1 |
| TDN0400141-6 | F4 | 03TGH01538/03TGH01099 | 69.11.19::36.11.19 | 05-147-0015 | 0.0 | 0.1 | 2.2 | 2.3 |
| TDN0400141-7 | F4 | 03TGH01538/03TGH01099 | 69.11.19::36.11.19 | 05-147-0016 | 0.0 | 0.1 | 2.2 | 2.5 |
| TDN0400141-8 | F4 | 03TGH01538/03TGH01099 | 69.11.19::36.11.19 | 05-147-0017 | 0.0 | 0.0 | 2.1 | 2.3 |
| TDN0400141-9 | F4 | 03TGH01538/03TGH01099 | 69.11.19::36.11.19 | 05-147-0018 | 0.0 | 0.1 | 2.1 | 2.2 |
| TDN0400142-1 | F4 | 03TGH01538/03TGH01099 | 69.11.19::36.11.19 | 05-147-0019 | 0.0 | 0.1 | 2.0 | 2.1 |
| TDN0400142-2 | F4 | 03TGH01538/03TGH01099 | 69.11.19::36.11.19 | 05-147-0020 | 0.0 | 0.0 | 2.0 | 2.3 |
| TDN0400142-3 | F4 | 03TGH01538/03TGH01099 | 69.11.19::36.11.19 | 05-147-0021 | 0.0 | 0.0 | 2.1 | 2.0 |
| TDN0400142-4 | F4 | 03TGH01538/03TGH01099 | 69.11.19::36.11.19 | 05-147-0022 | 0.0 | 0.0 | 2.0 | 2.4 |
| TDN0400142-5 | F4 | 03TGH01538/03TGH01099 | 69.11.19::36.11.19 | 05-147-0023 | 0.0 | 0.1 | 2.1 | 2.3 |
| TDN0400142-6 | F4 | 03TGH01538/03TGH01099 | 69.11.19::36.11.19 | 05-147-0024 | 0.0 | 0.0 | 2.1 | 2.1 |
| TDN0400142-7 | F4 | 03TGH01538/03TGH01099 | 69.11.19::36.11.19 | 05-147-0025 | 0.0 | 0.0 | 2.0 | 2.1 |
| TDN0400142-8 | F4 | 03TGH01538/03TGH01099 | 69.11.19::36.11.19 | 05-147-0026 | 0.0 | 0.1 | 2.0 | 2.1 |
| TDN0400142-9 | F4 | 03TGH01538/03TGH01099 | 69.11.19::36.11.19 | 05-147-0027 | 0.0 | 0.0 | 2.1 | 2.0 |
| TDN0400143-1 | F4 | 03TGH01538/03TGH01099 | 36.11.19 (from 69.11.19::36.11.19) | 05-147-0028 | 0.0 | 0.0 | 2.4 | 1.7 |
| TDN0400143-2 | F4 | 03TGH01538/03TGH01099 | 36.11.19 (from 69.11.19::36.11.19) | 05-147-0029 | 0.0 | 0.1 | 2.3 | 1.5 |
| TDN0400143-3 | F4 | 03TGH01538/03TGH01099 | 36.11.19 (from 69.11.19::36.11.19) | 05-147-0030 | 0.0 | 0.1 | 2.5 | 1.6 |
| TDN0400143-4 | F4 | 03TGH01538/03TGH01099 | 36.11.19 (from 69.11.19::36.11.19) | 05-147-0031 | 0.0 | 0.1 | 2.6 | 1.4 |

**Table 29.**

| Name | Generation | Source Pop | Event Name | QA Lab ID | C12:0 | C14:0 | C16:0 | C16:1 |
|---|---|---|---|---|---|---|---|---|
| TDN0400143-5 | F4 | 03TGH01538/03TGH01099 | 36.11.19 (from 69.11.19::36.11.19) | 05-147-0032 | 0.0 | 0.1 | 2.4 | 1.8 |
| TDN0400143-6 | F4 | 03TGH01538/03TGH01099 | 36.11.19 (from 69.11.19::36.11.19) | 05-147-0033 | 0.0 | 0.0 | 2.3 | 1.7 |
| TDN0400143-7 | F4 | 03TGH01538/03TGH01099 | 36.11.19 (from 69.11.19::36.11.19) | 05-147-0034 | 0.0 | 0.1 | 2.6 | 1.3 |
| TDN0400143-8 | F4 | 03TGH01538/03TGH01099 | 36.11.19 (from 69.11.19::36.11.19) | 05-147-0035 | 0.0 | 0.0 | 2.7 | 1.6 |
| TDN0400143-9 | F4 | 03TGH01538/03TGH01099 | 36.11.19 (from 69.11.19::36.11.19) | 05-147-0036 | 0.0 | 0.1 | 2.3 | 1.7 |
| TDN0400145-1 | F4 | 03TGH01538/03TGH01099 | 69.11.19::36.11.19 | 05-147-0037 | 0.0 | 0.1 | 2.4 | 1.6 |
| TDN0400145-2 | F4 | 03TGH01538/03TGH01099 | 69.11.19::36.11.19 | 05-147-0038 | 0.0 | 0.0 | 2.7 | 1.4 |
| TDN0400145-3 | F4 | 03TGH01538/03TGH01099 | 69.11.19::36.11.19 | 05-147-0039 | 0.0 | 0.1 | 2.3 | 1.7 |
| TDN0400145-4 | F4 | 03TGH01538/03TGH01099 | 69.11.19::36.11.19 | 05-147-0040 | 0.0 | 0.1 | 2.3 | 1.9 |
| TDN0400145-5 | F4 | 03TGH01538/03TGH01099 | 69.11.19::36.11.19 | 05-147-0041 | 0.0 | 0.1 | 2.4 | 1.6 |
| TDN0400145-6 | F4 | 03TGH01538/03TGH01099 | 69.11.19::36.11.19 | 05-147-0042 | 0.0 | 0.1 | 2.2 | 2.0 |
| TDN0400145-7 | F4 | 03TGH01538/03TGH01099 | 69.11.19::36.11.19 | 05-147-0043 | 0.0 | 0.0 | 2.3 | 2.0 |
| TDN0400145-8 | F4 | 03TGH01538/03TGH01099 | 69.11.19::36.11.19 | 05-147-0044 | 0.0 | 0.0 | 2.2 | 1.9 |
| TDN0400145-9 | F4 | 03TGH01538/03TGH01099 | 69.11.19::36.11.19 | 05-147-0045 | 0.0 | 0.0 | 2.1 | 2.2 |
| TDN0400155-1 | F4 | 03TGH01538/03TGH02193 | 69.11.19::218.11.30 | 05-147-0046 | | | | |
| TDN0400155-2 | F4 | 03TGH01538/03TGH02193 | 69.11.19::218.11.30 | 05-147-0047 | | | | |
| TDN0400155-3 | F4 | 03TGH01538/03TGH02193 | 69.11.19::218.11.30 | 05-147-0048 | 0.0 | 0.0 | 2.5 | 1.8 |
| TDN0400155-4 | F4 | 03TGH01538/03TGH02193 | 69.11.19::218.11.30 | 05-147-0049 | 0.0 | 0.1 | 2.2 | 2.0 |
| TDN0400155-5 | F4 | 03TGH01538/03TGH02193 | 69.11.19::218.11.30 | 05-147-0050 | | | | |
| TDN0400155-6 | F4 | 03TGH01538/03TGH02193 | 69.11.19::218.11.30 | 05-147-0051 | 0.0 | 0.0 | 2.4 | 2.0 |
| TDN0400155-7 | F4 | 03TGH01538/03TGH02193 | 69.11.19::218.11.30 | 05-147-0052 | 0.0 | 0.1 | 2.6 | 1.8 |
| TDN0400155-8 | F4 | 03TGH01538/03TGH02193 | 69.11.19::218.11.30 | 05-147-0053 | 0.0 | 0.0 | 2.2 | 2.1 |
| TDN0400155-9 | F4 | 03TGH01538/03TGH02193 | 69.11.19::218.11.30 | 05-147-0054 | 0.0 | 0.1 | 2.4 | 2.0 |
| TDN0400158-1 | F4 | 03TGH01538/03TGH02193 | 69.11.19::218.11.30 | 05-147-0055 | 0.0 | 0.0 | 2.2 | 2.1 |
| TDN0400158-2 | F4 | 03TGH01538/03TGH02193 | 69.11.19::218.11.30 | 05-147-0056 | 0.0 | 0.0 | 2.6 | 1.4 |
| TDN0400158-3 | F4 | 03TGH01538/03TGH02193 | 69.11.19::218.11.30 | 05-147-0057 | 0.0 | 0.0 | 2.2 | 1.9 |
| TDN0400158-4 | F4 | 03TGH01538/03TGH02193 | 69.11.19::218.11.30 | 05-147-0058 | 0.0 | 0.0 | 2.3 | 1.8 |
| TDN0400158-5 | F4 | 03TGH01538/03TGH02193 | 69.11.19::218.11.30 | 05-147-0059 | | | | |
| TDN0400158-6 | F4 | 03TGH01538/03TGH02193 | 69.11.19::218.11.30 | 05-147-0060 | 0.0 | 0.0 | 2.1 | 2.4 |
| TDN0400158-7 | F4 | 03TGH01538/03TGH02193 | 69.11.19::218.11.30 | 05-147-0061 | | | | |
| TDN0400158-8 | F4 | 03TGH01538/03TGH02193 | 69.11.19::218.11.30 | 05-147-0062 | | | | |
| TDN0400158-9 | F4 | 03TGH01538/03TGH02193 | 69.11.19::218.11.30 | 05-147-0063 | 0.0 | 0.0 | 2.1 | 2.5 |

**Table 29.**

| Name | Generation | Source Pop | Event Name | QA Lab ID | C12:0 | C14:0 | C16:0 | C16:1 |
|---|---|---|---|---|---|---|---|---|
| TDN0400160-1 | F4 | 03TGH01538/03TGH02193 | 69.11.19::218.11.30 | 05-147-0064 | 0.0 | 0.0 | 2.2 | 1.9 |
| TDN0400160-2 | F4 | 03TGH01538/03TGH02193 | 69.11.19::218.11.30 | 05-147-0065 | 0.0 | 0.0 | 2.2 | 2.0 |
| TDN0400160-3 | F4 | 03TGH01538/03TGH02193 | 69.11.19::218.11.30 | 05-147-0066 | 0.0 | 0.0 | 2.2 | 2.0 |
| TDN0400160-4 | F4 | 03TGH01538/03TGH02193 | 69.11.19::218.11.30 | 05-147-0067 | 0.0 | 0.0 | 2.6 | 1.6 |
| TDN0400160-5 | F4 | 03TGH01538/03TGH02193 | 69.11.19::218.11.30 | 05-147-0068 | 0.0 | 0.0 | 2.2 | 2.2 |
| TDN0400160-6 | F4 | 03TGH01538/03TGH02193 | 69.11.19::218.11.30 | 05-147-0069 | 0.0 | 0.0 | 2.2 | 2.1 |
| TDN0400160-7 | F4 | 03TGH01538/03TGH02193 | 69.11.19::218.11.30 | 05-147-0070 | 0.0 | 0.0 | 2.1 | 2.3 |
| TDN0400160-8 | F4 | 03TGH01538/03TGH02193 | 69.11.19::218.11.30 | 05-147-0071 | 0.0 | 0.1 | 2.1 | 2.2 |
| TDN0400160-9 | F4 | 03TGH01538/03TGH02193 | 69.11.19::218.11.30 | 05-147-0072 | | | | |
| TDN0400167-1 | F4 | 03TGH01538/03TGH02193 | 218.11.30 (from 69.11.19::218.11.30) | 05-147-0073 | 0.0 | 0.0 | 2.5 | 1.7 |
| TDN0400167-2 | F4 | 03TGH01538/03TGH02193 | 218.11.30 (from 69.11.19::218.11.30) | 05-147-0074 | 0.0 | 0.1 | 2.4 | 1.8 |
| TDN0400167-3 | F4 | 03TGH01538/03TGH02193 | 218.11.30 (from 69.11.19::218.11.30) | 05-147-0075 | 0.0 | 0.1 | 2.5 | 1.7 |
| TDN0400167-4 | F4 | 03TGH01538/03TGH02193 | 218.11.30 (from 69.11.19::218.11.30) | 05-147-0076 | 0.0 | 0.0 | 2.5 | 1.7 |
| TDN0400167-5 | F4 | 03TGH01538/03TGH02193 | 218.11.30 (from 69.11.19::218.11.30) | 05-147-0077 | 0.0 | 0.0 | 2.5 | 1.7 |
| TDN0400167-6 | F4 | 03TGH01538/03TGH02193 | 218.11.30 (from 69.11.19::218.11.30) | 05-147-0078 | 0.0 | 0.0 | 2.5 | 1.6 |
| TDN0400167-7 | F4 | 03TGH01538/03TGH02193 | 218.11.30 (from 69.11.19::218.11.30) | 05-147-0079 | 0.0 | 0.0 | 2.4 | 1.9 |
| TDN0400167-8 | F4 | 03TGH01538/03TGH02193 | 218.11.30 (from 69.11.19::218.11.30) | 05-147-0080 | 0.0 | 0.0 | 2.5 | 1.7 |
| TDN0400167-9 | F4 | 03TGH01538/03TGH02193 | 218.11.30 (from 69.11.19::218.11.30) | 05-147-0081 | 0.0 | 0.1 | 2.5 | 1.7 |
| TDN0400184-1 | F4 | 03TGH02193/03TGH01099 | 218.11.30 (from 218.11.30::36-11.19) | 05-147-0082 | 0.0 | 0.1 | 2.3 | 1.8 |
| TDN0400184-2 | F4 | 03TGH02193/03TGH01099 | 218.11.30 (from 218.11.30::36-11.19) | 05-147-0083 | 0.0 | 0.1 | 2.4 | 1.8 |
| TDN0400184-3 | F4 | 03TGH02193/03TGH01099 | 218.11.30 (from 218.11.30::36-11.19) | 05-147-0084 | 0.0 | 0.0 | 2.2 | 2.0 |
| TDN0400184-4 | F4 | 03TGH02193/03TGH01099 | 218.11.30 (from 218.11.30::36-11.19) | 05-147-0085 | 0.0 | 0.1 | 2.1 | 2.2 |
| TDN0400184-5 | F4 | 03TGH02193/03TGH01099 | 218.11.30 (from 218.11.30::36-11.19) | 05-147-0086 | 0.0 | 0.1 | 2.3 | 2.1 |
| TDN0400184-6 | F4 | 03TGH02193/03TGH01099 | 218.11.30 (from 218.11.30::36-11.19) | 05-147-0087 | 0.0 | 0.1 | 2.3 | 2.0 |
| TDN0400184-7 | F4 | 03TGH02193/03TGH01099 | 218.11.30 (from 218.11.30::36-11.19) | 05-147-0088 | 0.0 | 0.1 | 2.3 | 2.0 |
| TDN0400184-8 | F4 | 03TGH02193/03TGH01099 | 218.11.30 (from 218.11.30::36-11.19) | 05-147-0089 | 0.0 | 0.1 | 2.2 | 2.1 |
| TDN0400184-9 | F4 | 03TGH02193/03TGH01099 | 218.11.30 (from 218.11.30::36-11.19) | 05-147-0090 | 0.0 | 0.0 | 2.4 | 2.0 |
| TDN0400189-1 | F4 | 03TGH02193/03TGH01099 | 36-11.19 (from 218.11.30::36-11.19) | 05-147-0091 | 0.0 | 0.1 | 2.8 | 1.5 |
| TDN0400189-2 | F4 | 03TGH02193/03TGH01099 | 36-11.19 (from 218.11.30::36-11.19) | 05-147-0092 | 0.0 | 0.0 | 2.8 | 1.0 |
| TDN0400189-3 | F4 | 03TGH02193/03TGH01099 | 36-11.19 (from 218.11.30::36-11.19) | 05-147-0093 | 0.0 | 0.1 | 2.5 | 1.6 |
| TDN0400189-4 | F4 | 03TGH02193/03TGH01099 | 36-11.19 (from 218.11.30::36-11.19) | 05-147-0094 | 0.0 | 0.0 | 2.7 | 1.1 |
| TDN0400189-5 | F4 | 03TGH02193/03TGH01099 | 36-11.19 (from 218.11.30::36-11.19) | 05-147-0095 | 0.0 | 0.0 | 2.4 | 1.7 |

**Table 29.**

| Name | Gener-ation | Source Pop | Event Name | QA Lab ID | C12:0 | C14:0 | C16:0 | C16:1 |
|---|---|---|---|---|---|---|---|---|
| TDN0400189-6 | F4 | 03TGH02193/03TGH01099 | 36-11.19 (from 218.11.30::36-11.19) | 05-147-0096 | 0.0 | 0.1 | 2.5 | 1.8 |
| TDN0400189-7 | F4 | 03TGH02193/03TGH01099 | 36-11.19 (from 218.11.30::36-11.19) | 05-147-0097 | 0.0 | 0.0 | 2.7 | 1.5 |
| TDN0400189-8 | F4 | 03TGH02193/03TGH01099 | 36-11.19 (from 218.11.30::36-11.19) | 05-147-0098 | 0.0 | 0.0 | 2.6 | 1.2 |
| TDN0400189-9 | F4 | 03TGH02193/03TGH01099 | 36-11.19 (from 218.11.30::36-11.19) | 05-147-0099 | 0.0 | 0.1 | 2.7 | 1.3 |
| TDN0400197-1 | F4 | 03TGH02193/03TGH01099 | 218.11.30 (from 218.11.30::36-11.19) | 05-147-0100 | 0.0 | 0.1 | 3.0 | 1.5 |
| TDN0400197-2 | F4 | 03TGH02193/03TGH01099 | 218.11.30 (from 218.11.30::36-11.19) | 05-147-0101 | 0.0 | 0.1 | 2.5 | 1.9 |
| TDN0400197-3 | F4 | 03TGH02193/03TGH01099 | 218.11.30 (from 218.11.30::36-11.19) | 05-147-0102 | 0.0 | 0.0 | 2.6 | 1.7 |
| TDN0400197-4 | F4 | 03TGH02193/03TGH01099 | 218.11.30 (from 218.11.30::36-11.19) | 05-147-0103 | 0.0 | 0.1 | 2.5 | 1.8 |
| TDN0400197-5 | F4 | 03TGH02193/03TGH01099 | 218.11.30 (from 218.11.30::36-11.19) | 05-147-0104 | 0.0 | 0.1 | 2.7 | 1.7 |
| TDN0400197-6 | F4 | 03TGH02193/03TGH01099 | 218.11.30 (from 218.11.30::36-11.19) | 05-147-0105 | 0.0 | 0.1 | 2.8 | 1.5 |
| TDN0400197-7 | F4 | 03TGH02193/03TGH01099 | 218.11.30 (from 218.11.30::36-11.19) | 05-147-0106 | 0.0 | 0.1 | 2.8 | 2.5 |
| TDN0400197-8 | F4 | 03TGH02193/03TGH01099 | 218.11.30 (from 218.11.30::36-11.19) | 05-147-0107 | 0.0 | 0.1 | 2.5 | 1.4 |
| TDN0400197-9 | F4 | 03TGH02193/03TGH01099 | 218.11.30 (from 218.11.30::36-11.19) | 05-147-0108 | 0.0 | 0.1 | 2.7 | 1.7 |
| TDN0400198-1 | F4 | 03TGH01538/03TGH01099 | null (from 69-11.19::36-11.19) | 05-147-0109 | 0.0 | 0.1 | 3.7 | 0.3 |
| TDN0400198-2 | F4 | 03TGH01538/03TGH01099 | null (from 69-11.19::36-11.19) | 05-147-0110 | 0.0 | 0.1 | 3.9 | 0.3 |
| TDN0400198-3 | F4 | 03TGH01538/03TGH01099 | null (from 69-11.19::36-11.19) | 05-147-0111 | 0.0 | 0.1 | 3.7 | 0.3 |
| TDN0400198-4 | F4 | 03TGH01538/03TGH01099 | null (from 69-11.19::36-11.19) | 05-147-0112 | 0.0 | 0.1 | 3.6 | 0.3 |
| TDN0400198-5 | F4 | 03TGH01538/03TGH01099 | null (from 69-11.19::36-11.19) | 05-147-0113 | 0.0 | 0.1 | 3.7 | 0.3 |
| TDN0400198-6 | F4 | 03TGH01538/03TGH01099 | null (from 69-11.19::36-11.19) | 05-147-0114 | 0.0 | 0.1 | 3.8 | 0.3 |
| TDN0400198-7 | F4 | 03TGH01538/03TGH01099 | null (from 69-11.19::36-11.19) | 05-147-0115 | 0.0 | 0.1 | 3.7 | 0.3 |
| TDN0400198-8 | F4 | 03TGH01538/03TGH01099 | null (from 69-11.19::36-11.19) | 05-147-0116 | 0.0 | 0.1 | 3.7 | 0.3 |
| TDN0400198-9 | F4 | 03TGH01538/03TGH01099 | null (from 69-11.19::36-11.19) | 05-147-0117 | 0.0 | 0.1 | 3.5 | 0.3 |
| TDN0400199-1 | F4 | 03TGH01538/03TGH02193 | null (from 69-11.19/218-11.30) | 05-147-0118 | 0.0 | 0.1 | 3.7 | 0.3 |
| TDN0400199-2 | F4 | 03TGH01538/03TGH02193 | null (from 69-11.19/218-11.30) | 05-147-0119 | 0.0 | 0.0 | 3.7 | 0.3 |
| TDN0400199-3 | F4 | 03TGH01538/03TGH02193 | null (from 69-11.19/218-11.30) | 05-147-0120 | 0.0 | 0.1 | 3.6 | 0.3 |
| TDN0400199-4 | F4 | 03TGH01538/03TGH02193 | null (from 69-11.19/218-11.30) | 05-147-0121 | 0.0 | 0.1 | 3.6 | 0.2 |
| TDN0400199-5 | F4 | 03TGH01538/03TGH02193 | null (from 69-11.19/218-11.30) | 05-147-0122 | 0.0 | 0.0 | 3.7 | 0.3 |
| TDN0400199-6 | F4 | 03TGH01538/03TGH02193 | null (from 69-11.19/218-11.30) | 05-147-0123 | 0.0 | 0.0 | 3.9 | 0.3 |
| TDN0400199-7 | F4 | 03TGH01538/03TGH02193 | null (from 69-11.19/218-11.30) | 05-147-0124 | 0.0 | 0.1 | 3.7 | 0.3 |
| TDN0400199-8 | F4 | 03TGH01538/03TGH02193 | null (from 69-11.19/218-11.30) | 05-147-0125 | 0.0 | 0.0 | 3.7 | 0.3 |
| TDN0400199-9 | F4 | 03TGH01538/03TGH02193 | null (from 69-11.19/218-11.30) | 05-147-0126 | 0.0 | 0.1 | 3.6 | 0.3 |
| TDN0400202-1 | T8 | 02TGH00038 | 69.11.19 | 05-147-0127 | 0.0 | 0.0 | 2.5 | 1.6 |

**Table 29.**

| Name | Generation | Source Pop | Event Name | QA Lab ID | C12:0 | C14:0 | C16:0 | C16:1 |
|---|---|---|---|---|---|---|---|---|
| TDN0400202-2 | T8 | 02TGH00038 | 69.11.19 | 05-147-0128 | 0.0 | 0.0 | 2.6 | 1.5 |
| TDN0400202-3 | T8 | 02TGH00038 | 69.11.19 | 05-147-0129 | 0.0 | 0.0 | 2.6 | 1.4 |
| TDN0400202-4 | T8 | 02TGH00038 | 69.11.19 | 05-147-0130 | 0.0 | 0.0 | 2.5 | 1.5 |
| TDN0400202-5 | T8 | 02TGH00038 | 69.11.19 | 05-147-0131 | 0.0 | 0.0 | 2.4 | 1.8 |
| TDN0400202-6 | T8 | 02TGH00038 | 69.11.19 | 05-147-0132 | 0.0 | 0.0 | 2.6 | 1.4 |
| TDN0400202-7 | T8 | 02TGH00038 | 69.11.19 | 05-147-0133 | 0.0 | 0.1 | 2.5 | 1.4 |
| TDN0400202-8 | T8 | 02TGH00038 | 69.11.19 | 05-147-0134 | 0.0 | 0.0 | 2.4 | 1.5 |
| TDN0400202-9 | T8 | 02TGH00038 | 69.11.19 | 05-147-0135 | 0.0 | 0.0 | 2.5 | 1.5 |
| TDN0400204-1 | T7 | 02TGH00032 | 218.11.30 | 05-147-0136 | 0.0 | 0.0 | 2.3 | 1.8 |
| TDN0400204-2 | T7 | 02TGH00032 | 218.11.30 | 05-147-0137 | 0.0 | 0.0 | 2.4 | 1.8 |
| TDN0400204-3 | T7 | 02TGH00032 | 218.11.30 | 05-147-0138 | 0.0 | 0.0 | 2.3 | 1.8 |
| TDN0400204-4 | T7 | 02TGH00032 | 218.11.30 | 05-147-0139 | 0.0 | 0.0 | 2.4 | 1.7 |
| TDN0400204-5 | T7 | 02TGH00032 | 218.11.30 | 05-147-0140 | 0.0 | 0.0 | 2.4 | 1.5 |
| TDN0400204-6 | T7 | 02TGH00032 | 218.11.30 | 05-147-0141 | 0.0 | 0.0 | 2.4 | 1.7 |
| TDN0400204-7 | T7 | 02TGH00032 | 218.11.30 | 05-147-0142 | 0.0 | 0.0 | 2.5 | 1.6 |
| TDN0400204-8 | T7 | 02TGH00032 | 218.11.30 | 05-147-0143 | 0.0 | 0.0 | 2.5 | 1.6 |
| TDN0400204-9 | T7 | 02TGH00032 | 218.11.30 | 05-147-0144 | 0.0 | 0.0 | 2.4 | 1.7 |
| TDN0400208-1 | T7 | 02TGH00037 | 36.11.19 | 05-147-0145 | 0.0 | 0.1 | 2.1 | 1.7 |
| TDN0400208-2 | T7 | 02TGH00037 | 36.11.19 | 05-147-0146 | 0.0 | 0.1 | 2.1 | 1.9 |
| TDN0400208-3 | T7 | 02TGH00037 | 36.11.19 | 05-147-0147 | 0.0 | 0.1 | 2.2 | 1.8 |
| TDN0400208-4 | T7 | 02TGH00037 | 36.11.19 | 05-147-0148 | 0.0 | 0.1 | 2.2 | 1.8 |
| TDN0400208-5 | T7 | 02TGH00037 | 36.11.19 | 05-147-0149 | 0.0 | 0.0 | 2.5 | 1.6 |
| TDN0400208-6 | T7 | 02TGH00037 | 36.11.19 | 05-147-0150 | 0.0 | 0.0 | 2.4 | 1.5 |
| TDN0400208-7 | T7 | 02TGH00037 | 36.11.19 | 05-147-0151 | 0.0 | 0.0 | 2.2 | 1.3 |
| TDN0400208-8 | T7 | 02TGH00037 | 36.11.19 | 05-147-0152 | 0.0 | 0.1 | 2.1 | 1.8 |
| TDN0400208-9 | T7 | 02TGH00037 | 36.11.19 | 05-147-0153 | 0.0 | 0.0 | 2.1 | 1.8 |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| Nex 710 | 1.5 | 78.5 | 9.9 | 2.9 | 0.6 | 1.4 | 0.1 | 0.4 | 0.0 | 0.3 | 0.2 | 6.4 | 10.1 | G |
| Nex 710 | 1.5 | 77.7 | 10.5 | 3.0 | 0.6 | 1.4 | 0.1 | 0.4 | 0.0 | 0.3 | 0.0 | 6.6 | 9.2 | G |
| Nex 710 | 1.3 | 77.6 | 10.7 | 2.9 | 0.6 | 1.5 | 0.1 | 0.4 | 0.0 | 0.3 | 0.2 | 6.4 | 10.2 | G |
| Nex 710 | 1.5 | 78.8 | 9.2 | 2.7 | 0.7 | 1.6 | 0.1 | 0.5 | 0.0 | 0.4 | 0.2 | 6.7 | 5.7 | G |
| Nex 710 | 1.4 | 78.1 | 10.0 | 2.9 | 0.7 | 1.5 | 0.1 | 0.5 | 0.0 | 0.4 | 0.2 | 6.6 | 10.4 | G |
| Nex 710 | 1.4 | 77.0 | 11.2 | 3.0 | 0.6 | 1.4 | 0.1 | 0.4 | 0.0 | 0.3 | 0.2 | 6.5 | 13.3 | G |
| Nex 710 | 1.6 | 78.2 | 9.8 | 2.6 | 0.7 | 1.5 | 0.1 | 0.5 | 0.0 | 0.4 | 0.3 | 6.9 | 9.5 | G |
| Nex 710 | 1.4 | 78.0 | 10.2 | 2.9 | 0.6 | 1.4 | 0.1 | 0.4 | 0.0 | 0.4 | 0.2 | 6.5 | 11.1 | g |
| Nex 710 | 1.5 | 77.5 | 10.7 | 2.7 | 0.7 | 1.5 | 0.1 | 0.4 | 0.0 | 0.3 | 0.2 | 6.7 | 11.8 | g |
| TDN0400141-1 | 0.5 | 82.9 | 8.0 | 2.3 | 0.2 | 0.8 | 0.0 | 0.1 | 0.0 | 0.3 | 0.0 | 3.4 | 7.3 | g |
| TDN0400141-2 | 0.5 | 81.6 | 7.9 | 2.6 | 0.2 | 0.9 | 0.1 | 0.2 | 0.0 | 0.3 | 0.0 | 3.6 | 6.1 | g |
| TDN0400141-3 | 0.4 | 82.3 | 7.8 | 2.6 | 0.2 | 0.9 | 0.0 | 0.1 | 0.0 | 0.3 | 0.0 | 3.2 | 3.4 | g |
| TDN0400141-4 | 0.5 | 81.0 | 8.4 | 2.9 | 0.3 | 0.9 | 0.0 | 0.1 | 0.0 | 0.4 | 0.0 | 3.5 | 4.3 | g |
| TDN0400141-5 | 0.4 | 82.3 | 7.8 | 2.7 | 0.2 | 0.9 | 0.0 | 0.1 | 0.0 | 0.3 | 0.0 | 3.3 | 5.7 | g |
| TDN0400141-6 | 0.5 | 79.1 | 9.7 | 3.2 | 0.3 | 1.0 | 0.1 | 0.2 | 0.0 | 0.3 | 0.0 | 3.5 | 1.8 | g |
| TDN0400141-7 | 0.5 | 81.9 | 7.4 | 2.6 | 0.3 | 0.9 | 0.0 | 0.1 | 0.0 | 0.4 | 0.0 | 3.5 | 2.2 | g |
| TDN0400141-8 | 0.4 | 82.3 | 7.9 | 2.5 | 0.2 | 0.8 | 0.1 | 0.1 | 0.0 | 0.2 | 0.0 | 3.1 | 5.1 | g |
| TDN0400141-9 | 0.5 | 80.8 | 8.9 | 3.1 | 0.2 | 1.0 | 0.1 | 0.1 | 0.0 | 0.1 | 0.0 | 3.1 | 3.1 | g |
| TDN0400142-1 | 0.5 | 81.9 | 8.1 | 2.4 | 0.3 | 1.0 | 0.1 | 0.1 | 0.0 | 0.3 | 0.0 | 3.3 | 7.3 | g |
| TDN0400142-2 | 0.4 | 81.3 | 8.8 | 2.8 | 0.2 | 0.9 | 0.0 | 0.1 | 0.0 | 0.3 | 0.0 | 3.0 | 7.8 | g |
| TDN0400142-3 | 0.4 | 82.8 | 7.8 | 2.5 | 0.2 | 0.9 | 0.0 | 0.1 | 0.0 | 0.3 | 0.0 | 3.1 | 7.7 | g |
| TDN0400142-4 | 0.4 | 82.1 | 7.7 | 2.8 | 0.2 | 0.9 | 0.1 | 0.1 | 0.0 | 0.2 | 0.0 | 3.0 | 4.9 | g |
| TDN0400142-5 | 0.4 | 80.7 | 9.2 | 2.9 | 0.2 | 0.9 | 0.1 | 0.1 | 0.0 | 0.2 | 0.0 | 3.0 | 5 | g |
| TDN0400142-6 | 0.4 | 81.5 | 9.0 | 2.6 | 0.2 | 0.9 | 0.0 | 0.1 | 0.0 | 0.2 | 0.0 | 3.1 | 9.7 | g |
| TDN0400142-7 | 0.4 | 81.6 | 8.8 | 2.6 | 0.2 | 0.9 | 0.1 | 0.1 | 0.0 | 0.3 | 0.0 | 3.0 | 8.6 | g |
| TDN0400142-8 | 0.4 | 81.2 | 9.0 | 2.8 | 0.2 | 0.9 | 0.1 | 0.0 | 0.0 | 0.3 | 0.0 | 2.9 | 7.7 | g |
| TDN0400142-9 | 0.4 | 81.0 | 9.2 | 2.6 | 0.2 | 0.9 | 0.1 | 0.1 | 0.0 | 0.3 | 0.0 | 3.3 | 11.2 | g |
| TDN0400143-1 | 0.5 | 81.7 | 9.0 | 2.4 | 0.2 | 0.9 | 0.1 | 0.2 | 0.0 | 0.2 | 0.0 | 3.6 | 8.5 | g |
| TDN0400143-2 | 0.6 | 81.2 | 9.6 | 2.6 | 0.3 | 1.0 | 0.1 | 0.1 | 0.0 | 0.2 | 0.0 | 3.6 | 5.7 | g |
| TDN0400143-3 | 0.6 | 81.2 | 9.3 | 2.3 | 0.3 | 0.9 | 0.1 | 0.2 | 0.0 | 0.3 | 0.0 | 3.9 | 10.8 | g |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| TDN0400143-4 | 0.6 | 82.4 | 8.5 | 2.4 | 0.3 | 0.9 | 0.0 | 0.2 | 0.0 | 0.2 | 0.0 | 3.9 | 6.2 | g |
| TDN0400143-5 | 0.5 | 81.9 | 8.9 | 2.4 | 0.3 | 0.9 | 0.1 | 0.2 | 0.0 | 0.2 | 0.0 | 3.5 | 9.6 | g |
| TDN0400143-6 | 0.5 | 81.7 | 9.1 | 2.4 | 0.3 | 0.9 | 0.0 | 0.2 | 0.0 | 0.2 | 0.0 | 3.6 | 10.2 | g |
| TDN0400143-7 | 0.7 | 81.6 | 9.1 | 2.3 | 0.4 | 1.0 | 0.1 | 0.2 | 0.0 | 0.2 | 0.0 | 4.1 | 10.7 | g |
| TDN0400143-8 | 0.5 | 80.1 | 9.9 | 2.9 | 0.2 | 0.9 | 0.1 | 0.1 | 0.0 | 0.2 | 0.0 | 3.8 | 3.6 | g |
| TDN0400143-9 | 0.5 | 82.3 | 8.6 | 2.3 | 0.2 | 0.9 | 0.1 | 0.2 | 0.0 | 0.2 | 0.0 | 3.4 | 8.7 | g |
| TDN0400145-1 | 0.6 | 80.7 | 9.9 | 2.5 | 0.3 | 0.9 | 0.1 | 0.2 | 0.0 | 0.2 | 0.0 | 3.7 | 8.9 | g |
| TDN0400145-2 | 0.5 | 80.4 | 10.3 | 2.6 | 0.3 | 0.9 | 0.1 | 0.2 | 0.0 | 0.0 | 0.0 | 3.7 | 7.3 | g |
| TDN0400145-3 | 0.5 | 81.8 | 8.9 | 2.7 | 0.2 | 0.9 | 0.1 | 0.1 | 0.0 | 0.2 | 0.0 | 3.4 | 8 | g |
| TDN0400145-4 | 0.5 | 81.9 | 8.4 | 2.5 | 0.2 | 0.9 | 0.0 | 0.1 | 0.0 | 0.4 | 0.0 | 3.5 | 8.7 | g |
| TDN0400145-5 | 0.5 | 83.1 | 7.9 | 2.2 | 0.3 | 0.9 | 0.0 | 0.1 | 0.0 | 0.2 | 0.0 | 3.6 | 8.3 | g |
| TDN0400145-6 | 0.4 | 81.8 | 8.6 | 2.7 | 0.2 | 0.9 | 0.1 | 0.1 | 0.0 | 0.3 | 0.0 | 3.3 | 8.9 | g |
| TDN0400145-7 | 0.4 | 82.9 | 7.5 | 2.4 | 0.2 | 0.9 | 0.1 | 0.1 | 0.0 | 0.3 | 0.0 | 3.3 | 7.8 | g |
| TDN0400145-8 | 0.5 | 83.2 | 7.5 | 2.6 | 0.2 | 0.9 | 0.0 | 0.1 | 0.0 | 0.2 | 0.0 | 3.2 | 8.4 | g |
| TDN0400145-9 | 0.4 | 82.4 | 7.8 | 2.7 | 0.2 | 0.8 | 0.1 | 0.1 | 0.0 | 0.3 | 0.0 | 3.1 | 8.3 | g |
| TDN0400155-1 | | | | | | | | | | | | | 6.4 | g |
| TDN0400155-2 | | | | | | | | | | | | | 0 | g |
| TDN0400155-3 | 0.6 | 81.9 | 8.3 | 2.6 | 0.2 | 0.8 | 0.1 | 0.1 | 0.0 | 0.2 | 0.0 | 3.7 | 11.2 | g |
| TDN0400155-4 | 0.5 | 82.5 | 7.8 | 2.7 | 0.2 | 0.8 | 0.0 | 0.1 | 0.0 | 0.3 | 0.0 | 3.3 | 10.2 | g |
| TDN0400155-5 | | | | | | | | | | | | | 0 | g |
| TDN0400155-6 | 0.4 | 81.9 | 8.8 | 2.6 | 0.3 | 0.8 | 0.0 | 0.0 | 0.0 | 0.0 | 0.0 | 3.2 | 7.5 | g |
| TDN0400155-7 | 0.5 | 82.3 | 8.4 | 2.4 | 0.3 | 0.8 | 0.0 | 0.1 | 0.0 | 0.0 | 0.0 | 3.6 | 9 | g |
| TDN0400155-8 | 0.5 | 84.2 | 6.4 | 2.3 | 0.3 | 0.8 | 0.0 | 0.1 | 0.0 | 0.2 | 0.0 | 3.3 | 7.5 | g |
| TDN0400155-9 | 0.5 | 82.5 | 8.0 | 2.6 | 0.3 | 0.8 | 0.0 | 0.1 | 0.0 | 0.0 | 0.0 | 3.2 | 10 | g |
| TDN0400158-1 | 0.4 | 83.4 | 7.4 | 2.5 | 0.3 | 0.8 | 0.0 | 0.1 | 0.0 | 0.0 | 0.0 | 3.0 | 9 | g |
| TDN0400158-2 | 0.5 | 83.2 | 8.0 | 2.4 | 0.3 | 0.9 | 0.0 | 0.0 | 0.0 | 0.0 | 0.0 | 3.5 | 7.2 | g |
| TDN0400158-3 | 0.5 | 82.5 | 8.0 | 2.7 | 0.3 | 0.8 | 0.0 | 0.1 | 0.0 | 0.0 | 0.0 | 3.1 | 9.7 | g |
| TDN0400158-4 | 0.5 | 83.3 | 7.5 | 2.5 | 0.3 | 0.8 | 0.0 | 0.1 | 0.0 | 0.1 | 0.0 | 3.2 | 8.6 | g |
| TDN0400158-5 | | | | | | | | | | | | | 0 | g |
| TDN0400158-6 | 0.4 | 82.8 | 7.2 | 2.8 | 0.2 | 0.8 | 0.0 | 0.1 | 0.0 | 0.0 | 0.0 | 2.8 | 6.4 | g |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| TDN0400158-7 | | | | | | | | | | | | | 0 | g |
| TDN0400158-8 | | | | | | | | | | | | | 0 | g |
| TDN0400158-9 | 0.4 | 82.0 | 7.9 | 2.8 | 0.2 | 0.8 | 0.0 | 0.1 | 0.0 | 0.0 | 0.0 | 2.8 | 3.2 | g |
| TDN0400160-1 | 0.4 | 82.4 | 8.1 | 2.8 | 0.3 | 0.8 | 0.0 | 0.1 | 0.0 | 0.1 | 0.0 | 3.1 | 7.8 | g |
| TDN0400160-2 | 0.4 | 82.9 | 7.9 | 2.6 | 0.3 | 0.8 | 0.0 | 0.1 | 0.0 | 0.0 | 0.0 | 3.0 | 8.5 | g |
| TDN0400160-3 | 0.4 | 83.3 | 7.4 | 2.6 | 0.3 | 0.8 | 0.0 | 0.1 | 0.0 | 0.0 | 0.0 | 3.0 | 6.5 | g |
| TDN0400160-4 | 0.6 | 81.8 | 8.9 | 2.6 | 0.3 | 0.8 | 0.0 | 0.1 | 0.0 | 0.0 | 0.0 | 3.5 | 12 | g |
| TDN0400160-5 | 0.4 | 82.3 | 7.7 | 2.7 | 0.3 | 0.8 | 0.0 | 0.1 | 0.0 | 0.1 | 0.0 | 3.1 | 4 | g |
| TDN0400160-6 | 0.4 | 82.5 | 8.1 | 2.6 | 0.3 | 0.8 | 0.0 | 0.1 | 0.0 | 0.1 | 0.0 | 3.1 | 9.2 | g |
| TDN0400160-7 | 0.4 | 83.0 | 7.3 | 2.6 | 0.3 | 0.8 | 0.0 | 0.0 | 0.0 | 0.1 | 0.0 | 3.0 | 6.5 | g |
| TDN0400160-8 | 0.4 | 82.6 | 8.0 | 2.8 | 0.1 | 0.8 | 0.0 | 0.0 | 0.0 | 0.0 | 0.0 | 2.6 | 2.3 | g |
| TDN0400160-9 | | | | | | | | | | | | | 0 | g |
| TDN0400167-1 | 0.6 | 83.2 | 7.9 | 2.3 | 0.3 | 0.8 | 0.0 | 0.1 | 0.0 | 0.0 | 0.0 | 3.5 | 11.1 | g |
| TDN0400167-2 | 0.5 | 82.7 | 8.1 | 2.4 | 0.3 | 0.8 | 0.0 | 0.1 | 0.0 | 0.1 | 0.0 | 3.4 | 9.6 | g |
| TDN0400167-3 | 0.6 | 82.4 | 8.5 | 2.4 | 0.3 | 0.8 | 0.0 | 0.1 | 0.0 | 0.0 | 0.0 | 3.5 | 10.1 | g |
| TDN0400167-4 | 0.5 | 82.0 | 8.9 | 2.5 | 0.3 | 0.8 | 0.0 | 0.1 | 0.0 | 0.0 | 0.0 | 3.4 | 9.4 | g |
| TDN0400167-5 | 0.6 | 81.8 | 8.9 | 2.4 | 0.3 | 0.8 | 0.0 | 0.1 | 0.0 | 0.1 | 0.0 | 3.7 | 11.9 | g |
| TDN0400167-6 | 0.6 | 82.3 | 8.7 | 2.4 | 0.3 | 0.8 | 0.0 | 0.1 | 0.0 | 0.0 | 0.0 | 3.5 | 10.3 | g |
| TDN0400167-7 | 0.5 | 81.8 | 8.7 | 2.6 | 0.3 | 0.9 | 0.0 | 0.1 | 0.0 | 0.0 | 0.0 | 3.3 | 8.5 | g |
| TDN0400167-8 | 0.6 | 82.1 | 8.9 | 2.5 | 0.3 | 0.8 | 0.0 | 0.1 | 0.0 | 0.0 | 0.0 | 3.5 | 9.2 | g |
| TDN0400167-9 | 0.5 | 82.7 | 8.3 | 2.4 | 0.3 | 0.8 | 0.0 | 0.1 | 0.0 | 0.0 | 0.0 | 3.5 | 8.7 | g |
| TDN0400184-1 | 0.5 | 82.9 | 8.1 | 2.5 | 0.3 | 0.8 | 0.0 | 0.1 | 0.0 | 0.0 | 0.0 | 3.1 | 10.2 | g |
| TDN0400184-2 | 0.5 | 83.1 | 7.9 | 2.4 | 0.3 | 0.8 | 0.0 | 0.0 | 0.0 | 0.0 | 0.0 | 3.3 | 8.7 | g |
| TDN0400184-3 | 0.5 | 83.5 | 7.3 | 2.3 | 0.3 | 0.8 | 0.0 | 0.1 | 0.0 | 0.0 | 0.0 | 3.1 | 8.6 | g |
| TDN0400184-4 | 0.5 | 83.7 | 7.1 | 2.2 | 0.3 | 0.8 | 0.0 | 0.1 | 0.0 | 0.1 | 0.0 | 3.1 | 10.3 | g |
| TDN0400184-5 | 0.5 | 83.3 | 7.1 | 2.3 | 0.3 | 0.8 | 0.0 | 0.1 | 0.0 | 0.1 | 0.0 | 3.3 | 6.9 | g |
| TDN0400184-6 | 0.5 | 82.4 | 8.1 | 2.6 | 0.3 | 0.8 | 0.0 | 0.1 | 0.0 | 0.0 | 0.0 | 3.2 | 10.5 | g |
| TDN0400184-7 | 0.5 | 84.0 | 6.9 | 2.1 | 0.3 | 0.8 | 0.0 | 0.1 | 0.0 | 0.1 | 0.0 | 3.3 | 9.6 | g |
| TDN0400184-8 | 0.5 | 82.2 | 8.2 | 2.6 | 0.1 | 0.8 | 0.0 | 0.1 | 0.0 | 0.1 | 0.0 | 3.0 | 10.1 | g |
| TDN0400184-9 | 0.5 | 82.0 | 8.5 | 2.4 | 0.3 | 0.8 | 0.0 | 0.1 | 0.0 | 0.1 | 0.0 | 3.5 | 8.1 | g |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| TDN0400189-1 | 0.8 | 79.9 | 10.6 | 2.4 | 0.3 | 0.8 | 0.0 | 0.2 | 0.0 | 0.0 | 0.0 | 4.2 | 10 | g |
| TDN0400189-2 | 0.7 | 80.9 | 9.8 | 2.6 | 0.3 | 0.9 | 0.0 | 0.2 | 0.0 | 0.0 | 0.0 | 4.1 | 8.5 | g |
| TDN0400189-3 | 0.7 | 83.4 | 7.8 | 2.1 | 0.3 | 0.9 | 0.0 | 0.1 | 0.0 | 0.0 | 0.0 | 3.7 | 10.7 | g |
| TDN0400189-4 | 0.6 | 79.5 | 11.0 | 2.9 | 0.3 | 1.0 | 0.0 | 0.1 | 0.0 | 0.0 | 0.0 | 3.8 | 8.8 | g |
| TDN0400189-5 | 0.6 | 82.4 | 8.5 | 2.3 | 0.3 | 0.9 | 0.0 | 0.1 | 0.0 | 0.0 | 0.0 | 3.4 | 10.1 | g |
| TDN0400189-6 | 0.6 | 82.2 | 8.5 | 2.5 | 0.3 | 0.8 | 0.0 | 0.1 | 0.0 | 0.0 | 0.0 | 3.5 | 11.6 | g |
| TDN0400189-7 | 0.6 | 81.5 | 9.2 | 2.5 | 0.3 | 0.8 | 0.0 | 0.1 | 0.0 | 0.0 | 0.0 | 3.8 | 10.6 | g |
| TDN0400189-8 | 0.7 | 82.2 | 8.8 | 2.3 | 0.3 | 0.9 | 0.0 | 0.2 | 0.0 | 0.0 | 0.0 | 3.8 | 11.2 | g |
| TDN0400189-9 | 0.8 | 81.3 | 9.2 | 2.4 | 0.4 | 1.0 | 0.1 | 0.0 | 0.0 | 0.3 | 0.0 | 4.2 | 8.7 | g |
| TDN0400197-1 | 0.7 | 79.6 | 10.3 | 2.6 | 0.3 | 0.9 | 0.0 | 0.1 | 0.0 | 0.1 | 0.0 | 4.3 | 9 | g |
| TDN0400197-2 | | 79.1 | 10.5 | 3.0 | 0.3 | 0.9 | 0.0 | 0.0 | 0.0 | 0.1 | 0.0 | 3.5 | 11 | g |
| TDN0400197-3 | 0.5 | 81.5 | 8.8 | 2.7 | 0.3 | 0.9 | 0.0 | 0.1 | 0.0 | 0.3 | 0.0 | 3.8 | 11.6 | g |
| TDN0400197-4 | 0.5 | 81.4 | 8.7 | 2.7 | 0.3 | 0.8 | 0.0 | 0.0 | 0.0 | 0.3 | 0.0 | 3.7 | 12.3 | g |
| TDN0400197-5 | 0.5 | 79.3 | 10.6 | 3.0 | 0.3 | 0.9 | 0.1 | 0.1 | 0.0 | 0.2 | 0.0 | 3.8 | 10.6 | g |
| TDN0400197-6 | 0.5 | 79.8 | 10.2 | 2.6 | 0.4 | 1.0 | 0.0 | 0.0 | 0.0 | 0.4 | 0.0 | 4.4 | 12.3 | g |
| TDN0400197-7 | 0.7 | 77.2 | 11.3 | 3.3 | 0.3 | 0.9 | 0.0 | 0.0 | 0.0 | 0.3 | 0.0 | 4.0 | 6.9 | g |
| TDN0400197-8 | 0.5 | 80.4 | 9.9 | 2.6 | 0.3 | 1.1 | 0.1 | 0.0 | 0.0 | 0.3 | 0.0 | 3.9 | 9.7 | g |
| TDN0400197-9 | 0.7 | 80.2 | 9.8 | 3.1 | 0.3 | 0.8 | 0.0 | 0.0 | 0.0 | 0.2 | 0.0 | 3.8 | 11.1 | g |
| TDN0400198-1 | 0.5 | 77.1 | 11.1 | 3.3 | 0.6 | 1.5 | 0.0 | 0.5 | 0.0 | 0.0 | 0.0 | 6.3 | 11.2 | g |
| TDN0400198-2 | 1.4 | 77.4 | 11.3 | 3.0 | 0.6 | 1.6 | 0.1 | 0.1 | 0.0 | 0.0 | 0.0 | 5.9 | 11.6 | g |
| TDN0400198-3 | 1.4 | 77.5 | 11.0 | 3.0 | 0.6 | 1.6 | 0.0 | 0.3 | 0.0 | 0.1 | 0.0 | 6.0 | 11.1 | g |
| TDN0400198-4 | 1.3 | 78.0 | 10.2 | 3.1 | 0.6 | 1.5 | 0.0 | 0.3 | 0.0 | 0.6 | 0.0 | 6.5 | 12.8 | g |
| TDN0400198-5 | 1.4 | 77.8 | 10.5 | 3.0 | 0.5 | 1.5 | 0.0 | 0.3 | 0.0 | 0.5 | 0.0 | 6.4 | 12.5 | g |
| TDN0400198-6 | 1.3 | 77.3 | 11.1 | 3.1 | 0.6 | 1.5 | 0.0 | 0.4 | 0.0 | 0.2 | 0.0 | 6.3 | 6.7 | g |
| TDN0400198-7 | 1.3 | 78.1 | 10.3 | 3.0 | 0.6 | 1.5 | 0.1 | 0.4 | 0.0 | 0.0 | 0.0 | 6.2 | 11.1 | g |
| TDN0400198-8 | 1.4 | 78.5 | 10.1 | 2.9 | 0.6 | 1.6 | 0.0 | 0.2 | 0.0 | 0.0 | 0.0 | 6.0 | 13.8 | g |
| TDN0400198-9 | 1.5 | 76.1 | 12.1 | 3.4 | 0.5 | 1.7 | 0.1 | 0.5 | 0.0 | 0.0 | 0.0 | 5.8 | 7.6 | g |
| TDN0400199-1 | 1.2 | 77.6 | 10.7 | 3.1 | 0.6 | 1.5 | 0.0 | 0.1 | 0.0 | 0.1 | 0.0 | 6.0 | 11.3 | g |
| TDN0400199-2 | 1.5 | 78.3 | 10.3 | 2.9 | 0.6 | 1.6 | 0.0 | 0.0 | 0.0 | 0.1 | 0.0 | 5.9 | 11.9 | g |
| TDN0400199-3 | 1.4 | 77.5 | 11.0 | 3.0 | 0.6 | 1.6 | 0.0 | 0.2 | 0.0 | 0.0 | 0.0 | 5.9 | 11.2 | g |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| TDN0400199-4 | 1.4 | 79.3 | 9.8 | 2.7 | 0.6 | 1.6 | 0.0 | 0.3 | 0.0 | 0.0 | 0.0 | 5.9 | 8.4 | g |
| TDN0400199-5 | 1.4 | 78.9 | 10.1 | 2.7 | 0.6 | 1.7 | 0.0 | 0.0 | 0.0 | 0.0 | 0.0 | 5.9 | 10 | g |
| TDN0400199-6 | 1.5 | 78.4 | 10.2 | 2.7 | 0.7 | 1.6 | 0.0 | 0.3 | 0.0 | 0.0 | 0.0 | 6.4 | 8.2 | g |
| TDN0400199-7 | 1.5 | 77.5 | 10.5 | 3.0 | 0.6 | 1.5 | 0.1 | 0.4 | 0.0 | 0.1 | 0.0 | 6.2 | 12.4 | g |
| TDN0400199-8 | 1.4 | 78.8 | 10.0 | 2.7 | 0.6 | 1.5 | 0.1 | 0.2 | 0.0 | 0.1 | 0.0 | 6.1 | 8.6 | g |
| TDN0400199-9 | 1.5 | 78.3 | 10.1 | 2.8 | 0.7 | 1.6 | 0.1 | 0.1 | 0.0 | 0.1 | 0.0 | 5.9 | 10.3 | g |
| TDN0400202-1 | 1.4 | 81.9 | 8.3 | 2.7 | 0.3 | 0.9 | 0.0 | 0.1 | 0.0 | 0.2 | 0.0 | 3.6 | 14.3 | g |
| TDN0400202-2 | 0.5 | 83.3 | 7.8 | 2.1 | 0.3 | 0.9 | 0.0 | 0.0 | 0.0 | 0.0 | 0.0 | 3.5 | 10.3 | g |
| TDN0400202-3 | 0.6 | 82.3 | 8.6 | 2.6 | 0.2 | 0.8 | 0.0 | 0.0 | 0.0 | 0.0 | 0.0 | 3.3 | 9.5 | g |
| TDN0400202-4 | 0.5 | 81.2 | 9.2 | 2.8 | 0.3 | 0.9 | 0.0 | 0.0 | 0.0 | 0.2 | 0.0 | 3.6 | 8.1 | g |
| TDN0400202-5 | 0.5 | 82.9 | 7.8 | 2.5 | 0.3 | 0.9 | 0.0 | 0.0 | 0.0 | 0.0 | 0.0 | 3.1 | 6.5 | g |
| TDN0400202-6 | 0.4 | 82.4 | 8.4 | 2.6 | 0.3 | 0.9 | 0.1 | 0.1 | 0.0 | 0.0 | 0.0 | 3.5 | 11.8 | g |
| TDN0400202-7 | 0.5 | 82.5 | 8.4 | 2.6 | 0.2 | 0.9 | 0.0 | 0.0 | 0.0 | 0.1 | 0.0 | 3.4 | 8.2 | g |
| TDN0400202-8 | 0.5 | 82.0 | 8.7 | 2.7 | 0.3 | 0.9 | 0.0 | 0.0 | 0.0 | 0.0 | 0.0 | 3.3 | 8.2 | g |
| TDN0400202-9 | 0.5 | 82.5 | 8.3 | 2.5 | 0.3 | 0.9 | 0.0 | 0.0 | 0.0 | 0.2 | 0.0 | 3.5 | 10.6 | g |
| TDN0400204-1 | 0.5 | 82.8 | 7.8 | 2.5 | 0.3 | 0.9 | 0.1 | 0.0 | 0.0 | 0.1 | 0.0 | 3.4 | 10.4 | g |
| TDN0400204-2 | 0.5 | 82.4 | 8.0 | 2.5 | 0.3 | 0.9 | 0.0 | 0.1 | 0.0 | 0.1 | 0.0 | 3.5 | 11.6 | g |
| TDN0400204-3 | 0.6 | 82.1 | 8.5 | 2.6 | 0.3 | 0.8 | 0.0 | 0.0 | 0.0 | 0.1 | 0.0 | 3.3 | 11.3 | g |
| TDN0400204-4 | 0.5 | 81.8 | 8.9 | 2.6 | 0.3 | 0.8 | 0.0 | 0.0 | 0.0 | 0.0 | 0.0 | 3.2 | 10.1 | g |
| TDN0400204-5 | 0.5 | 82.8 | 8.1 | 2.5 | 0.2 | 0.8 | 0.0 | 0.0 | 0.0 | 0.0 | 0.0 | 3.2 | 11 | g |
| TDN0400204-6 | 0.5 | 82.8 | 7.8 | 2.6 | 0.3 | 0.8 | 0.0 | 0.0 | 0.0 | 0.1 | 0.0 | 3.4 | 9.4 | g |
| TDN0400204-7 | 0.6 | 82.1 | 8.7 | 2.5 | 0.3 | 0.8 | 0.0 | 0.0 | 0.0 | 0.0 | 0.0 | 3.4 | 10.3 | g |
| TDN0400204-8 | 0.5 | 82.4 | 8.6 | 2.4 | 0.2 | 0.8 | 0.1 | 0.0 | 0.0 | 0.1 | 0.0 | 3.3 | 7.7 | g |
| TDN0400204-9 | 0.5 | 83.1 | 7.7 | 2.4 | 0.3 | 0.8 | 0.0 | 0.0 | 0.1 | 0.0 | 0.0 | 3.4 | 10.5 | g |
| TDN0400208-1 | 0.6 | 82.2 | 8.7 | 2.2 | 0.4 | 1.0 | 0.0 | 0.0 | 0.0 | 0.0 | 0.0 | 3.2 | 10.3 | g |
| TDN0400208-2 | 0.7 | 80.9 | 9.5 | 2.5 | 0.3 | 1.0 | 0.0 | 0.1 | 0.0 | 0.1 | 0.0 | 3.3 | 8 | g |
| TDN0400208-3 | 0.6 | 82.4 | 8.3 | 2.2 | 0.4 | 1.0 | 0.1 | 0.0 | 0.0 | 0.0 | 0.0 | 3.3 | 8.6 | g |
| TDN0400208-4 | 0.7 | 82.6 | 8.3 | 2.3 | 0.3 | 1.0 | 0.1 | 0.0 | 0.0 | 0.0 | 0.0 | 3.2 | 8.3 | g |
| TDN0400208-5 | 0.6 | 81.1 | 9.5 | 2.4 | 0.3 | 0.9 | 0.0 | 0.0 | 0.0 | 0.1 | 0.0 | 3.6 | 12.6 | g |
| TDN0400208-6 | 0.6 | 80.8 | 10.0 | 2.6 | 0.3 | 0.9 | 0.1 | 0.1 | 0.0 | 0.0 | 0.0 | 3.4 | 10.5 | g |

**Table 29 (continued).**

| Name | C18:0 | C18:1 | C18:2 | C18:3 | C20:0 | C20:1 | C20:2 | C22:0 | C22:1 | C24:0 | C24:1 | % Sats | Seed Weight | Seed Weight Units |
|---|---|---|---|---|---|---|---|---|---|---|---|---|---|---|
| TDN0400208-7 | 0.6 | 81.6 | 9.6 | 2.2 | 0.3 | 1.0 | 0.0 | 0.1 | 0.0 | 0.0 | 0.0 | 3.4 | 10.1 | g |
| TDN0400208-8 | 0.7 | 82.4 | 8.5 | 2.3 | 0.3 | 1.0 | 0.0 | 0.1 | 0.0 | 0.0 | 0.0 | 3.3 | 8.4 | g |
| TDN0400208-9 | 0.7 | 81.8 | 8.6 | 2.4 | 0.4 | 1.0 | 0.1 | 0.0 | 0.0 | 0.0 | 0.0 | 3.2 | 8.2 | g |

1. A canola plant that produces seed having an oil fraction comprising less than 3.5 % total saturates and less than 80% oleic acid.

2. The plant of item 1 wherein said oil fraction comprises 70% to 78% oleic acid.

3. The plant of item 1 wherein said oil fraction comprises no more than 3% linolenic acid.

4. The plant of item 1 wherein said oil fraction comprises 70% to 78% oleic acid and no more than 3.5% linolenic acid.

5. Seed produced by the canola plant of item 1.

6. Canola oil comprising less than 3.5 % total saturates and less than 80% oleic acid.

7. A fried food composition comprising potato material and canola oil according to item 6.

8. The canola plant of item 1 wherein said oil fraction has no more than 2.7% total saturates.

9. The canola seed of item 5 having an oil fraction comprising no more than 2.7% total saturates.

10. The canola oil of item 6 having an oil fraction comprising no more than 2.7% total saturates.

11. A method of producing a fried food composition wherein said method comprises frying potato material in canola oil according to item 6.

12. A canola plant comprising at least one polynucleotide, stably incorporated in a genome of said plant, that encodes a delta-9 desaturase protein wherein the full complement of a nucleic acid molecule that encodes a protein of SEQ ID NO:5 maintains hybridization, after wash, with said polynucleotide, wherein said wash conditions are 2x SSC (Standard Saline Citrate) and 0.1% SDS (Sodium Dodecyl Sulfate) for 15 minutes at room temperature.

13. The plant of item 12 wherein said nucleic acid molecule comprises SEQ ID NO:1.

14. The plant of item 12 wherein said wash conditions are 0.1x SSC and 0.1% SDS for 15 minutes at room temperature.

15. The plant of item 12 wherein said wash conditions are 0.1x SSC and 0.1% SDS for 30 minutes at 55° C.

16. The plant of item 12 wherein said genome comprises two of said polynucleotides.

17. The plant of item 12 wherein said genome comprises three of said polynucleotides.

18. The plant of item 12 wherein said polynucleotide is operably linked to a seed-specific promoter.

19. A plant produced by the seed of item 5.

20. A method of reducing saturated fat in the oil fraction of at least one seed of a transgenic canola plant, as compared to a wild-type oil fraction of seeds of a corresponding wild-type canola plant, wherein said method comprises producing a canola plant that expresses a polynucleotide that encodes a delta-9 desaturase protein wherein a nucleotide molecule that encodes said protein hybridizes with the molecule of SEQ ID NO:1.

21. The method of item 20 wherein the oil fraction comprises less than 80% oleic acid.

22. The method of item 20 wherein said protein causes a reduction in palmitic acid (16:0), a reduction in behenic acid (22:0), and an increase in palmitoleic acid (16:1) in the oil fraction of said seed of said transgenic canola plant relative to said corresponding wild-type canola plant.

23. The method of item 20 wherein said saturated fat is reduced by at least 60%.

24. A polynucleotide comprising a sequence of nucleotides shown in SEQ ID NO:1.

25. The plant of item 1 wherein said plant is at least 100cm in height with an average seed weight above 3 mg.

## Claims

**1.** A Canola oil comprising less than 3.5% total saturated fatty acids and less than 80% oleic acid, wherein the oil is obtained from seeds of a canola plant by crushing the seeds, wherein said total saturated fatty acids comprises

a) from 0.26% to 0.51% C18:0, and/or
b) from 0.11% to 0.28% C20:0, and/or
c) from 0.03% to 0.13% C22:0.

**2.** The canola oil according to claim 1 having an oil fraction comprising no more than 2.7% total saturated fatty acids, wherein the percentage of the total saturated fatty acids is determined by extracting the oil to obtain fatty acid methyl esters and analyzing the fatty acid methyl esters for saturated fatty acid content.

**3.** The canola oil according to any one of claims 1 or 2 wherein said oil comprises 70% to 78% oleic acid.

**4.** The canola oil according to any one of claims 1-3 wherein said oil comprises no more than 3% linolenic acid.

**5.** The canola oil according to any one of claims 1-3 wherein said oil comprises 70% to 78% oleic acid and no more than 3.5% linolenic acid.

**6.** The canola oil according to any one of the claims 1-5 wherein said total saturated fatty acids comprises

a) from 0.26% to 0.51% C18:0 and from 0.11% to 0.28% C20:0, or
b) from 0.26% to 0.51% C18:0 and from 0.03% to 0.13% C22:0, or
c) from 0.11% to 0.28% C20:0 and from 0.03% to 0.13% C22:0, or
d) from 0.26% to 0.51% C18:0 and from 0.11% to 0.28% C20:0 and from 0.03% to 0.13% C22:0.

**7.** A fried food composition comprising potato material and canola oil according to any one of the claims 1-6.

**8.** A method of producing a fried food composition wherein said method comprises frying potato material in canola oil according to any one of the claims 1-6.

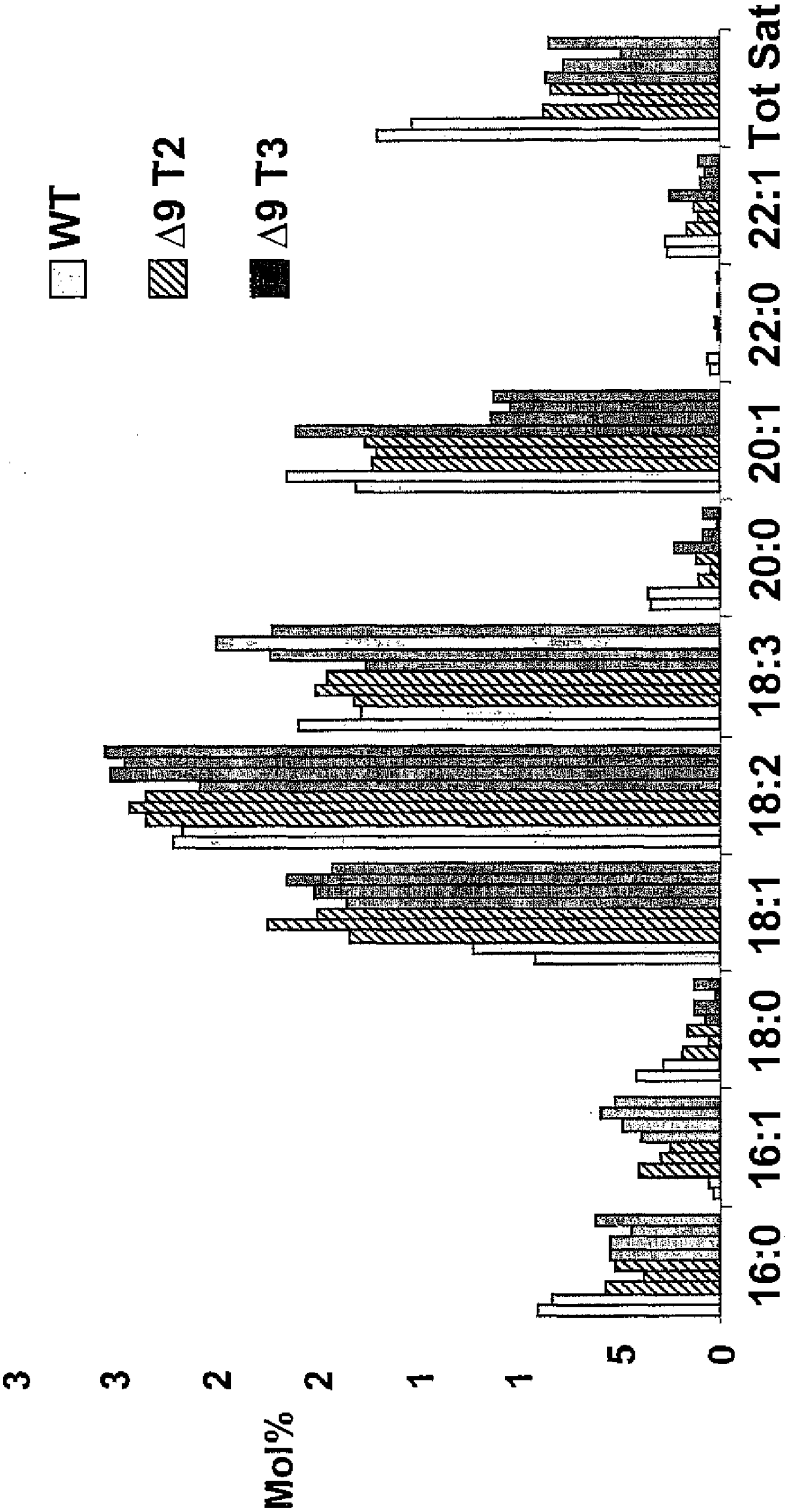


> 60% reduction of saturated fatty acids.

→ In best case more 16:1 (5.9%) than 16:0 (4.4%).

FIG. 1

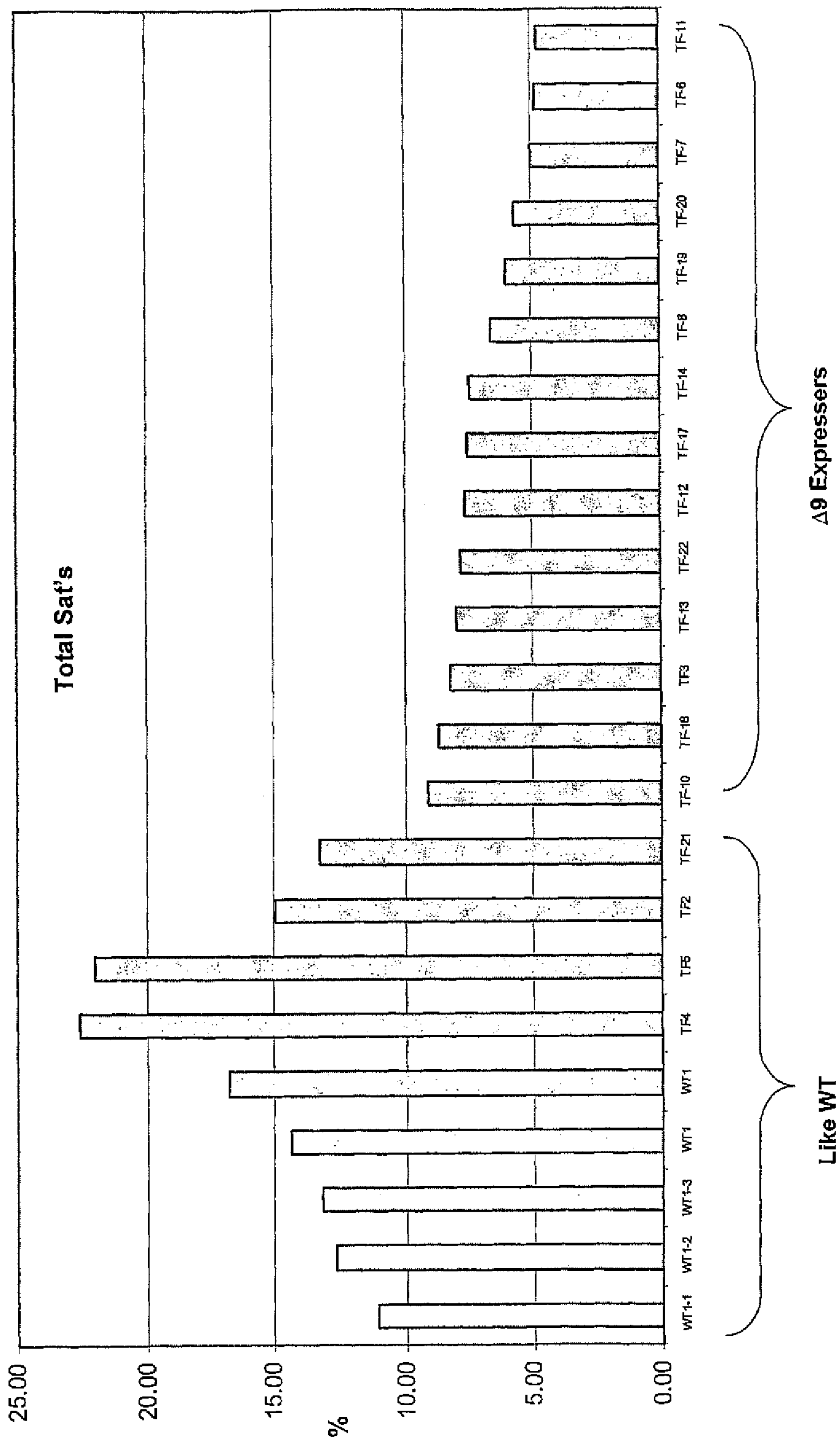
Total Sat's
25.00
20.00
15.00
10.00
5.00
0.00
%
WT1-1
WT1-2
WT1-3
WT1
WT1
TF4
TF5
TF2
TF-21
TF-10
TF-18
TF3
TF-13
TF-22
TF-12
TF-17
TF-14
TF-8
TF-19
TF-20
TF-7
TF-6
TF-11
Like WT
Δ9 Expressers

FIG. 2

- Three transgenic plants from one callus, all Southern positive, Southern pattern indicates one event.
- FAME analysis on single seeds of T1 generation (⇒ T2 in Arabidopsis).

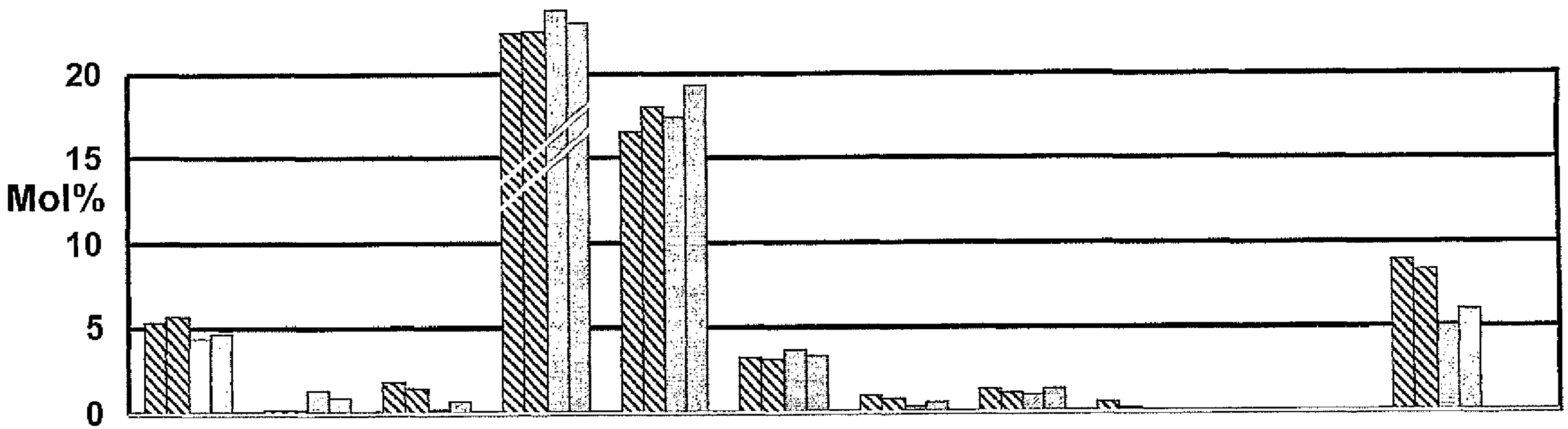


| | 16:0 | 16:1 | 18:0 | 18:1 | 18:2 | 18:3 | 20:0 | 20:1 | 22:0 | 22:1 | Tot Sat |
|---|---|---|---|---|---|---|---|---|---|---|---|
| □ WT | 5.43 | 0.22 | 1.87 | 66.94 | 16.47 | 3.26 | 0.99 | 1.38 | 0.64 | 0.08 | 9.0 |
| □ WT | 5.76 | 0.20 | 1.49 | 67.29 | 17.99 | 3.21 | 0.84 | 1.16 | 0.27 | 0.05 | 8.4 |
| □ #10 | 4.43 | **1.43** | **0.20** | 70.90 | 17.47 | 3.71 | 0.33 | 1.02 | 0.11 | 0.14 | **5.0** |
| □ #11 | 4.72 | **0.95** | **0.66** | 68.63 | 19.26 | 3.42 | 0.56 | 1.39 | 0.00 | 0.14 | **5.9** |

**In best case 43% reduction in Sats (50% when 24:0 included)**

FIG. 3

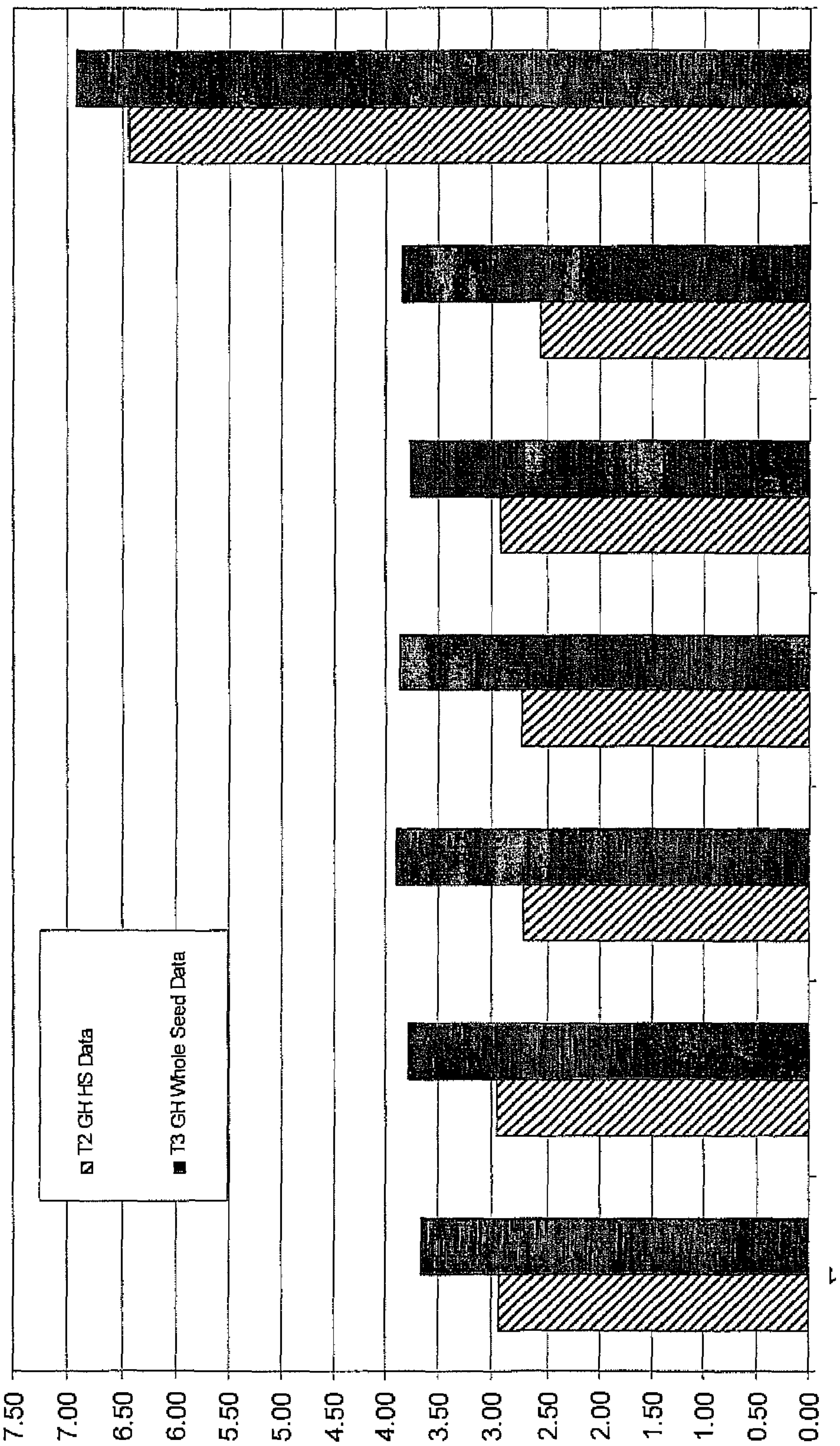
T2 GH HS Data
T3 GH Whole Seed Data
7.50
7.00
6.50
6.00
5.50
5.00
4.50
4.00
3.50
3.00
2.50
2.00
1.50
1.00
0.50
0.00

FIG. 4A

**36-11.19 T3 Fatty Acid Data**

| C14:0 | C16:0 | C16:1 | C18:0 | C18:1 | C18:2 | C18:3 | C20:0 | C20:1 | C20:2 | C22:0 | C22:1 | C24:0 | C24:1 | TOTSAT |
|---|---|---|---|---|---|---|---|---|---|---|---|---|---|---|
| 0.05 | 2.52 | 1.83 | 0.59 | 80.27 | 9.71 | 2.51 | 0.32 | 0.90 | 0.04 | 0.15 | nd | 0.04 | 0.27 | 3.66 |
| 0.05 | 2.56 | 1.86 | 0.61 | 79.73 | 10.31 | 2.43 | 0.34 | 0.92 | 0.06 | 0.16 | 0.05 | 0.07 | 0.07 | 3.78 |
| 0.06 | 2.72 | 1.93 | 0.58 | 77.72 | 12.07 | 2.61 | 0.33 | 0.90 | 0.05 | 0.15 | nd | 0.06 | 0.10 | 3.89 |
| 0.05 | 2.66 | 2.00 | 0.56 | 77.65 | 11.80 | 2.61 | 0.33 | 0.91 | 0.04 | 0.16 | nd | 0.11 | 0.20 | 3.87 |
| 0.05 | 2.65 | 1.79 | 0.56 | 78.27 | 11.67 | 2.68 | 0.30 | 0.81 | 0.04 | 0.14 | nd | 0.07 | 0.23 | 3.76 |
| 0.06 | 2.56 | 1.85 | 0.63 | 78.48 | 11.42 | 2.48 | 0.35 | 0.94 | 0.05 | 0.16 | nd | 0.09 | 0.17 | 3.85 |
| 0.06 | 4.16 | 0.34 | 1.45 | 73.03 | 14.87 | 2.72 | 0.64 | 1.39 | 0.07 | 0.42 | nd | 0.21 | 0.18 | 6.93 |

FIG. 4B

**36-11.19 T2 Fatty Acid Data**

| C16:0 | C16:1 | C18:0 | C18:1 | C18:2 | C18:3 | TOTSAT |
|---|---|---|---|---|---|---|
| 2.16 | 1.68 | 0.36 | 79.63 | 11.99 | 2.82 | 2.94 |
| 2.16 | 1.69 | 0.40 | 78.95 | 12.72 | 2.73 | 2.95 |
| 1.98 | 1.85 | 0.36 | 82.20 | 9.73 | 2.58 | 2.72 |
| 1.95 | 1.80 | 0.34 | 79.21 | 12.08 | 3.17 | 2.72 |
| 2.15 | 1.74 | 0.36 | 80.66 | 11.35 | 2.45 | 2.92 |
| 1.96 | 1.80 | 0.28 | 77.36 | 13.73 | 3.62 | 2.57 |
| nd | 0.06 | 4.13 | 68.82 | 18.42 | 4.57 | 6.44 |

FIG. 4C

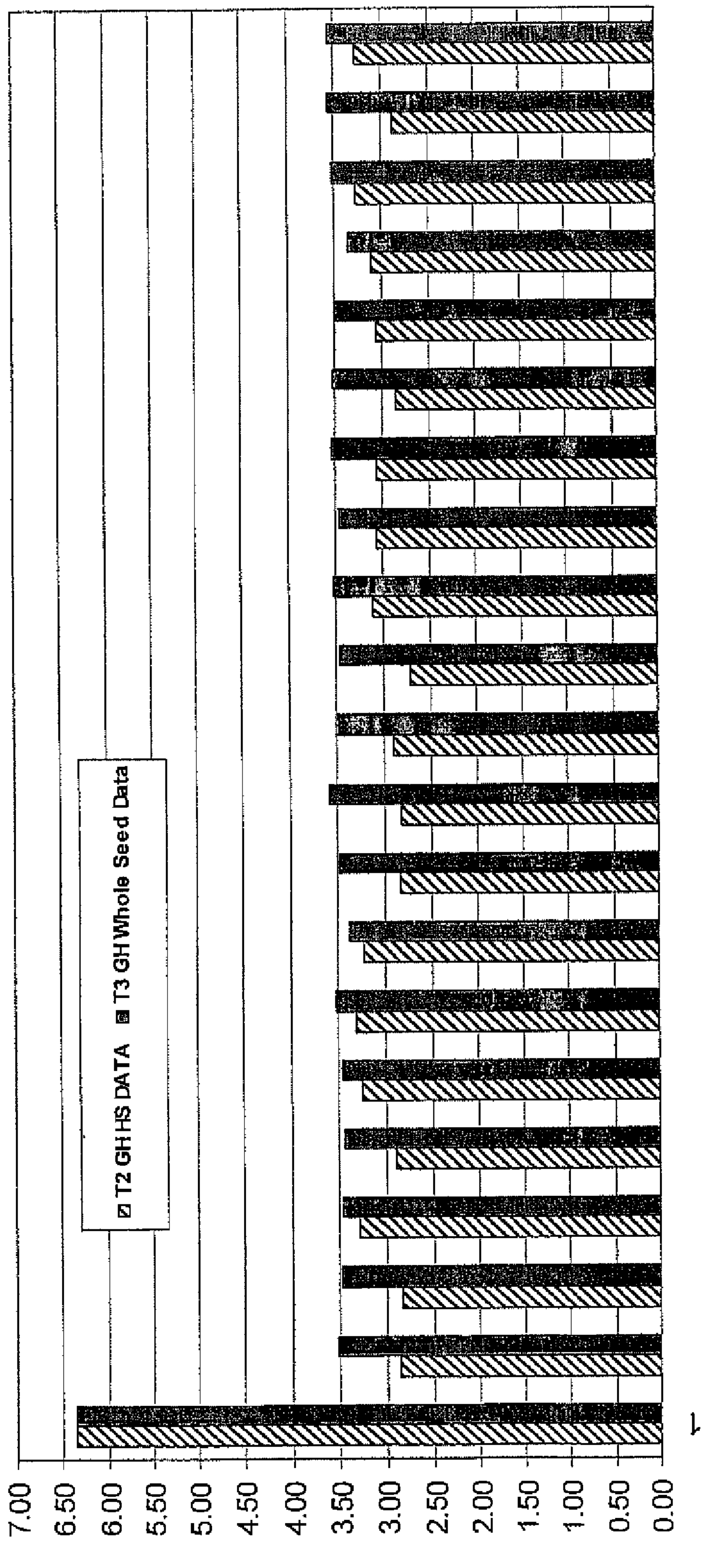
T2 GH HS DATA
T3 GH Whole Seed Data
7.00
6.50
6.00
5.50
5.00
4.50
4.00
3.50
3.00
2.50
2.00
1.50
1.00
0.50
0.00
1

FIG. 5A

218-11.30 T3 Fatty Acid Data-10 seed Bulk

| Seed Amount | C14:0 | 218-11.30 T3 Fatty Acid Data-10 seed Bulk | C16:1 | C18:0 | C18:1 | C18:2 | C18:3 | C20:0 | C20:1 | C20:2 | C22:0 | C22:1 | C24:0 | C24:1 | TOTSAT |
|---|---|---|---|---|---|---|---|---|---|---|---|---|---|---|---|
| 7.5 | 0.04 | 3.54 | 0.23 | 1.44 | 77.55 | 10.76 | 2.83 | 0.65 | 1.49 | 0.06 | 0.43 | 0.04 | 0.27 | 0.14 | 6.36 |
| 4 | 0.04 | 2.35 | 1.84 | 0.53 | 81.00 | 8.97 | 2.75 | 0.30 | 0.85 | 0.06 | 0.14 | nd | 0.16 | 0.04 | 3.53 |
| 5.5 | 0.06 | 2.37 | 1.81 | 0.50 | 80.85 | 9.29 | 2.75 | 0.31 | 0.82 | 0.04 | 0.11 | nd | 0.14 | 0.04 | 3.49 |
| 7.5 | 0.05 | 2.38 | 1.96 | 0.53 | 82.71 | 7.60 | 2.37 | 0.31 | 0.82 | 0.04 | 0.14 | 0.04 | 0.04 | 0.01 | 3.45 |
| 2.7 | 0.06 | 2.38 | 1.95 | 0.53 | 81.47 | 8.50 | 2.76 | 0.30 | 0.81 | 0.06 | 0.14 | nd | 0.05 | 0.03 | 3.44 |
| 5.1 | 0.05 | 2.36 | 1.79 | 0.50 | 80.35 | 9.67 | 2.88 | 0.30 | 0.81 | 0.06 | 0.12 | nd | 0.12 | 0.05 | 3.45 |
| 5.9 | 0.04 | 2.42 | 1.90 | 0.54 | 81.45 | 8.43 | 2.53 | 0.30 | 0.78 | 0.05 | 0.14 | nd | 0.09 | 0.07 | 3.52 |
| 7.6 | 0.05 | 2.38 | 1.76 | 0.50 | 79.99 | 10.17 | 2.91 | 0.28 | 0.81 | 0.05 | 0.12 | nd | 0.04 | 0.01 | 3.37 |
| 5.6 | 0.06 | 2.34 | 1.96 | 0.51 | 79.85 | 9.85 | 3.02 | 0.30 | 0.81 | 0.05 | 0.12 | nd | 0.16 | 0.04 | 3.49 |
| 5.6 | 0.05 | 2.45 | 1.88 | 0.51 | 79.38 | 10.36 | 3.02 | 0.29 | 0.81 | 0.05 | 0.12 | nd | 0.15 | 0.04 | 3.58 |
| 4.9 | 0.06 | 2.43 | 1.96 | 0.51 | 79.58 | 10.16 | 2.97 | 0.29 | 0.80 | 0.05 | 0.12 | nd | 0.06 | 0.02 | 3.48 |
| 3 | 0.07 | 2.13 | 2.03 | 0.63 | 81.26 | 8.83 | 2.51 | 0.33 | 0.86 | 0.05 | 0.16 | nd | 0.15 | 0.23 | 3.47 |
| 4.7 | 0.06 | 2.41 | 2.07 | 0.51 | 79.58 | 10.17 | 2.92 | 0.27 | 0.78 | 0.04 | 0.12 | nd | 0.13 | 0.24 | 3.52 |
| 6.1 | 0.07 | 2.41 | 2.05 | 0.46 | 79.62 | 10.17 | 2.99 | 0.27 | 0.79 | 0.05 | 0.12 | nd | 0.13 | 0.21 | 3.45 |
| 5 | 0.05 | 2.40 | 2.03 | 0.52 | 81.02 | 9.15 | 2.65 | 0.30 | 0.78 | 0.04 | 0.13 | nd | 0.13 | 0.12 | 3.53 |
| 5 | 0.07 | 2.39 | 2.00 | 0.51 | 80.00 | 9.95 | 2.81 | 0.29 | 0.77 | 0.05 | 0.12 | nd | 0.14 | 0.21 | 3.52 |
| 3.2 | 0.07 | 2.40 | 2.04 | 0.49 | 79.91 | 9.89 | 2.91 | 0.28 | 0.78 | 0.05 | 0.12 | nd | 0.14 | 0.22 | 3.50 |
| 3.4 | 0.06 | 2.24 | 1.97 | 0.50 | 80.99 | 9.10 | 2.73 | 0.30 | 0.86 | 0.05 | 0.14 | nd | 0.14 | 0.19 | 3.36 |
| 5.1 | 0.06 | 2.38 | 2.00 | 0.53 | 80.59 | 9.42 | 2.74 | 0.30 | 0.79 | 0.04 | 0.14 | nd | 0.12 | 0.19 | 3.52 |
| 4.3 | 0.07 | 2.45 | 2.13 | 0.50 | 80.64 | 9.20 | 2.70 | 0.29 | 0.78 | 0.05 | 0.14 | nd | 0.12 | 0.19 | 3.56 |
| 1 | 0.07 | 2.40 | 2.12 | 0.52 | 80.71 | 9.05 | 2.66 | 0.31 | 0.82 | 0.04 | 0.13 | nd | 0.14 | 0.20 | 3.56 |

FIG. 5B

**218-11.30 T2 Fatty Acid Data-HS data**

| C14:0 | C16:0 | C16:1 | C18:0 | C18:1 | C18:2 | C18:3 | C20:0 | C20:1 | C20:2 | C22:0 | C24:0 | C24:1 | TOTSAT |
|---|---|---|---|---|---|---|---|---|---|---|---|---|---|
| 0.04 | 3.92 | 0.18 | 1.32 | 78.50 | 10.76 | 2.77 | 0.62 | 1.27 | 0.06 | 0.32 | 0.14 | 0.10 | 6.36 |
| 0.06 | 2.19 | 1.93 | 0.32 | 81.51 | 10.01 | 2.92 | 0.19 | 0.70 | 0.07 | 0.07 | 0.03 | 0.01 | 2.85 |
| 0.07 | 2.24 | 2.07 | 0.28 | 80.02 | 10.79 | 3.45 | 0.19 | 0.73 | 0.08 | 0.06 | nd | 0.03 | 2.84 |
| 0.05 | 2.44 | 1.80 | 0.44 | 82.08 | 9.32 | 2.57 | 0.23 | 0.82 | 0.05 | 0.10 | 0.04 | 0.05 | 3.30 |
| 0.06 | 2.21 | 1.97 | 0.33 | 83.22 | 8.46 | 2.63 | 0.19 | 0.76 | 0.06 | 0.08 | 0.03 | 0.01 | 2.91 |
| 0.05 | 2.27 | 2.09 | 0.50 | 83.32 | 8.01 | 2.50 | 0.27 | 0.71 | 0.05 | 0.12 | 0.04 | 0.07 | 3.24 |
| 0.05 | 2.43 | 1.93 | 0.47 | 79.71 | 11.02 | 3.24 | 0.24 | 0.69 | 0.04 | 0.08 | 0.04 | 0.06 | 3.31 |
| 0.05 | 2.34 | 1.67 | 0.44 | 80.81 | 10.33 | 3.09 | 0.26 | 0.77 | 0.05 | 0.12 | 0.03 | 0.05 | 3.23 |
| 0.05 | 2.25 | 1.94 | 0.28 | 79.50 | 11.57 | 3.37 | 0.20 | 0.71 | 0.04 | 0.03 | 0.02 | 0.02 | 2.84 |
| 0.05 | 2.24 | 1.91 | 0.26 | 77.77 | 12.80 | 3.94 | 0.21 | 0.71 | 0.05 | 0.04 | 0.02 | 0.01 | 2.82 |
| 0.05 | 2.26 | 1.95 | 0.34 | 78.65 | 12.13 | 3.52 | 0.11 | 0.76 | 0.06 | 0.12 | 0.03 | 0.02 | 2.91 |
| 0.05 | 2.03 | 1.94 | 0.34 | 79.83 | 11.69 | 2.82 | 0.16 | 0.86 | 0.07 | 0.08 | 0.04 | 0.08 | 2.71 |
| 0.05 | 2.29 | 1.77 | 0.38 | 81.32 | 9.85 | 3.01 | 0.23 | 0.77 | 0.04 | 0.09 | 0.08 | 0.13 | 3.10 |
| 0.05 | 2.36 | 1.88 | 0.33 | 81.69 | 9.51 | 2.99 | 0.23 | 0.76 | 0.05 | 0.08 | 0.02 | 0.05 | 3.08 |
| 0.06 | 2.37 | 2.06 | 0.34 | 80.82 | 10.19 | 3.02 | 0.22 | 0.73 | 0.05 | 0.08 | nd | 0.06 | 3.07 |
| 0.06 | 2.16 | 2.01 | 0.36 | 80.30 | 10.81 | 3.14 | 0.14 | 0.73 | 0.06 | 0.09 | 0.06 | 0.07 | 2.86 |
| 0.06 | 2.31 | 2.00 | 0.32 | 78.80 | 12.47 | 2.82 | 0.22 | 0.74 | 0.05 | 0.07 | 0.10 | 0.06 | 3.07 |
| 0.04 | 2.16 | 2.01 | 0.47 | 81.75 | 9.76 | 2.44 | 0.28 | 0.79 | 0.05 | 0.13 | 0.03 | 0.10 | 3.10 |
| 0.05 | 2.40 | 1.64 | 0.44 | 79.87 | 11.47 | 2.95 | 0.22 | 0.72 | 0.05 | 0.13 | 0.03 | 0.02 | 3.26 |
| 0.05 | 2.14 | 2.00 | 0.35 | 79.75 | 11.10 | 3.42 | 0.20 | 0.74 | 0.08 | 0.11 | 0.02 | 0.04 | 2.88 |
| 0.04 | 2.33 | 1.77 | 0.51 | 81.86 | 9.66 | 2.63 | 0.26 | 0.74 | 0.04 | 0.11 | 0.03 | 0.03 | 3.28 |

FIG. 5C

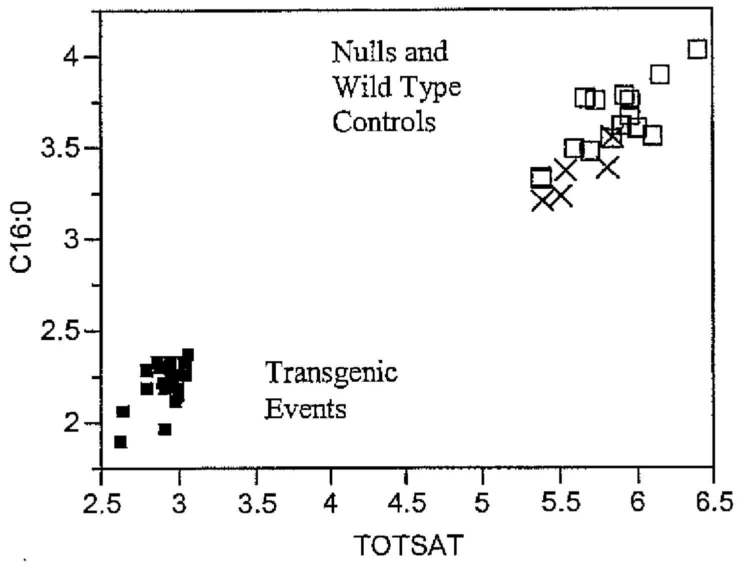
Nulls and
Wild Type
Controls
Transgenic
Events
C16:0
4
3.5
3
2.5
2
2.5
3
3.5
4
4.5
5
5.5
6
6.5
TOTSAT

FIG. 6A

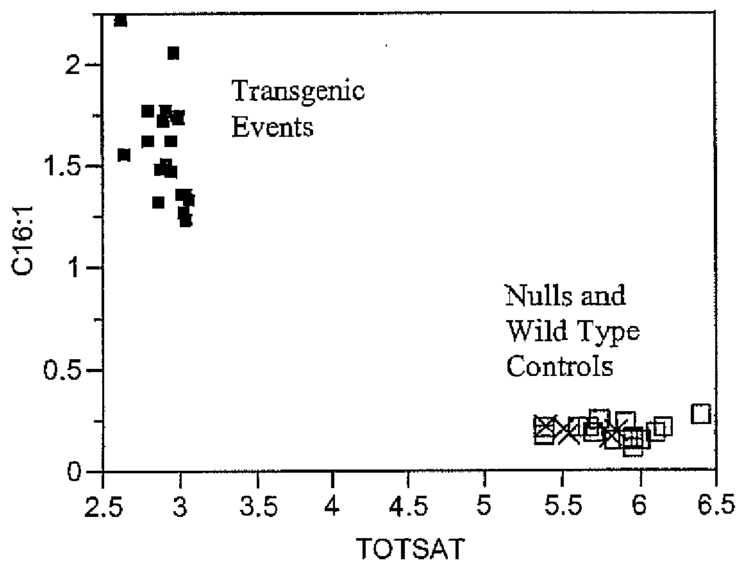
Transgenic
Events
Nulls and
Wild Type
Controls
C16:1
2
1.5
1
0.5
0
2.5
3
3.5
4
4.5
5
5.5
6
6.5
TOTSAT

FIG. 6B

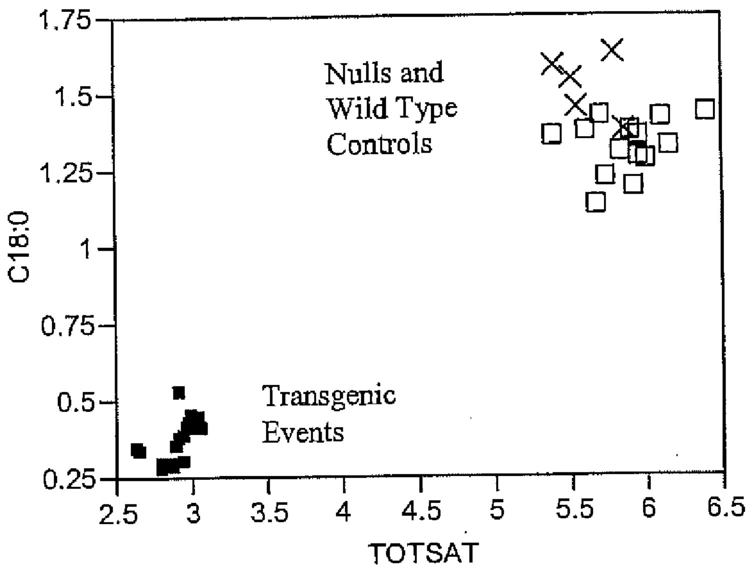
1.75
1.5
1.25
1
0.75
0.5
0.25
C18:0
Nulls and
Wild Type
Controls
Transgenic
Events
2.5
3
3.5
4
4.5
5
5.5
6
6.5
TOTSAT

FIG. 6C

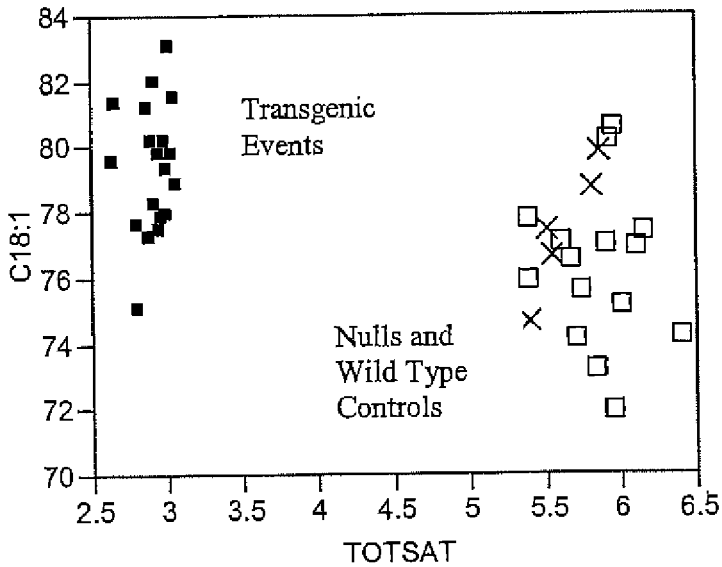
84
82
80
78
76
74
72
70
C18:1
Transgenic
Events
Nulls and
Wild Type
Controls
2.5
3
3.5
4
4.5
5
5.5
6
6.5
TOTSAT

FIG. 6D

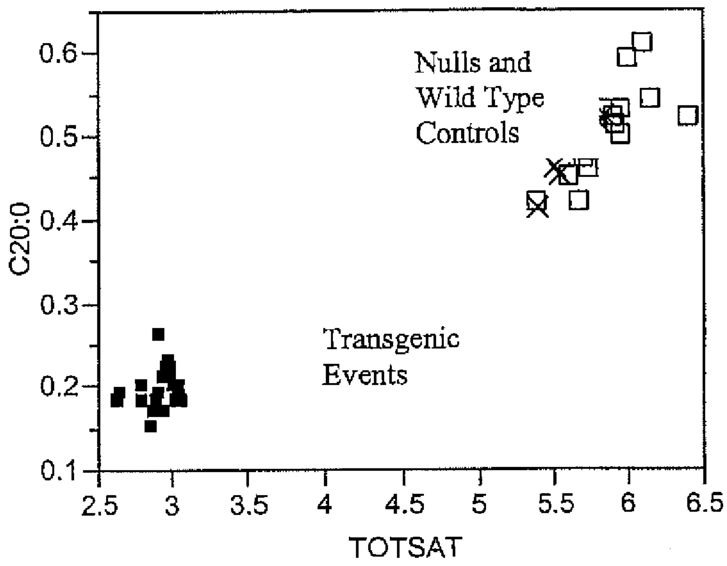
Nulls and
Wild Type
Controls
Transgenic
Events
C20:0
0.6
0.5
0.4
0.3
0.2
0.1
2.5
3
3.5
4
4.5
5
5.5
6
6.5
TOTSAT

FIG. 6E

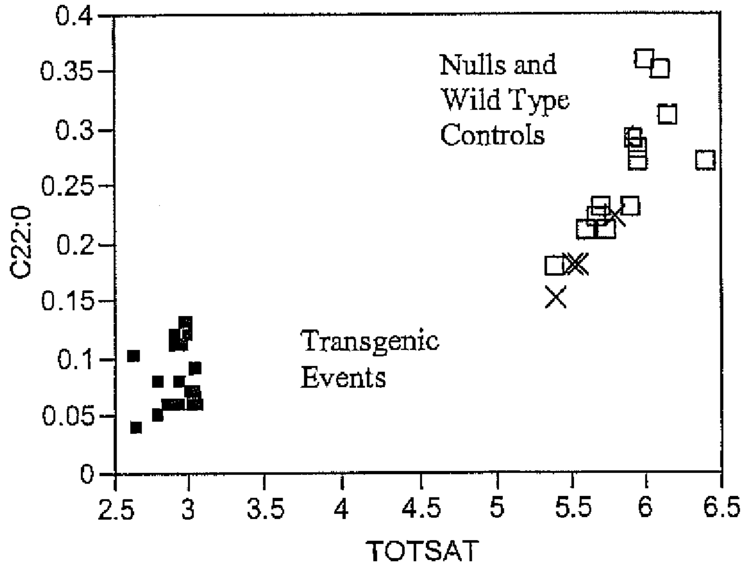
Nulls and
Wild Type
Controls
Transgenic
Events
C22:0
0.4
0.35
0.3
0.25
0.2
0.15
0.1
0.05
0
2.5
3
3.5
4
4.5
5
5.5
6
6.5
TOTSAT

FIG. 6F

Distributions
C16:0 % Total Fatty Acids

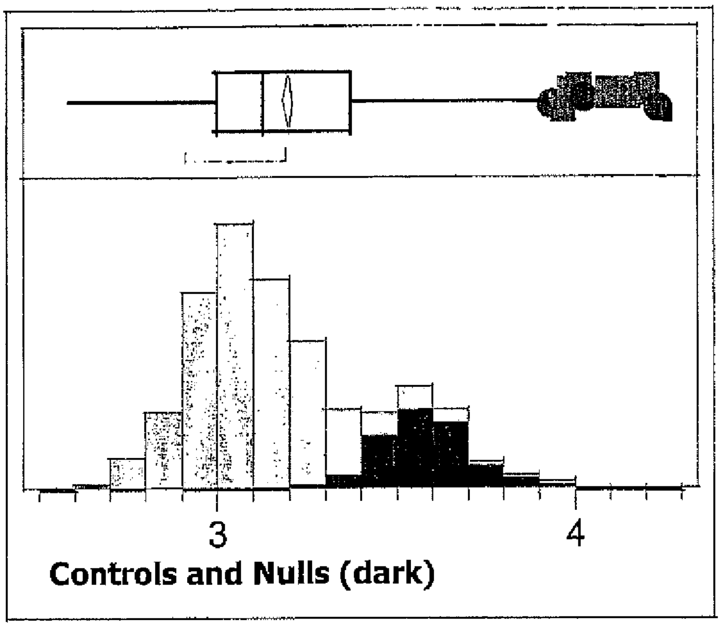


FIG. 6G

Distributions
C16:1% Total Fatty Acids

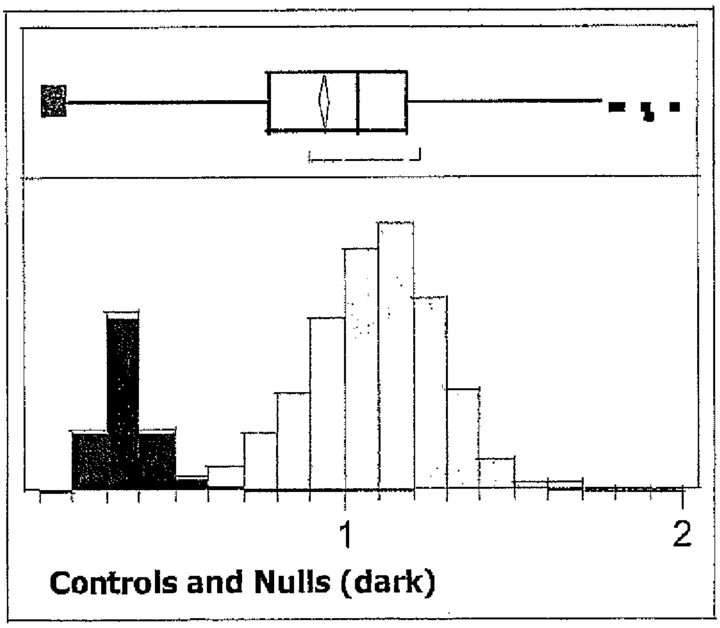


FIG. 6H

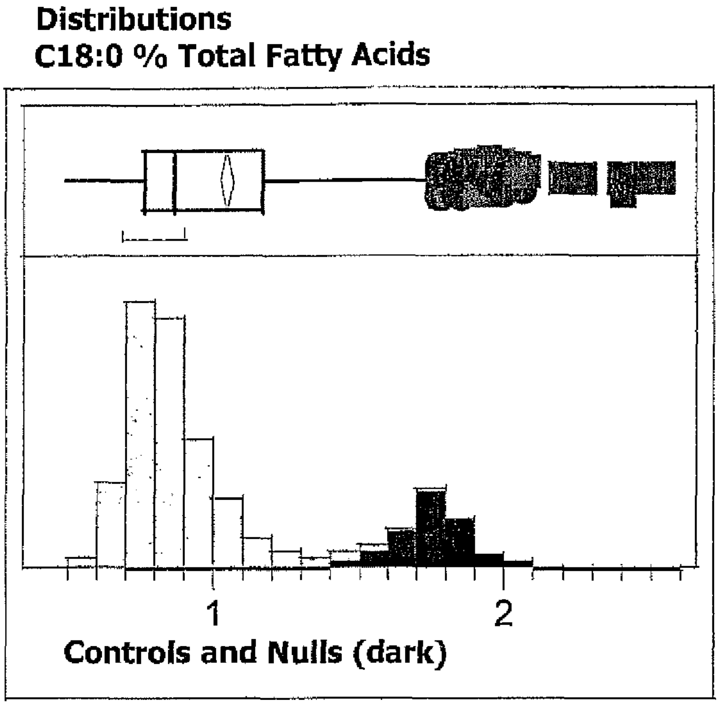
Distributions
C18:0 % Total Fatty Acids
1
2
Controls and Nulls (dark)

FIG. 6I

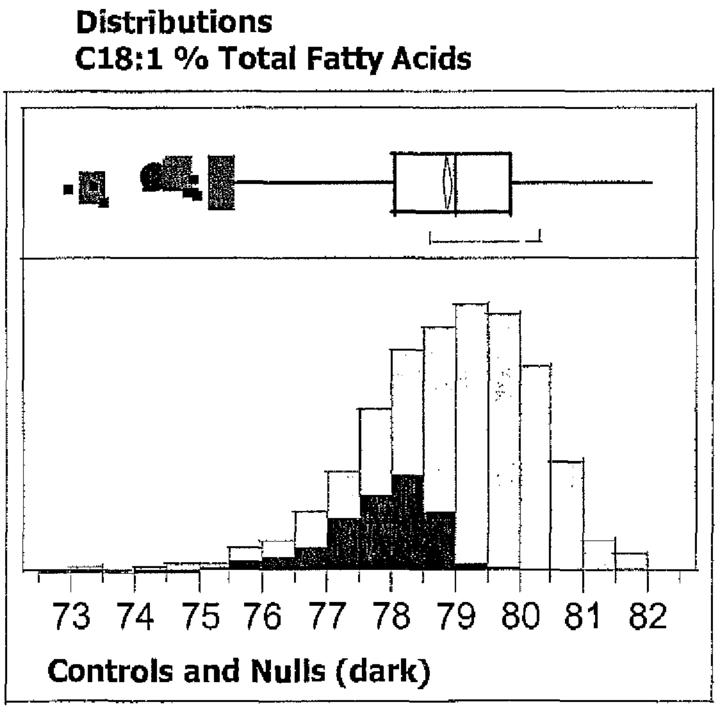
Distributions
C18:1 % Total Fatty Acids
73 74 75 76 77 78 79 80 81 82
Controls and Nulls (dark)

FIG. 6J

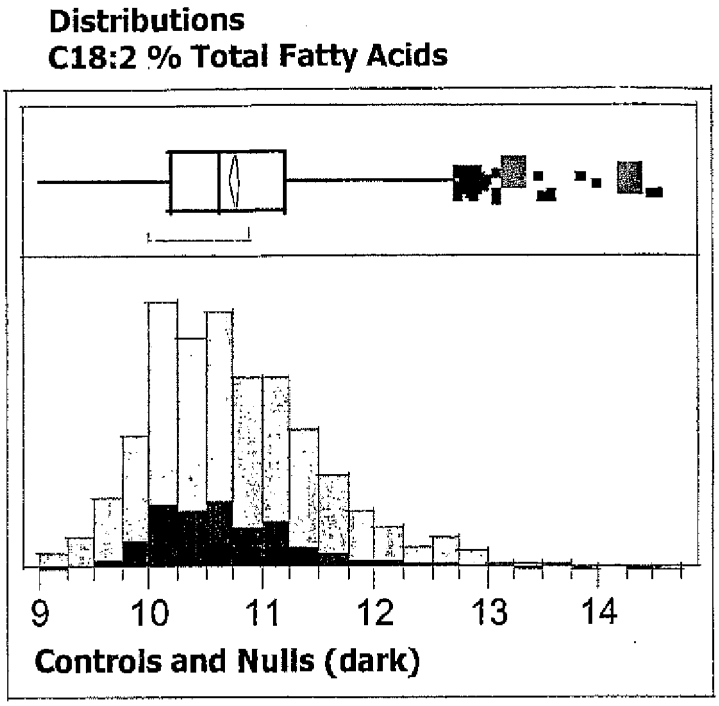
Distributions
C18:2 % Total Fatty Acids
9
10
11
12
13
14
Controls and Nulls (dark)

FIG. 6K

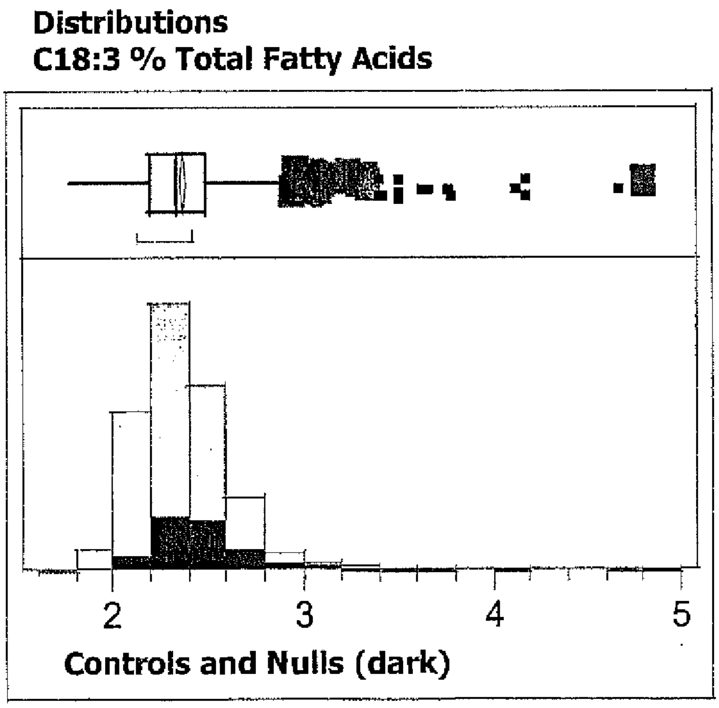
Distributions
C18:3 % Total Fatty Acids
2
3
4
5
Controls and Nulls (dark)

FIG. 6L

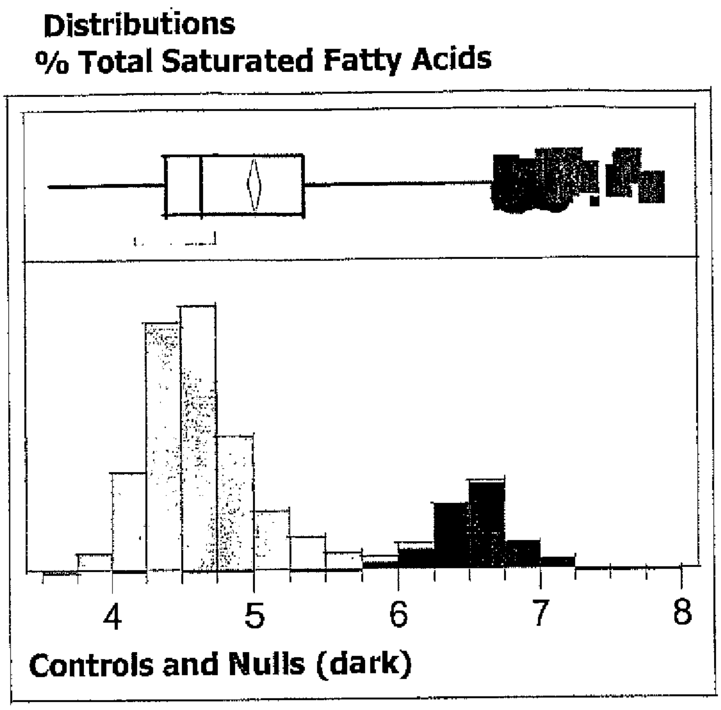
Distributions
% Total Saturated Fatty Acids
4
5
6
7
8
Controls and Nulls (dark)

FIG. 6M

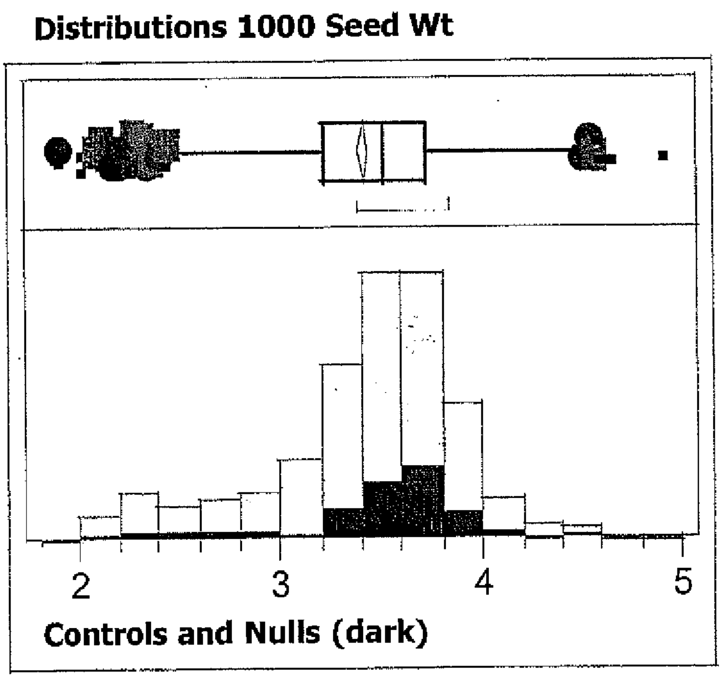
Distributions 1000 Seed Wt
2
3
4
5
Controls and Nulls (dark)

FIG. 6N

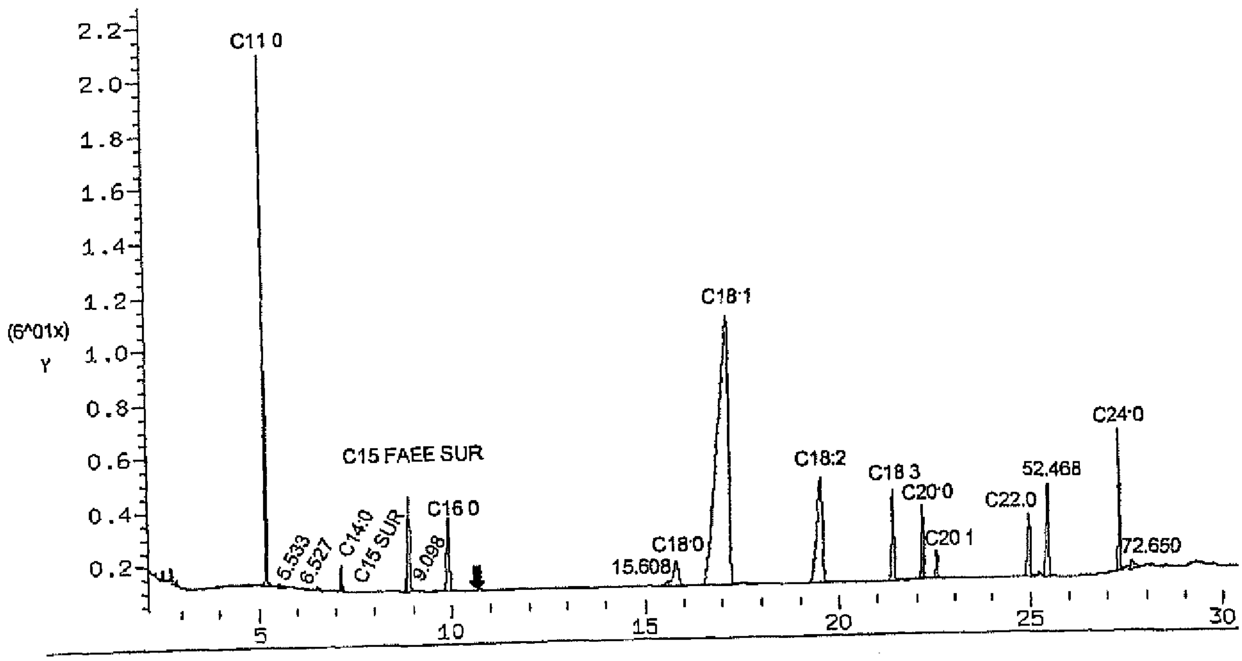
C11 0
C15 FAEE SUR
C18:1
C18:2
C18 3
C20:0
C20 1
C22.0
52.468
C24:0
72.650
C16 0
C14:0
C15 SUR
C18:0
15.608
5.533
6.527
9.098
(6^01x)
Y

FIG. 7A

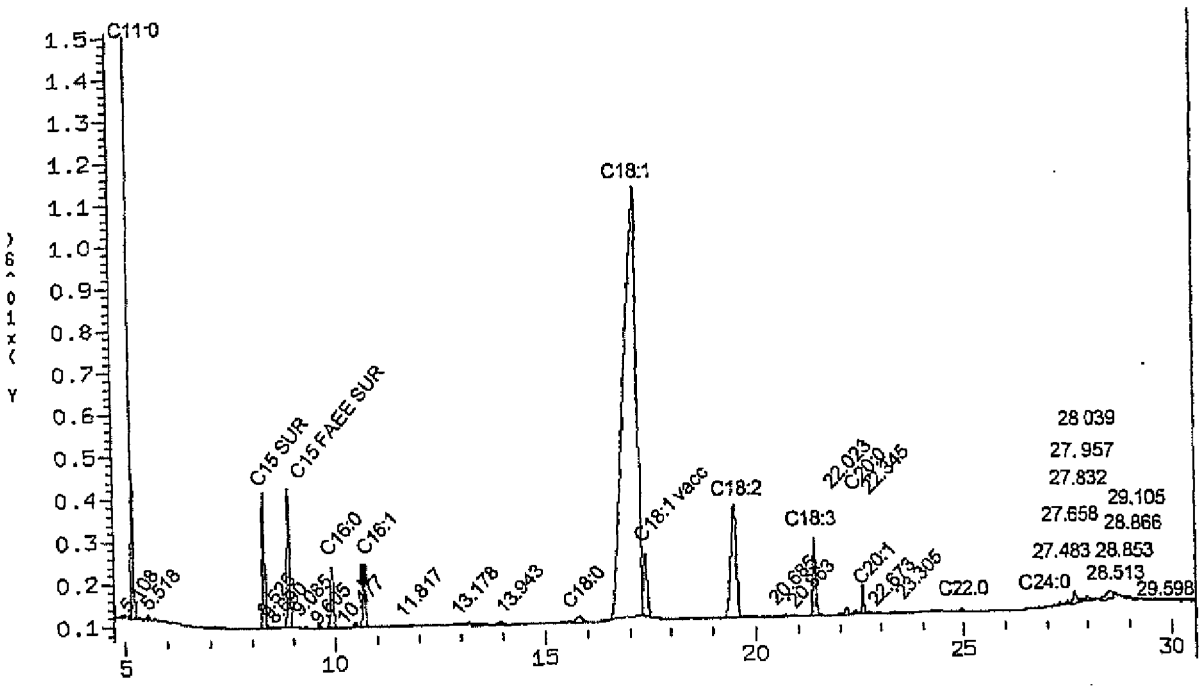
C11:0
C18:1
C15 SUR
C15 FAEE SUR
C16:0
C16:1
C18:0
C18:1 vacc
C18:2
C18:3
C20:0
C20:1
C22.0
C24:0
28 039
27.957
27.832
29.105
27.658
28.866
27.483
28.853
28.513
29.598
11.817
13.178
13.943
22.023
22.345
22.673
23.305
20.685

FIG. 7B

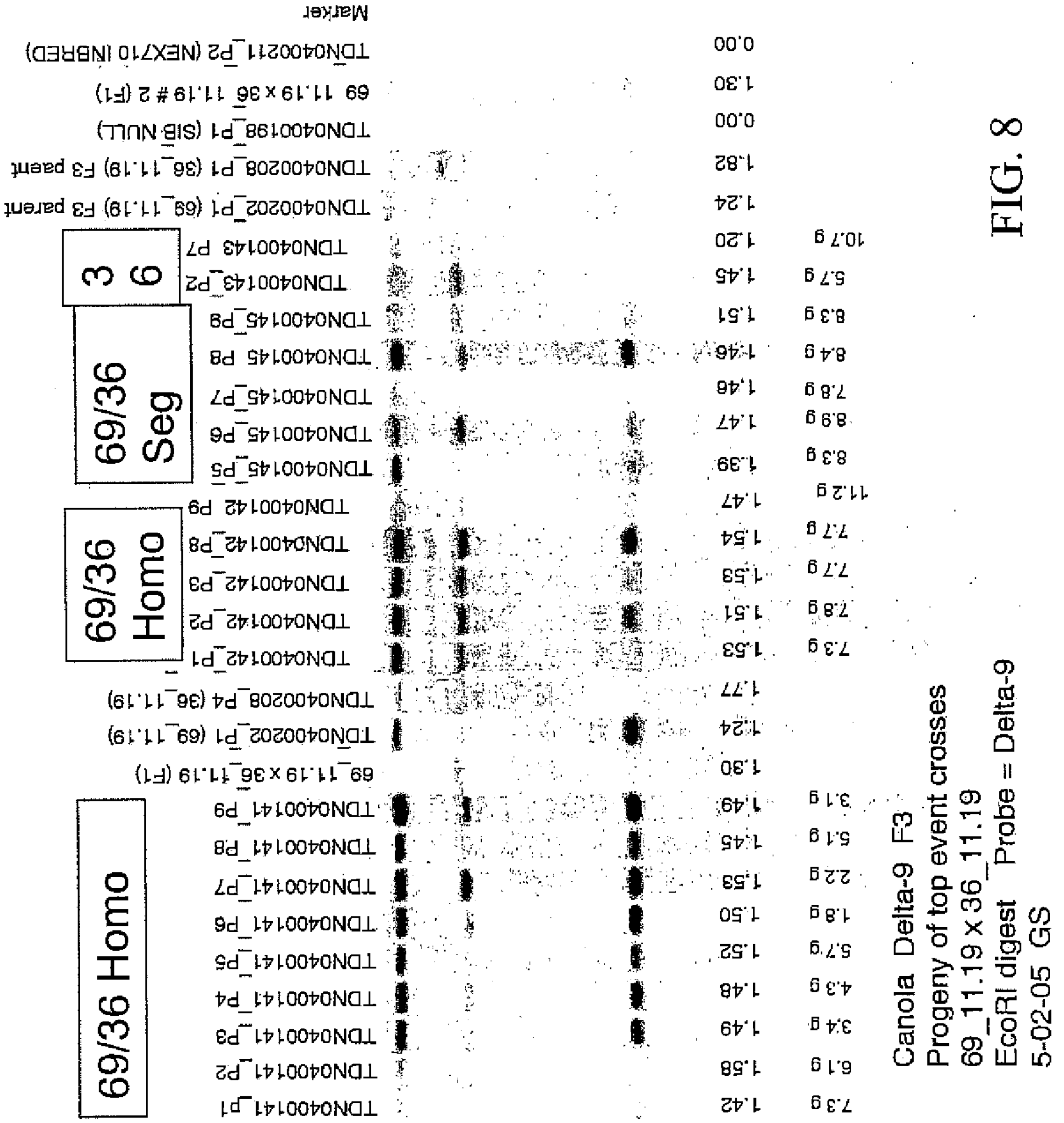
Marker
TDN0400211_P2 (NEX710 INBRED)
69 11.19 x 36 11.19 # 2 (F1)
TDN0400198_P1 (SIB NULL)
TDN0400208_P1 (36_11.19) F3 parent
TDN0400202_P1 (69_11.19) F3 parent
TDN0400143_P7
TDN0400143_P2
TDN0400145_P9
TDN0400145_P8
TDN0400145_P7
TDN0400145_P6
TDN0400145_P5
TDN0400142_P9
TDN0400142_P8
TDN0400142_P3
TDN0400142_P2
TDN0400142_P1
TDN0400208_P4 (36_11.19)
TDN0400202_P1 (69_11.19)
69_11.19 x 36_11.19 (F1)
TDN0400141_P9
TDN0400141_P8
TDN0400141_P7
TDN0400141_P6
TDN0400141_P5
TDN0400141_P4
TDN0400141_P3
TDN0400141_P2
TDN0400141_P1
3 6
69/36 Seg
69/36 Homo
69/36 Homo
Canola Delta-9 F3
Progeny of top event crosses
69_11.19 x 36_11.19
EcoRI digest Probe = Delta-9
5-02-05 GS
FIG. 8

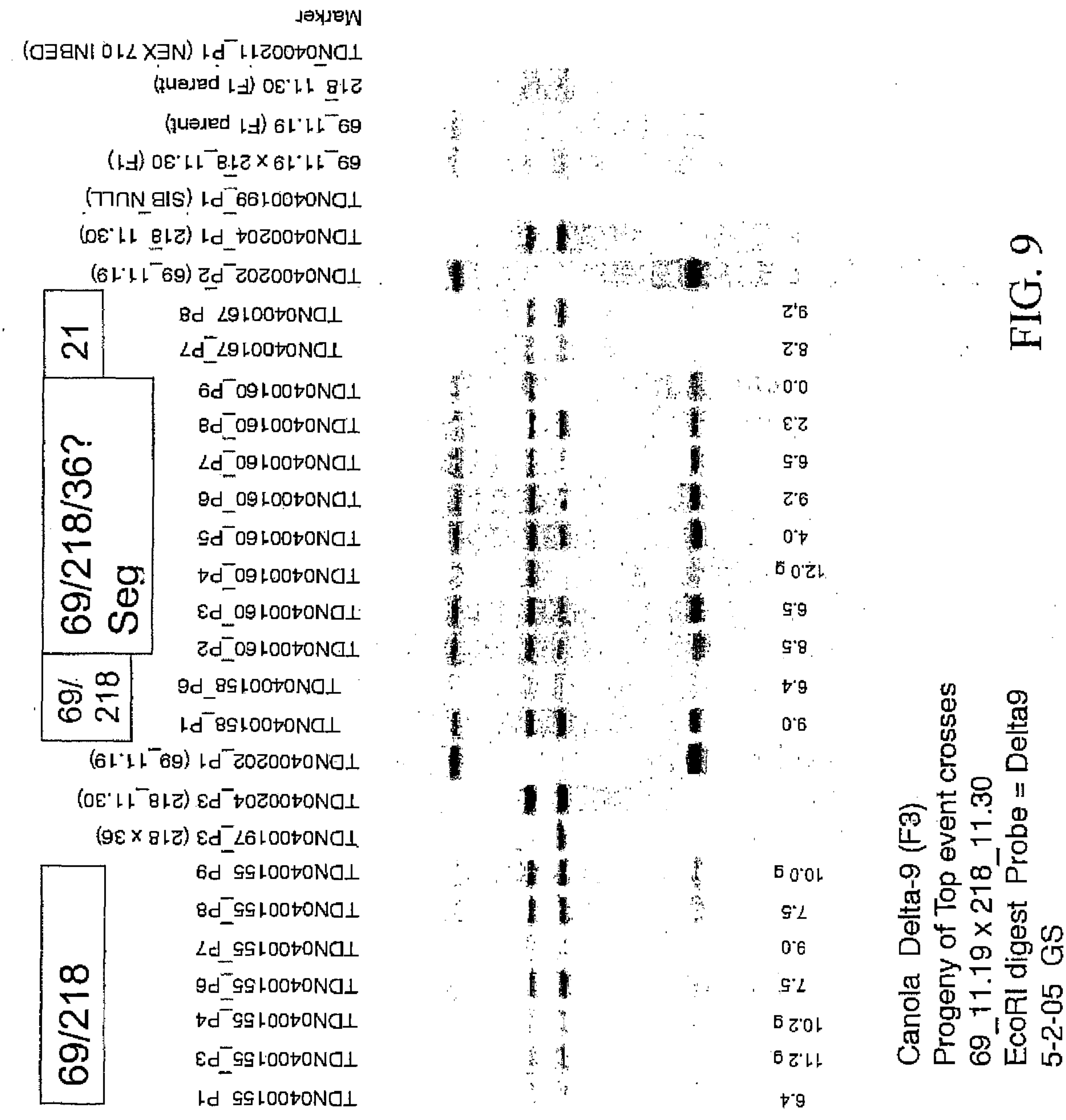
Marker
TDN0400211_P1 (NEX 710 INBED)
218_11.30 (F1 parent)
69_11.19 (F1 parent)
69_11.19 x 218_11.30 (F1)
TDN0400199_P1 (SIB_NULL)
TDN0400204_P1 (218_11.30)
TDN0400202_P2 (69_11.19)
TDN0400167_P8
TDN0400167_P7
TDN0400160_P9
TDN0400160_P8
TDN0400160_P7
TDN0400160_P6
TDN0400160_P5
TDN0400160_P4
TDN0400160_P3
TDN0400160_P2
TDN0400158_P6
TDN0400158_P1
TDN0400202_P1 (69_11.19)
TDN0400204_P3 (218_11.30)
TDN0400197_P3 (218 x 36)
TDN0400155_P9
TDN0400155_P8
TDN0400155_P7
TDN0400155_P5
TDN0400155_P4
TDN0400155_P3
TDN0400155_P1
21
69/218/36? Seg
69/218
69/218
9.2
8.2
0.0
2.3
6.5
9.2
4.0
12.0 g
6.5
8.5
6.4
9.0
10.0 g
7.5
9.0
7.5
10.2 g
11.2 g
6.4
Canola Delta-9 (F3)
Progeny of Top event crosses
69_11.19 x 218_11.30
EcoRI digest Probe = Delta9
5-2-05 GS

FIG. 9

Europäisches Patentamt
European Patent Office
Office européen des brevets

# EUROPEAN SEARCH REPORT

Application Number
EP 17 19 1951

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 0 326 198 A2 (PROCTER & GAMBLE [US]) 2 August 1989 (1989-08-02) * the whole document * ----- | 1-8 | INV. A01H5/10 C07C57/00 A23L19/18 |
| X | US 5 861 187 A (DEBONTE LORIN ROGER [US] ET AL) 19 January 1999 (1999-01-19) * the whole document * ----- | 1-3,6-8 | |
| X | WO 91/15578 A1 (PIONEER HI BRED INT [US]) 17 October 1991 (1991-10-17) * the whole document * ----- | 1-3,6-8 | |
| A | US 6 495 738 B1 (FOLKERTS OTTO [US] ET AL) 17 December 2002 (2002-12-17) * the whole document * ----- | 1 | |
| A | US 5 912 041 A (COVINGTON JR ROBERT MELVIN [US] ET AL) 15 June 1999 (1999-06-15) * the whole document * ----- | 1 | |
| A | US 6 407 317 B2 (FAN ZHEGONG [US]) 18 June 2002 (2002-06-18) * the whole document * ----- | 1 | TECHNICAL FIELDS SEARCHED (IPC) C12N A01H |

The present search report has been drawn up for all claims

1

| Place of search | Date of completion of the search | Examiner |
|---|---|---|
| The Hague | 19 January 2018 | Oderwald, Harald |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 17 19 1951

This annex lists the patent family members relating to the patent documents cited in the above-mentioned European search report. The members are as contained in the European Patent Office EDP file on
The European Patent Office is in no way liable for these particulars which are merely given for the purpose of information.

19-01-2018

| Patent document cited in search report | Publication date | Patent family member(s) | Publication date |
| --- | --- | --- | --- |
| EP 0326198 A2 | 02-08-1989 | AT 106672 T | 15-06-1994 |
| | | AU 2876289 A | 27-07-1989 |
| | | CA 1337251 C | 10-10-1995 |
| | | DE 68915791 D1 | 14-07-1994 |
| | | DE 68915791 T2 | 27-10-1994 |
| | | DK 34289 A | 27-07-1989 |
| | | EP 0326198 A2 | 02-08-1989 |
| | | HK 1006130 A1 | 12-02-1999 |
| | | IE 63733 B1 | 14-06-1995 |
| | | JP H023498 A | 09-01-1990 |
| | | JP 3159975 B2 | 23-04-2001 |
| | | PH 25324 A | 30-04-1991 |
| | | US 4948811 A | 14-08-1990 |
| US 5861187 A | 19-01-1999 | US 5668299 A | 16-09-1997 |
| | | US 5861187 A | 19-01-1999 |
| WO 9115578 A1 | 17-10-1991 | AT 152572 T | 15-05-1997 |
| | | AU 639319 B2 | 22-07-1993 |
| | | CA 2056988 A1 | 05-10-1991 |
| | | CA 2123715 A1 | 05-10-1991 |
| | | CA 2279606 A1 | 05-10-1991 |
| | | DE 69125991 D1 | 12-06-1997 |
| | | DE 69125991 T2 | 25-09-1997 |
| | | DK 0476093 T3 | 02-06-1997 |
| | | EP 0476093 A1 | 25-03-1992 |
| | | EP 0746974 A2 | 11-12-1996 |
| | | US 5387758 A | 07-02-1995 |
| | | US 5434283 A | 18-07-1995 |
| | | US 5545821 A | 13-08-1996 |
| | | WO 9115578 A1 | 17-10-1991 |
| US 6495738 B1 | 17-12-2002 | AR 023301 A1 | 04-09-2002 |
| | | AT 440143 T | 15-09-2009 |
| | | AU 3367899 A | 18-10-1999 |
| | | CA 2323754 A1 | 07-10-1999 |
| | | CY 1109436 T1 | 13-08-2014 |
| | | DK 1068342 T3 | 02-11-2009 |
| | | EP 1068342 A2 | 17-01-2001 |
| | | ES 2328427 T3 | 12-11-2009 |
| | | JP 2002529049 A | 10-09-2002 |
| | | PT 1068342 E | 17-11-2009 |
| | | US 6495738 B1 | 17-12-2002 |
| | | WO 9950430 A2 | 07-10-1999 |
| US 5912041 A | 15-06-1999 | AU 7100494 A | 17-01-1995 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 17 19 1951

This annex lists the patent family members relating to the patent documents cited in the above-mentioned European search report.
The members are as contained in the European Patent Office EDP file on
The European Patent Office is in no way liable for these particulars which are merely given for the purpose of information.

19-01-2018

| Patent document cited in search report | Publication date | Patent family member(s) | Publication date |
|---|---|---|---|
| | | US 5912041 A | 15-06-1999 |
| | | WO 9500035 A1 | 05-01-1995 |
| US 6407317 B2 | 18-06-2002 | AU 695940 B2 | 27-08-1998 |
| | | CA 2161512 A1 | 10-11-1994 |
| | | DE 69420718 D1 | 21-10-1999 |
| | | DE 69420718 T2 | 27-04-2000 |
| | | DK 0696885 T3 | 20-12-1999 |
| | | EP 0696885 A1 | 21-02-1996 |
| | | EP 0928556 A2 | 14-07-1999 |
| | | EP 1329154 A2 | 23-07-2003 |
| | | JP 3527242 B2 | 17-05-2004 |
| | | JP H08509766 A | 15-10-1996 |
| | | US 5767338 A | 16-06-1998 |
| | | US 5969169 A | 19-10-1999 |
| | | US 6201145 B1 | 13-03-2001 |
| | | US 2001031884 A1 | 18-10-2001 |
| | | US 2002083490 A1 | 27-06-2002 |
| | | US 2003126639 A1 | 03-07-2003 |
| | | US 2003140373 A1 | 24-07-2003 |
| | | US 2003140374 A1 | 24-07-2003 |
| | | US 2003180434 A1 | 25-09-2003 |
| | | US 2004234669 A1 | 25-11-2004 |
| | | US 2004237140 A1 | 25-11-2004 |
| | | US 2005008755 A1 | 13-01-2005 |
| | | US 2005008756 A1 | 13-01-2005 |
| | | WO 9424849 A1 | 10-11-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 61753204 P
- WO 9115578 PCT
- US 4948811 A
- US 554526 A
- US 6495738 B
- WO 9950430 A
- US 5723595 A
- US 6706950 B
- US 5057419 A
- US 5777201 A
- WO 0011012 A
- US 6825335 B
- EP 323753 A
- US 5840946 A
- US 5638637 A
- US 5387758 A
- US 5434283 A
- US 5545821 A
- WO 9203919 A
- US 5668299 A
- US 5861187 A
- US 6084157 A
- WO 9306714 A
- US 6270828 B
- US 6562397 B
- US 6680396 B
- US 6689409 B
- US 6169190 B
- US 6063947 A
- US 5850026 A
- US 5952544 A
- US 4627192 A
- US 4743402 A
- WO 2003002751 A
- US 5605793 A
- US 5674485 A
- WO 9316094 A
- US 5380831 A
- US 20030005485 A1
- US 20030159173 A1
- US 5767363 A
- US 6100450 A
- WO 9218634 A1
- WO 0116340 A1
- WO 2003014347 A2
- US 20030233677 A1
- WO 2003092361 A2
- US 20030148300 A1
- US 5932479 A
- US 6004782 A
- US 6642053 B
- US 5693507 A
- US 6680426 B


### Non-patent literature cited in the description


- **STEFANSSON, B. R. et al.** High and Low Erucic Acid Rapeseed Oils. Academic Press, 1983, 144-161
- **AULD, M. et al.** *Crop Sci.,* 1992
- **STUKEY, J. E. et al.** *J. Biol. Chem.,* 1989, vol. 264, 16537-16544
- **STUKEY, J. E. et al.** *J. Biol. Chem.,* 1990, vol. 265, 20144-20149
- **POLASHCOK, J. et al.** *FASEB J.,* 1991, vol. 5, A1157
- **WANG et al.** *J. Agric Food Chem.,* 1996, vol. 44, 3399-3402
- **C. WANG et al.** *Phytochemistry,* 2001, vol. 58, 227-232
- **ADANG et al.** *Gene,* 1985, vol. 36, 289-300
- **KELLER, G.H. ; M.M. MANAK.** DNA. Probes. Stockton Press, 1987, 169-170
- **MANIATIS, T. ; E.F. FRITSCH ; J. SAMBROOK.** Molecular Cloning: A Laboratory Manual. Cold Spring Harbor Laboratory, 1982
- **BELTZ, G.A. ; K.A. JACOBS ; T.H. EICKBUSH ; P.T. CHERBAS ; F.C. KAFATOS.** Methods of Enzymology. Academic Press, 1983, vol. 100, 266-285
- **SUGGS, S.V. ; T. MIYAKE ; E.H. KAWASHIME ; M.J. JOHNSON ; K. ITAKURA ; R.B. WALLACE.** ICN-UCLA Symp. Dev. Biol. Using Purified Genes. Academic Press, 1981, vol. 23, 683-693
- **KARLIN ; ALTSCHUL.** *Proc. Natl. Acad. Sci. USA,* 1990, vol. 87, 2264-2268
- **KARLIN ; ALTSCHUL.** *Proc. Natl. Acad. Sci. USA,* 1993, vol. 90, 5873-5877
- **ALTSCHUL et al.** *J. Mol. Biol.,* 1990, vol. 215, 402-410

- **ALTSCHUL et al.** *Nucl. Acids Res.,* 1997, vol. 25, 3389-3402
- **J. K. DAUN et al.** *J. Amer. Oil Chem. Soc.,* 1983, vol. 60, 1751-1754
- **KATAVIC, CAMPBELL, L. ; FRIESEN, L. ; PALMER, D. ; KELLER, W. ; TAYLOR, D.C.** Agrobacterium-mediated genetic transformation of selected high erucic acid B. napus cultivars. *4th Ganadian Plant Tissue Culture and Genetic Engineering Conference,* 1996
- *CHEMICAL ABSTRACTS,* 506-17-2